# EUROPEAN PATENT SPECIFICATION

(11) **EP 3 030 180 B1**
(45) Date of publication and mention of the grant of the patent: **25.09.2019**
(21) Application number: 14835045.7
(22) Date of filing: 08.08.2014
(51) Int. Cl.: A61B 18/12, A61K 41/00, A61M 37/00, A61P 17/10, A61Q 19/00, B82Y 5/00

(54) **APPARATUS FOR USE WITH ENERGY ACTIVATIBLE MATERIALS**
VORRICHTUNG ZUR VERWENDUNG MIT ENERGIEAKTIVIERBAREN MATERIALIEN
APPAREIL UTILISABLE AVEC DES MATÉRIAUX ACTIVABLES PAR L'ÉNERGIE

(30) Priority: 09.08.2013 US 201361864220 P; 10.01.2014 US 201461925891 P
(43) Date of publication of application: 15.06.2016
(73) Proprietor: Sebacia, Inc., Duluth, GA 30097 (US)
(72) Inventor: PAITHANKAR, Dilip, Duluth, GA 30097 (US); BLOMGREN, Richard Dean, Duluth, GA 30097 (US); MEYER, Todd Joseph, Duluth, GA 30097 (US)
(74) Representative: Jaeger, Michael David
(86) International application number: PCT/US2014/050444
(87) International publication number: WO 2015/021446

(56) References cited:
- WO-A1-98/58699
- US-A- 4 301 968
- US-A- 4 607 185
- US-A- 5 171 215
- US-A- 5 665 141
- US-A1- 2002 138 037
- US-A1- 2009 099 485
- US-A1- 2009 255 979
- US-A1- 2010 121 259
- US-A1- 2010 305 495
- US-A1- 2011 040 235
- US-A1- 2011 060 270
- US-A1- 2012 029 394
- US-A1- 2012 116 263

## Description

### CROSS REFERENCE TO RELATED APPLICATIONS

This application claims the benefit under 35 U.S.C. 119 of U.S. Provisional Patent Application No. 61/864,220, filed August 9, 2013, titled "ULTRASOUND APPARATUS FOR MATERIAL DELIVERY INTO SKIN," and also to U.S. Provisional Patent Application No. 61/925,891, filed January 10, 2014, titled "ULTRASOUND APPARATUS AND METHODS FOR MATERIAL DELIVERY INTO SKIN."

### FIELD

This application relates to ultrasound systems for delivery of materials into the skin of a mammal.

### BACKGROUND

Ultrasound has been suggested for use in assisting in the delivery of materials into the body through the skin. The ultrasound assisted delivery has several applications. In one exemplary system, ultrasound is used to deliver various molecules through the skin at a single location. In this exemplary application, a fluid reservoir is placed on top of the skin in which an ultrasound horn is immersed. The fluid reservoir/horn assembly is pressed into the skin to form a seal.

While perhaps satisfactory for some single spot applications, a more robust design would not be limited to a single spot. It is possible to deliver materials at multiple treatment locations but not until numerous practical limitations are addressed. One example of such a practical issue is having an applicator that may translate over skin to treat different areas of skin. Another practical issue is providing that the delivery fluid should not be spilled, either during application at a single spot, movement to another spot, or during continuous translation. Still another practical factor to address is how to adjust operations when - during ultrasound operations - the delivery fluid is heated. Increases in delivery fluid temperature may degrade or alter the structural make up or ingredients of the fluid or worse cause burn or injury to the skin treatment site.

While ultrasound delivery systems are available, improvements are needed to provide systems and methods for permitting multiple sites or large areas to be treated while also maintaining a sealed surface or translating the sealed surface over the skin. In addition, improved control of the environment during application and optimization of immersion ultrasound parameters are desirous.

US patent publication US5171215 discloses a method and device for transferring substances through the skin at a treatment site. A device having a cup-shaped body is pressed against the skin and the treating substance is supplied to an interior chamber of the body. The treatment site is subjected to pressure and ultrasonic energy whilst a vacuum is drawn thereabout through a surrounding annular chamber.

### SUMMARY OF THE DISCLOSURE

The embodiments and/or examples of the following description which are not covered by the appended claims are considered as not being part of the present invention. The invention is defined in the appended set of claims.

In general, in one embodiment a delivery device includes a housing, an ultrasound converter within and supported by the housing and an ultrasound horn coupled to the ultrasound converter and extending through an opening in the housing. The device may also include a first mating surface adjacent the opening, a cup assembly having an upper surface and a lower surface and a generally cylindrical interior portion extending between an opening in the upper surface and an opening in the lower surface. Additionally, the device may include a second mating surface adjacent the cup assembly upper surface, wherein when the first mating surface is coupled to the second mating surface a distal most portion of the ultrasound horn is disposed within the generally cylindrical interior portion without contacting the cup assembly.

This and other embodiments can include one or more of the following features. In one aspect, the ultrasound horn coupled to the ultrasound converter can extend through without contacting an interior portion of the cup assembly. In another aspect, the ultrasound converter can be coupled to the housing without coupling adjacent to an area of the ultrasound converter having one or more piezoelectric elements. In a further aspect, the ultrasound converter can be coupled to the housing while providing a gap between an interior of the housing and a portion of the ultrasound converter having one or more piezoelectric elements. In yet another aspect, the diameter of the opening in the upper surface, the diameter of the opening in the lower surface and the diameter of the generally cylindrical interior portion can be about the same.

In an additional aspect, when the first mating surface is coupled to the second mating surface, a distal most portion of the ultrasound horn can be proximal to the opening in the lower surface, or, a distal most portion of the ultrasound horn can be proximal to and spaced about 13 mm from the opening in the lower surface, or, a distal most portion of the ultrasound horn can be proximal to and spaced from about 5 mm to about 13 mm from the opening in the lower surface, or, a distal most portion of the ultrasound horn can be proximal to and spaced from about 2 mm to about 30 mm from the opening in the lower surface, or, a distal most portion of the ultrasound horn can be adjustably spaced from about 0 mm to about 30 mm from the opening in the lower surface by sliding one or more of the ultrasound components relative to a clamping surface in the interior of the housing, or, a distal most portion of the ultrasound horn can be adjustably spaced from about 0 mm to about 30 mm from the opening in the lower surface by a process of loosening a securing device fixing the position of the ultrasound components relative to a securing surface in the housing interior, which may cause movement of one or more of the ultrasound components relative to the securing surface and tightening the securing device to hold the ultrasound components in the desired position, or a distal most portion of the ultrasound horn can be adjustably spaced from about 0 mm to about 30 mm from the opening in the lower surface by a process of operating a securing device to permit axial movement of the ultrasound components relative to a securing surface of the securing device, which may cause axial movement of one or more of the ultrasound components relative to the securing surface and operating the securing device to inhibit axial movement of the ultrasound components relative to the securing surface so as to maintain the ultrasound components in the desired position during the operation of the ultrasound components.

In a further aspect, the delivery device may include a calibration disc or gage which can have one or more of the following attributes: a portion of the disc or gage adapted and configured to fit within the cup assembly interior, a portion of the disc or gage adapted or configured to engage with a portion of the ultrasound horn, and an indication of the resulting horn-skin spacing provided by a calibration disc or gage.

In yet another aspect, when the first mating surface is coupled to the second mating surface and the a surface of the cup assembly forms a seal on a surface of skin to be treated, a distal most portion of the ultrasound horn can be proximal to and spaced apart from the skin such that sufficient fluid may be introduced between the skin and the distal most portion of the ultrasound horn can facilitate an immersion ultrasound operation within the interior of the cup assembly, or, a distal most portion of the ultrasound horn can be proximal to and spaced apart from the skin such that sufficient fluid may be introduced between the skin and the distal most portion of the ultrasound horn can facilitate a cavitation based ultrasound operation within the interior of the cup assembly.

In an additional aspect, the first mating surface or the second mating surface can be attached to or formed in an interior surface or an exterior surface of the housing or the cup assembly, respectively. In another aspect, the first mating surface can be a detent and the second mating surface can have a complementary shape to receive the detent. In still another aspect, the first mating surface can be a threaded portion and the second mating surface can have a complementary shape to mate with the threaded portion. In a further aspect, the first mating surface can be sized for a friction fit with the second mating surface. In still another aspect, the first mating surface can be attached to an interior surface of the housing. In a further aspect, the first mating surface and the second mating surface can form a pin and groove pair. In an additional aspect, less than a full rotation between the housing and the cup assembly can join the first mating surface to the second mating surface.

In an additional aspect, the delivery device includes an annulus formed in the cup assembly lower surface, an opening in a wall of the cup assembly and a conduit in communication with the opening and a portion of the cup assembly. In still another aspect, the annulus can have a depth of about 1 mm to about 4 mm. In a further aspect the annulus can have a radius of curvature. In yet another aspect, the annulus can have a generally semi-circular cross section shape. In a further aspect, the delivery device can further include a plurality of holes formed in the annulus and in communication with the conduit. Alternatively, in another aspect, the portion of the cup assembly can be directly adjacent a portion of the annulus. In another aspect, the annulus can have a generally rectangular cross section shape.

In an additional aspect, the delivery device may further include a seal along the cup assembly lower surface. In another aspect, the seal can circumscribe the interior portion of the cup assembly. In a further aspect, the seal can have a plurality of elements each one can have a cross section shape that can be one of: u-shaped, t-shaped, v-shaped, and j-shaped. In yet a further aspect, the first seal can have a cross section shape that can have one of: u-shaped, t-shaped, v-shaped and j-shaped. In another aspect, the delivery device may further include a recess in the lower surface to receive a portion of the seal. Additionally, the delivery device can further include a first seal and a second seal attached to the cup assembly lower surface, the first seal can be closer to the cup housing interior portion. Alternatively, the first seal and the second seal can circumscribe the cup assembly interior portion. In additional aspect, the delivery device can further include a pair of recesses in the lower surface with one recess of the pair of recess sized to receive a portion of the first seal and the other of the recesses in the pair of recesses sized to receive the second seal. In another aspect, the first seal and the second seal can be O-rings. In still another aspect, the first seal or the second seal can have a cross section shape that can be one of: u-shaped, t-shaped, v-shaped and j-shaped. In yet another aspect, the first seal or the second seal can have a plurality of elements each one can have a cross section shape that can be one of: u-shaped, t-shaped, v-shaped and j-shaped. In still another aspect, a seal of the cup assembly can be adapted and configured to engage with the tissue surface and can provide sealing as in a labyrinth seal. In another aspect, a sealing surface of the cup assembly can include an array of teeth and grooves. Additionally, when the cup assembly is pressed into a tissue at a delivery site, the array of teeth and grooves can form a complementary array of teeth and grooves in the delivery site whereby the array and the complementary array can function as a labyrinth seal. In another aspect, the first seal or the second seal can have a beveled edge. In still another aspect, a distal most edge portion of the beveled edge can be directed towards the perimeter of the lower surface. In one aspect, a distal most edge portion of the beveled edge can be directed towards the cup assembly interior portion. In an addition, the delivery device can further include a seal on the cup assembly lower surface between an outer edge of the annulus and a perimeter of the lower surface. In yet another aspect, the delivery device can further include a seal on the cup assembly lower surface between an inner edge of the annulus and a perimeter of the cup assembly interior portion.

In still another aspect, the delivery device can further include an inlet in the cup assembly interior portion. In yet another aspect, the delivery device can further include a container of a formulation for introduction into the cup assembly interior portion, the container in communication with the inlet. In still a further aspect, the delivery device can further include when the first mating surface can be joined to the second mating surface, the distal most portion of the ultrasound horn can be positioned below the inlet in the cup assembly interior portion. In yet another aspect, the delivery device can further include a fluid channel formed within a wall of the cup assembly and positioned adjacent the opening in the lower surface, the channel can have an inlet and an outlet in an exterior of the cup assembly. In a further aspect, the delivery device can further include an opening extending through a wall of the cup assembly to the interior portion and a probe disposed within the opening and in communication with the cup assembly interior portion. Additionally, the probe can be a temperature sensor. In another aspect, the probe can be selected for a capability to detect, determine or monitor a parameter of the treatment site, a parameter of a fluid in the cup assembly or a characteristic, a parameter or an indication of the environment within the interior portion of the cup assembly.

In a further aspect, the delivery device can further include a gasket having a first portion sized to seal against an exterior portion of the ultrasound horn and a second portion sized to seal against the cup assembly interior portion. In another aspect, the gasket can have a conical shape, a trapezoidal shape or truncated conical shape. In yet another aspect, the gasket can form a fluid tight seal around the ultrasound horn without impairing ultrasound operations performed by the horn. In still another aspect, the sealing force or placement of the gasket seal on the ultrasound horn can be selected to provide a suitable fluid seal over the cup assembly interior without dampening the horn ultrasound output. In an additional aspect, the cup assembly can further include a first cup assembly portion. Further, a second cup assembly portion can be configured to engage with the first cup assembly portion. In an additional aspect, the functional operation of the cup assembly can be divided between the first portion and the second portion. In a further aspect, the first cup assembly portion can be inoperable as a cup assembly unless coupled to the second cup assembly portion. In still another aspect, when the first cup assembly portion is coupled to the second cup assembly portion a fluid conduit can be formed between the adjacent walls of the first and second cup assembly portions. In yet another aspect, at least a portion of the cup assembly can be transparent. In a further aspect, the cup assembly can be transparent in a portion selected to permit viewing of the cup assembly interior portion in use.

In general, there is provided a method of enhancing penetration of particles in a formulation into a follicle, comprising mating an ultrasound horn to a cup assembly, positioning an interior portion of the cup assembly over the follicle selected to receive the particles in the formulation, and introducing the formulation into the cup assembly interior portion. The method may also include operating an ultrasound system coupled to the ultrasound horn to produce cavitation within the formulation and driving a plurality of particles in the formulation into the follicle during the operating step.

This and other embodiments can include one or more of the following features. In one aspect, the method may include applying vacuum with a portion of the cup assembly to a portion of a treatment site which may include the follicle adjacent the cup assembly. In a further aspect, the method may further include moving the cup assembly across the skin during the operating and the driving step. In an additional aspect, the applying vacuum step can further include maintaining a constant vacuum level, adjusting to a decreased vacuum level or adjusting to an increased vacuum level during the operating step or the driving step. In an additional aspect, the method can further include moving the cup assembly across the skin during the operating step or and the driving step. In a further aspect, the applying vacuum step can further include maintaining a constant vacuum level, adjusting to a decreased vacuum level or adjusting to an increased vacuum level while translating the cup assembly across a treatment site and thereafter maintaining a constant vacuum level, adjusting to a decreased vacuum level or adjusting to an increased vacuum level prior to performing another operating step. In a further aspect, during one or more of the steps of applying vacuum, maintaining a constant vacuum level, adjusting to a decreased vacuum level or adjusting to an increased vacuum level the vacuum level can remain between about -0.8 atm to about -0.1 atm. In still another aspect, during one or more of the steps of applying vacuum, maintaining a constant vacuum level, adjusting to a decreased vacuum level or adjusting to an increased vacuum level the vacuum level can remain between about -0.5 atm to about -0.1 atm. In still another aspect, during one or more of the steps of applying vacuum, maintaining a constant vacuum level, adjusting to a decreased vacuum level or adjusting to an increased vacuum level the vacuum level can remain between about -0.33 atm to about -0.1 atm. In yet another aspect, the applying vacuum step can be performed before the introducing step. In a further aspect, the method can further include applying vacuum to a portion of the skin surrounding the follicle during the operating and the driving step. In still another aspect, the method can further include moving the cup assembly across the skin during the operating and the driving step. In an additional aspect, the method can further include circulating a fluid at a controlled temperature within a portion of the cup assembly during at least one the introducing, the operating or the driving step. In yet another aspect, the step circulating a fluid at a controlled temperature within a portion of the cup assembly during at least one the introducing, the operating or the driving step can be performed in a fluid channel within a side wall of the cup assembly. In still a further aspect, the step circulating a fluid at a controlled temperature within a portion of the cup assembly during at least one the introducing, the operating or the driving step can be performed within the cup assembly interior portion. In yet another aspect, the fluid at a controlled temperature can be a delivery fluid, a cleaning fluid, a delivery pre-treatment fluid or a pharmaceutical formulation. In an additional aspect, the method can further include performing the circulating step based on an input from a temperature sensor providing an indication of the temperature of the skin or the formulation. In an additional aspect, the method can further include after the mating step: releasing a securing device, adjusting a horn to skin spacing and engaging the securing device. In still another aspect, the method can further include adjusting an amount of vacuum applied before, during or after performing the moving the cup assembly across the skin step.

In one aspect, there are embodiments of the present invention relating to a delivery system adapted and configured to draw negative pressure in a distal annular ring to allow for suction of skin into the ring to form a spill-proof seal. In another aspect of the an embodiment of the present invention, there is provided a delivery device that draws negative pressure in a distal annular ring to allow for suction of skin into the ring to form a spill-proof seal to allow for single spot treatment, or to allow for translation across skin surface. In still another aspect, the distal end or a portion of the ring is made of flexible material with characteristics of appropriate durometer, lubricity coefficient of friction and the like to allow for translation across different planes of skin while keeping the seal intact. Still further, there may be provided one or more cooling channels in a portion of the delivery device to control temperature of the fluid in the internal chamber. In still another aspect, there is provided in the delivery device one or more ports in the internal chamber to introduce and withdraw fluid and/or for introduction of monitoring probes such as, for example, a temperature monitoring probe.

In still other aspects, there are provided methods of delivery of substances into follicles and follicular appendages of skin in which ultrasound horn is immersed and operated in a suspension or solution of the substance in a fluid on top of the skin where the skin-horn distance is in the range of 1 mm - 20 mm, the frequency is in the range of 20 kHz to 200 kHz, the amplitude of the horn is in the range of 5-35 microns.

In still other aspects, there are provided methods of delivery of substances into follicles and follicular appendages of skin in which ultrasound horn is immersed and operated in a suspension or solution of the substance in a fluid on top of the skin where the skin-horn distance is 11-14 mm and peak-to-peak displacement of 8.8-12.0 micrometers.

In still other aspects, any of the above methods include modifications of the ultrasound operation or ultrasound exposure that is pulsed, with on-time in the range of 0.1 s - 1 s and off-time in the range of 0.1 s-1 s, and the number of pulses in the range of 1 to 200. In one aspect, the on-time and off-time are of equal durations. In another aspect, the on-time is of a longer duration than the off time. In still another aspect, the on-time is of a shorter duration than the off time.

In one aspect, when the first mating surface is coupled to the second mating surface, a distal most portion of the ultrasound horn can be adjustably spaced from about 0 mm to about 30 mm from the opening in the lower surface using a mechanical, a motor driven, or computer controlled process.

In general, in one embodiment, a method for enhancing penetration of particles in a formulation into a target volume of a treatment site, includes positioning an interior portion of a cup assembly over an intended ultrasound transport pathway in communication with the target volume selected to receive the particles in the formulation; operating an ultrasound system coupled to the cup assembly to produce cavitation within the formulation; and driving a plurality of particles in the formulation along the intended ultrasound transport pathway into the target volume.

This and other embodiments can include one or more of the following features. In one aspect, the method can further include applying vacuum with a portion of the cup assembly to a portion of the treatment site adjacent the cup assembly. In another aspect, the method can further include moving the cup assembly across the skin during the operating and the driving step. In an alternative aspect the method of applying the vacuum step may include maintaining a constant vacuum level, adjusting to a decreased vacuum level or adjusting to an increased vacuum level during the operating step or the driving step. In yet another aspect, the method of applying vacuum step can further include maintaining a constant vacuum level, adjusting to a decreased vacuum level or adjusting to an increased vacuum level while translating the cup assembly across a treatment site and thereafter maintaining a constant vacuum level, adjusting to a decreased vacuum level or adjusting to an increased vacuum level prior to performing another operating step. In a further aspect, during one or more of the steps of applying vacuum, maintaining a constant vacuum level, adjusting to a decreased vacuum level or adjusting to an increased vacuum level the vacuum level can remain between about -0.33 atm to about -0.1 atm.

In still another aspect, the method can further include applying vacuum to a portion of the skin surrounding the treatment site during the operating step or and the driving step. In one aspect, the method can further include adjusting an ultrasound horn to skin spacing based on the ultrasound transport mode and the ultrasound transport characteristics of the formulation. In another aspect, the attribute of the calibration disc can be adapted an configured to produce a horn-skin spacing for a desired ultrasound transport mode for a particle formulation, a modified particle formulation or an enhanced particle formulation as in any of the above embodiments.

In general, in one embodiment, a method of delivery of a substance into target volumes within the skin, including positioning an ultrasound horn immersed in fluid about 1 mm - 20 mm from the skin, wherein the fluid comprises a nanoparticle formulation to be delivered; applying ultrasound to the ultrasound horn at a frequency of about 20 kHz to about 200 kHz and an amplitude of about 5-35 microns.

This and other embodiments can include one or more of the following features. In one aspect, positioning the ultrasound horn can include positioning the ultrasound horn about 11-14 mm from the skin. In another aspect, applying ultrasound can include applying ultrasound with an amplitude peak-to-peak displacement of about 8.8-12.0 microns. In a further aspect, applying ultrasound can include applying pulsed ultrasound. In an alternative aspect, the pulsed ultrasound can include pulses of about 0.1-1s on and about 0.1 s - 1 s off. In yet another aspect, a number of pulses can be about 1-200.

In general, in one embodiment, a method for enhancing penetration of particles in a formulation into a target volume, includes mating an ultrasound horn to a cup assembly; positioning an interior portion of the cup assembly over a selected target volume to receive the particles in the formulation, wherein a distance between the skin surface of the target volume and the ultrasound horn is about 1-20 mm; introducing the formulation into the cup assembly interior portion; operating an ultrasound system coupled to the ultrasound horn at a frequency of about 20-200 kHz and at an amplitude of about 5-35 microns; and driving a plurality of particles in the formulation into the target volume during the operating step.

This and other embodiments can include one or more of the following features. In one aspect, a distance between the target volume and the ultrasound horn is about 11-14 mm. In another aspect, the amplitude of the ultrasound can have a peak-to-peak displacement of about 8.8-12.0 microns. In a further aspect, the ultrasound can be pulsed ultrasound. In an alternative aspect, the pulsed ultrasound can include pulses of about 0.1-1s on and about 0.1 s - 1 s off. In yet another aspect, a number of pulses can be about 1-200. In still another aspect, the method can further include applying vacuum with a portion of the cup assembly to a portion of a treatment site comprising the follicle adjacent the cup assembly. In an alternative aspect, the method can further include moving the cup assembly across the skin during the operating and the driving step. In another aspect, the formulation can be a particle formulation, a modified particle formulation or an enhanced particle formulation.

In one embodiment, there is a method of treating or ameliorating a follicular skin disease in a subject by: topically applying a formulation comprising sub-micron particles comprising a light absorbing material to the subject's skin, and operating an ultrasound device in communication with the material for facilitating delivery of said material into a hair follicle, sebaceous gland, sebaceous gland duct, or infundibulum of the skin. Thereafter or concurrently therewith, there is a step of exposing said sub-micron particles to energy activation, thereby treating or ameliorating the follicular skin disease in the subject. In one aspect, the operating step is facilitated by ultrasound-created microjets within the formulation. In one aspect, the step of exposing said sub-micron particles to energy activation comprises irradiating said sub-micron particle with light, thereby heating the particle. In some treatment embodiments the particles are within a portion of a pilosebaceous unit during irradiation, are within a sebaceous gland during irradiation, are substantially completely within the sebaceous gland during irradiation, are within a sebaceous gland duct during irradiation, are substantially completely within the sebaceous gland duct during irradiation, are within an infundibulum involved in the follicular skin disease during irradiation.

In some embodiments, the particle formulation, the modified particle formulation or the enhanced particle formulation comprises a photoactive compound, photodynamic therapy (PDT) pro-drug or PDT drug.

In some embodiments, there is an ultrasound device operated cooperatively with a particle formulation, a modified particle formulation or an enhanced particle formulation wherein the operating an ultrasound device is at a frequency in the range of 20 kHz to 500 kHz, in the range of 20 kHz to 100 kHz, in the range of 20 kHz to 60 kHz, in the range of 30 kHz to 50 kHz.

In some embodiments, there is an ultrasound device operated cooperatively with a particle formulation, a modified particle formulation or an enhanced particle formulation wherein the ultrasound power density during the operating step is from about 0.5-50 W/cm2.

In one specific aspect, an ultrasound horn utilized for applying ultrasound to a formulation, a modified or enhanced formulation has a face peak-to-peak amplitude displacement in the range of 0.5 to 30 microns.

In yet another aspect, the particle, modified or enhanced particle formulation includes a sub-micron particle size selected for passage through the hair follicle and into a sebaceous gland of the hair follicle, or a terminal follicle or a vellus follicle or a sebaceous follicle or, alternatively, a particle size between about 0.01 microns to about 1.0 microns or between about 0.05 to about 0.25 microns.

This and other embodiments can include one or more of the following features. In still another aspect, the operating step can further include selecting characteristics for the ultrasound-created microjets to create bubbles in the formulation about the same size as the hair follicle pore. In yet another aspect, the hair follicle can be a terminal follicle. In a further aspect, the hair follicle can be a vellus follicle. In one aspect, the hair follicle can be a sebaceous follicle. In another aspect, the operating step can further include selecting characteristics for low frequency ultrasound induced cavitation for creating bubbles in the formulation about the same size as the hair follicle. In an alternative aspect, the hair follicle can be a terminal follicle. In still another aspect, the hair follicle can be a vellus follicle. In one aspect the hair follicle can be a sebaceous follicle.

In another aspect, the ultrasound-created microjets in the formulation can be within about 50 microns to about 100 microns of the surface of the skin. In a further aspect, the follicular disease for treatment can be hyperhidrosis and the operating step can deliver particles into an eccrine gland via the eccrine gland duct. In an alternative aspect, the formulation can be a modified particle formulation or an enhanced particle formulation.

In general, in one embodiment, a method of improving the appearance of enlarged pores in the skin of a subject, the method including a) topically applying a formulation in any of the embodiments herein to the subject's skin; b) ultrasonically facilitating delivery of said materials to a hair follicle, sebaceous gland, sebaceous gland duct, or infundibulum of the skin; and c) exposing said sub-micron particles to energy activation, thereby improving the appearance of enlarged pores in the skin of the subject.

In general, in one embodiment, a method of improving the appearance of oily skin of a subject, the method including a) topically applying a formulation in any of the embodiments herein to the subject's skin; b) ultrasonically facilitating delivery of said sub-micron particles to a hair follicle, sebaceous gland, sebaceous gland duct, or infundibulum of the skin; and c) exposing said sub-micron particles to energy activation, thereby improving the appearance of oily skin of the subject.

In general, in one embodiment, a method for permanently removing hair of a subject, the method including a) topically applying a light-absorbing material with in a formulation in any of the embodiments herein to the skin of the subject, and b) ultrasonically facilitating delivery of the material in the formulation along the hair shaft; c) exposing said material to energy activation, thereby permanently removing said hair.

In general, in one embodiment, a method for treating hyperhidrosis by thermally damaging eccrine glands or their surrounding area, the method including a) topically applying a light-absorbing material recited in any of the embodiments herein to the skin of a subject, and b) ultrasonically facilitating delivery of the material in the formulation into an eccrine gland or the surrounding area; c) exposing said material to energy activation, thereby permanently removing said glands, inhibiting a function of said gland or thermoablating said gland and treating hyperhidrosis.

This and other embodiments can include the following features. In another aspect, there is a method of facilitating delivery of a light absorbing material within a modified formulation to a target volume within the skin of a subject to achieve a therapeutic effect, by topically applying the modified formulation comprising a light absorbing material to a subject's skin to deliver the material to a reservoir within the skin; ultrasonically facilitating delivery of said material to a target volume within the skin of the subject; and exposing said light absorbing material to a series of light pulses to heat the material and thermally damage the target volume to achieve a therapeutic effect.

In general, in one embodiment, a method of facilitating delivery of a light absorbing material within an enhanced formulation to a target volume within the skin of a subject to achieve a therapeutic effect, the method including a) topically applying the modified formulation comprising a light absorbing material to a subject's skin to deliver the material to a reservoir within the skin; b) ultrasonically facilitating delivery of said material to a target volume within the skin of the subject through interaction of a portion of the enhanced formulation adapted and configured to facilitate ultrasound transport of said material; and c) exposing said light absorbing material to a series of light pulses to heat the material and thermally damage the target volume to achieve a therapeutic effect.

In general, in one embodiment, a method of treating or ameliorating a follicular skin disease of a subject, the method including a) topically applying a particle formulation, a modified particle formulation or an enhanced particle formulation comprising a sub-micron particle comprising a light absorbing material to a subject's skin; b) facilitating delivery of said material from the skin into a hair follicle by acoustically created microjets in the formulation; and c) exposing said sub-micron particle to energy activation, thereby treating the follicular skin disease.

In general, in one embodiment, a method of treating or ameliorating a follicular skin disease of a subject, the method including a) exposing the subject's skin to a formulation, a particle formulation, a modified particle formulation or an enhanced particle formulation; b) facilitating delivery of said material from the skin into a hair follicle by low frequency ultrasound induced cavitation within the particle formulation, the modified particle formulation or the enhanced particle formulation near the surface of the skin adjacent to the hair follicle; and c) exposing said sub-micron particles to energy activation, thereby treating the follicular skin disease.

In general, in one embodiment, a method of facilitating delivery of a light absorbing material to a target volume within the skin of a subject, the method including a) topically applying a particle formulation, a modified particle formulation or an enhanced particle formulation to a subject's skin to deliver the material to a reservoir within the target volume of the skin; b) facilitating an ultrasonic delivery mode of said material to a target volume within the skin of the subject substantially via a transfollicular pathway; and c) exposing said light absorbing material to a series of light pulses to heat the material and thermally damage the target volume to achieve a therapeutic effect.

In general, in one embodiment, a method of treating or ameliorating a follicular skin disease of a subject, the method including a) topically applying a particle formulation, a modified particle formulation or an enhanced particle formulation comprising particles of a light absorbing material to a subject's skin; b) acoustically cavitating the particle formulation, the modified particle formulation or the enhanced particle formulation for selectively facilitating delivery of said particles in the formulation into a sebaceous gland primarily through the corresponding hair follicle; and c) irradiating said particles with light to treat the follicular skin disease.

In general, in one embodiment, a method of treating or ameliorating a follicular skin disease of a subject, the method including a) topically applying a particle formulation, a modified particle formulation or an enhanced particle formulation to a subject's skin; b) facilitating delivery of said material into a sebaceous gland using immersion cavitation of the particle formulation, the modified particle formulation or the enhanced particle formulation; and c) exposing said sub-micron particles to energy activation, thereby treating the follicular skin disease.

In general, in one embodiment, a method of treating or ameliorating a follicular skin disease of a subject, the method including a) topically applying a particle formulation, a modified particle formulation or an enhanced particle formulation to a subject's skin; b) delivering said particle formulation, said modified particle formulation or said enhanced particle formulation into one or more sebaceous glands substantially via a transfollicular pathway; and c) exposing said sub-micron particles to energy activation, thereby treating the follicular skin disease.

In general, in one embodiment, a method of treating or ameliorating a follicular skin disease of a subject, the method including a) topically applying a particle formulation, a modified particle formulation or an enhanced particle formulation to a subject's skin; b) facilitating delivery of said material into a hair follicle by low frequency ultrasound induced cavitation near the surface of the skin adjacent to the hair follicle; and c) treating or ameliorating the follicular skin disease adjacent to the sub-micron particle using heat produced by irradiating said sub-micron particle with light.

In general, in one embodiment, a method of treating or ameliorating a follicular skin disease in a subject, the method including a) topically applying a formulation comprising particle, nanoparticle, or microparticle, particle formulation, modified particle formulation or enhanced particle formulation comprising an energy activatible material to the subject's skin; b) facilitating delivery of said material into a hair follicle, sebaceous gland, sebaceous gland duct, or infundibulum of the skin by mechanical agitation, acoustic vibration, ultrasound, alternating suction and pressure, or microjets; and c) exposing said energy activatable material to energy activation, thereby treating or ameliorating the follicular skin disease in the subject.

This and other embodiments can include one or more of the following features. In one aspect, a portion the particle formulation, modified particle formulation or enhanced particle formulation can be provided to increasing the effectiveness of facilitating delivery into a hair follicle, sebaceous gland, sebaceous gland duct, or infundibulum of the skin by enhancing the effectiveness of mechanical agitation, acoustic vibration, ultrasound, alternating suction and pressure, or microjets for transporting a portion of the energy activatible material into a hair follicle, sebaceous gland, sebaceous gland duct, eccrine gland or infundibulum of the skin. In another aspect, the particle formulation, modified particle formulation or enhanced particle formulation can include a first therapeutic portion and a second transport portion the first therapeutic portion can include one or more particles or properties selected for performing a therapy and the second transport portion can include one or more particles or properties selected for enhancing a transport mode for delivery of a portion of the first therapeutic portion to one or more target volumes within the skin. In a further aspect, the transport mode for delivery of a portion of the first therapeutic portion can include mechanical agitation, acoustic vibration, ultrasound, alternating suction and pressure, or microjets. In an alternative aspect, the particles or materials within the second transport portion can be also energy activatable materials that can be activated to the same degree as the activation of the particles in the first therapeutic portion. In yet another aspect, the particles or materials within the second transport portion can be also energy activatable materials that are unactivated or less activated or responsive as the activation of the particles in the first therapeutic portion. In still another aspect, a portion of the particles within the first therapeutic portion can have a shape selected from spheres, ovals, cylinders, squares, rectangles, rods, stars, tubes, pyramids, stars, prisms, triangles, branches, plates or a shape including a planar surface. In one aspect, a portion of the particles within the second transport portion can have a shape selected from spheres, ovals, cylinders, squares, rectangles, rods, stars, tubes, pyramids, stars, prisms, triangles, branches, plates or a shape including a planar surface. In another aspect, the shape of the particles in a first therapeutic portion can be different from the shape of the particles in the second transport portion. In a further aspect, the shape of the particles in a first therapeutic portion can be the same shape of a portion of the particles in the second transport portion. In an alternative aspect, the size and shape of the particles in a first therapeutic portion can be different from the shape of the particles in the second transport portion. In yet another aspect, the size and shape of the particles in a first therapeutic portion can be the same shape of a portion of the particles in the second transport portion. In still another aspect, the particle, nanoparticle, or microparticle, particle formulation, modified particle formulation or enhanced particle formulation the nanoparticles can include one or more particles, nanoparticles, or microparticles having a shape including spheres, ovals, cylinders, squares, rectangles, rods, stars, tubes, pyramids, stars, prisms, triangles, branches, plates or a shape including a planar surface. In one aspect, the particle, nanoparticle, or microparticle, particle formulation, modified particle formulation or enhanced particle formulation can include one or a plurality of nanoplates, solid nanoshells, hollow nanoshells, hollow or solid nanorods, nanorice, nanospheres, nanofibers, nanowires, nanopyramids, nanoprisms, nanoplates. In another aspect, the composition of the particle, nanoparticle, or microparticle, particle formulation, modified particle formulation, enhanced particle formulation, first therapeutic portion or transport delivery portion can be initially an ordered array and can be converted to a disordered array after performing a step of facilitating delivery of a material. In a further aspect, the composition of the particle, nanoparticle, or microparticle, particle formulation, modified particle formulation, enhanced particle formulation, first therapeutic portion or transport delivery portion can be initially ordered and remains ordered after performing a step of facilitating delivery of a material. In an alternative aspect, the composition of the particle, nanoparticle, or microparticle, particle formulation, modified particle formulation, enhanced particle formulation, first therapeutic portion or transport delivery portion can be initially assembled and can remain assembled after performing a step of facilitating delivery the particle formulation. In yet another aspect, the composition of the particle, nanoparticle, or microparticle, particle formulation, modified particle formulation, enhanced particle formulation, first therapeutic portion or transport delivery portion can be initially assembled and can be converted to an unassembled material after performing a step of facilitating delivery of a material. In still another aspect, the composition of the particle, nanoparticle, or microparticle, particle formulation, modified particle formulation, enhanced particle formulation, first therapeutic portion or transport delivery portion can include a macrostructure from individual parts that can be patterned or unpatterned initially and can remain patterned or unpatterned after performing a step of facilitating delivery the particle formulation. In one aspect, the pattern can include a macrostructure of individual particles. In another aspect, the macrostructure can be in the form of one of spheres, colloids, beads, ovals, squares, rectangles, fibers, wires, rods, shells, thin films, planar surface or combinations thereof. In a further aspect, the composition of the particle, nanoparticle, or microparticle, particle formulation, modified particle formulation or enhanced particle formulation can include a metal, metallic composite, metal oxide, metallic salt, intermetallic, electric conductor, electric superconductor, electric semiconductor, dielectric, or quantum dot that is coated, uncoated or partially coated. In an alternative aspect, the composition of the particle, nanoparticle, or microparticle, particle formulation, modified particle formulation or enhanced particle formulation can include gold, silver, silver and silica, gold and silica, iron oxide, titanium oxide, potassium oxalate, strontium chloride, titanium aluminide, alnico, copper, aluminum, yttrium barium copper oxide, bismuth strontium calcium copper oxide, silicon, germanium, silica, plastic, zinc sulfide, or cadmium selenium that is coated, uncoated or partially coated. In yet another aspect, the composition of the particle, nanoparticle, microparticle, particle formulation, modified particle formulation or enhanced particle formulation one or a combination of gold, silver, nickel, platinum, titanium, palladium, silicon, galadium. In still another aspect, the composition of the particle, nanoparticle, microparticle or particle formulation, modified particle formulation or enhanced particle formulation can include a composite including a metal and a dielectric, a metal and a semiconductor, or a metal, semiconductor and dielectric. In still another alternative, there is a method of treating or ameliorating a follicular skin disease in a subject by topically applying a formulation comprising particle, nanoparticle, or microparticle, particle formulation, modified particle formulation or enhanced particle formulation comprising an energy activatible material to the subject's skin; facilitating delivery of said material into a hair follicle, sebaceous gland, sebaceous gland duct, or infundibulum of the skin by mechanical agitation, acoustic vibration, ultrasound, alternating suction and pressure, or microjets; and exposing said energy activatable material to energy activation, thereby treating or ameliorating the follicular skin disease in the subject. In some variations of the method above, a portion the particle formulation, modified particle formulation or enhanced particle formulation is provided to increasing the effectiveness of facilitating delivery into a hair follicle, sebaceous gland, sebaceous gland duct, or infundibulum of the skin by enhancing the effectiveness of mechanical agitation, acoustic vibration, ultrasound, alternating suction and pressure, or microjets for transporting a portion of the energy activatible material into a hair follicle, sebaceous gland, sebaceous gland duct, eccrine gland or infundibulum of the skin. In one alternative, the particle formulation, modified particle formulation or enhanced particle formulation comprising a first therapeutic portion and a second transport portion wherein the first therapeutic portion includes one or more particles or properties selected for performing a therapy and the second transport portion includes one or more particles or properties selected for enhancing a transport mode for delivery of a portion of the first therapeutic portion to one or more target volumes within the skin. In still another alternative, the transport mode for delivery of a portion of the first therapeutic portion includes mechanical agitation, acoustic vibration, ultrasound, alternating suction and pressure, or microjets. In still further variations, the particles or materials within the second transport portion are also energy activatable materials that are activated to the same degree as the activation of the particles in the first therapeutic portion. In yet another variation, the particles or materials within the second transport portion are also energy activatable materials that are unactivated or less activated or responsive as the activation of the particles in the first therapeutic portion. In still another alternative, an activation energy or light activation source is a Q-switched laser operated to provide a selective therapy using a pulse duration from 0.1 to 100 ns for treatment of acne in a bearded skin region. The method of therapy as described herein is performed for the treatment of acne in a region of skin having beard hair where the acne is treated and the beard hair is not permanently removed or harmed. In still other embodiments, there is described a variety of compositions one or more particles, nanoparticles, or microparticles, or particle formulations, modified particle formulations, enhanced particle formulations, first therapeutic portions or transport delivery portions encompassing the use of delivery of light absorbing particles and molecules followed by irradiation with an appropriate source, such as near-IR wavelengths. It is believed that the typical pulse durations (i.e., in a range of 1 to 1,000 ms.) are effective in hair removal. However, in some treatment or therapy situations, additional selectivity to one target volume over another target volume may be beneficial in some patient populations. In one embodiment, there is provided one or more particles, nanoparticles, or microparticles, or particle formulations, modified particle formulations, enhanced particle formulations, first therapeutic portions or transport delivery portions selected so as to enable the selectivity of one tissue structure over an adjacent tissue structure. The selectivity could be accomplished using a variety techniques. One may select different light source, different materials or a modification to a level of transport effectiveness. In one aspect, an enhanced transport mode level may result in deeper penetration of activatable materials or, put another way, a more complete transport mode or a series of transport modes, or additional transport steps in the absence of additional activatable materials may reduce materials within more shallow regions of a target tissue volume. Conversely, reducing the transport mode or adjusting the transport mode for shallower penetration into a targeted tissue volume may be used to be more selective to treating shallow structure over deeper tissue volumes.

An additional variation as to being selective between adjacent tissue volumes includes adjustments to one or more steps of a method or adjustment of characteristics to a particle or a particle formulation so as to provide a desired or primary therapeutic effect while minimizing or reducing an undesired effect or an undesired side effect. In one aspect, there is provided a method for treating acne in a portion in a bearded area of a male wherein the side effect to be avoided is the removal of the beard hair. In one aspect, a bearded region is treated with a Q-switched laser at the same wavelengths for activation of the activatable material selected. In one aspect, the Q-switched laser is operated to provide a selective therapy using a pulse duration from 0.1 to 100 ns. It is believed that light activation energy will still be absorbed by the deposited energy activatable materials or light absorbers and lead to damage to the sebaceous units. Advantageously, such pulse durations do not lead to long term hair removal thereby avoiding the undesired side effect of loss of beard hair while treating acne in a bearded portion of a tissue volume.

In still another alternative, there is a method of treating or ameliorating a follicular skin disease in a subject by topically applying a formulation comprising particle, nanoparticle, or microparticle, particle formulation, modified particle formulation or enhanced particle formulation comprising an energy activatible material to the subject's skin; facilitating delivery of said material into a hair follicle, sebaceous gland, sebaceous gland duct, or infundibulum of the skin by mechanical agitation, acoustic vibration, ultrasound, alternating suction and pressure, or microjets; and exposing said energy activatable material to energy activation, thereby treating or ameliorating the follicular skin disease in the subject. In some variations of the method above, a portion the particle formulation, modified particle formulation or enhanced particle formulation is provided to increasing the effectiveness of facilitating delivery into a hair follicle, sebaceous gland, sebaceous gland duct, or infundibulum of the skin by enhancing the effectiveness of mechanical agitation, acoustic vibration, ultrasound, alternating suction and pressure, or microjets for transporting a portion of the energy activatible material into a hair follicle, sebaceous gland, sebaceous gland duct, eccrine gland or infundibulum of the skin. In one alternative, the particle formulation, modified particle formulation or enhanced particle formulation comprising a first therapeutic portion and a second transport portion wherein the first therapeutic portion includes one or more particles or properties selected for performing a therapy and the second transport portion includes one or more particles or properties selected for enhancing a transport mode for delivery of a portion of the first therapeutic portion to one or more target volumes within the skin. In still another alternative, the transport mode for delivery of a portion of the first therapeutic portion includes mechanical agitation, acoustic vibration, ultrasound, alternating suction and pressure, or microjets. In still further variations, the particles or materials within the second transport portion are also energy activatable materials that are activated to the same degree as the activation of the particles in the first therapeutic portion. In yet another variation, the particles or materials within the second transport portion are also energy activatable materials that are unactivated or less activated or responsive as the activation of the particles in the first therapeutic portion.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:
FIG. 1 is a section view of a delivery device having a housing and a cup assembly.
FIG. 1A is a section view of the cup assembly of FIG. 1.
FIG. 1B is a section view of the housing or handle of FIG. 1.
FIG. 2 is a section view of the delivery device of FIG. 1 with a modified housing to provide a gap between the housing and the ultrasound converter.
FIG. 3 is a section view of the delivery device of FIG. 1 with a modified ultrasound converter to provide a gap between the housing and the ultrasound converter.
FIG. 4 is a schematic view of a representative control system for a delivery device.
FIG. 5 is a flow chart of an exemplary method of operating a delivery device.
FIG. 6 is a section view of a cup assembly adapted and configured for vacuum seal and with a port to inject a delivery fluid.
FIG. 7 is a section view of a cup assembly adapted as in FIG. 6 also including a cooling conduit and loop.
FIG. 8A is a section view of a cup assembly modified to show a pair of seals along the cup assembly lower surface.
FIGs. 8B and 8H are section views of the cup assembly of FIG. 8A having multiple V-shaped sealing surface features.
FIGs. 8C and 8I are section views of a seal having a plurality of U-shaped sealing surface features.
FIGs. 8D and 8G are section views of a single rounded or U-shaped sealing feature.
FIGs. 8E and 8J are section views of a sealing surface having a pair of O rings fitted within a recess in the lower surface of the cup assembly.
FIG. 8F is a simplified section view of a cup assembly indicating the area of detail for the various sealing detail views.
FIG. 9 is a section view of the housing or handle of FIG. 1B modified to provide a flexible seal about the ultrasound horn.
FIG. 10 is a section view of an ultrasound horn and cup assembly showing a flexible gasket and clamp around the horn and cup.
FIGs. 11, 12, 13, and 14 are various views of one embodiment of a cup assembly.
FIG. 12 is a bottom up view of the cup assembly in FIG. 11.
FIGs. 13 and 14 are side and isometric views of the cup assembly in FIG. 11 with the gasket removed.
FIGs. 15 through 23 are various views of the top inner cup assembly of a two part cup assembly adapted and configured to operate with the second part or bottom outer cup of the two part assembly shown in the various views of FIGs. 24 to 32.
FIGs. 24 to 32 are various views of the second part or bottom outer cup of the two part housing shown in FIGs. 36 and 37.
FIGs. 33-37 illustrate top down, section A-A, section B-B and first and second isometric views respectively of an embodiment of a two part cup assembly from the assembled two part cup assembly portions illustrated in FIGs. 15 through 32.
FIGs. 38A to 41 illustrate various views of a delivery device having the two part cup assembly of FIGs. 36 and 37.
FIG. 38B illustrates various views of the housing in FIG. 38 including isometric views, exterior views and cross section views.
FIG. 42 is an exploded view of a prototype delivery device.
FIG. 43 is an assembled view of the delivery device of FIG. 42.
FIG. 44 is an isometric view of an exploded view of the handle and housing assembly.
FIG. 45 is an isometric view of the handle assembly of FIG. 44 assembled about the components of FIG. 43. Also shown in FIG. 45 is the calibration disc in position adjacent to the cup assembly.
FIG. 46 is an isometric view of the delivery device of FIG. 45 with the calibration disc in place.
FIG. 47 is an isometric view of the delivery device after use of the calibration disc.
FIG. 48 illustrates a section view of two representative ultrasound horn diameter devices in position over a tissue site (T) having a radius (r).
FIG. 49 illustrates a graph showing performance and perception as a function of skin-horn distance and ultrasound horn amplitude.
FIGs. 50A and 50B illustrate A1 foil showing pitting under continuous ultrasound application of ultrasound.
FIGs. 51A and 51B illustrate A1 foil showing pitting under pulsed application of ultrasound.
FIGs. 52A-52C illustrate A1 foil showing pitting under continuous ultrasound application of ultrasound.
FIGs. 53A-53C illustrate A1 foil showing pitting under pulsed application of ultrasound.
FIGs. 54A-54C illustrate A1 foil showing pitting under pulsed application of ultrasound.

### DETAILED DESCRIPTION

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements, these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

It is to be appreciated that this applications describes a multi-function ultrasound applicator for use with a wide range of particle formulations in a number of different ultrasound transport modes for delivery of therapeutic particles into target volumes within a human or animal body. The variety of particle formulations includes nanoparticle formulations, modified nanoparticle formulations as well as enhanced nanoparticle formulations. In some particle or nanoparticle formulations there are provided particle formulations having a first portion of particles selected based on therapeutic factors (i.e., optical properties in the case of a light therapy) and a second portion of particles selected based on one or more characteristics to enhance the overall or selected ultrasound transport characteristics of the formulation to reach a pre-selected target tissue volume. Still further, as to the second portion of particles, there may particle response characteristics either partially or completely within or completely without of a therapeutic response range or response characteristics of the first portion (i.e., the second portion may or may contribute to the therapeutic process, dose or method performed using the therapeutic response of the first portion or the therapeutic portion of the formulation. In some embodiments, there is a portion of the formulation provided for the therapeutic effect and another or second portion provided for the purpose of aiding the first portion to reach the desired target site. One exemplary transport mode includes a second portion of particles selected to assist in delivery to one or more of a sweat gland, an eccrine sweat gland or one or more coiled structures of a gland in the dermis.

In the exemplary embodiments that follow, the shortcomings of the conventional ultrasound applicators are addressed with a number of improvements and modifications. In one specific aspect, there are described methods and apparatus to address the translation of the applicator without spill or leakage. In another aspect, there are provided a method and apparatus to addresses temperature control or monitoring of the delivery fluid and/or the environment of the delivery cup.

The general, the ultrasound applicator described herein consists of a vibrating ultrasound horn immersed in a delivery fluid. Under the proper conditions, vibration of the horn in the delivery fluid leads to formation and collapse of cavitation bubbles and acoustic streaming. The collapse of the bubbles near the fluid-skin interface generates microjects directed toward skin which assist in driving material into skin via transdermal or transfollicular routes of entry. Also, acoustic streaming is also believed to assist in driving of the material into skin. The ultrasound frequency is in the range of 20 kHz to 200 kHz. The distance between the horn and skin is in the range of 1 mm to 30 mm. The diameter of the horn is in the range of 2 mm to 30 mm. The distal surface of the horn can be, for example, flat, convex, or concave. The ultrasound horn may be made from any suitable biocompatible material such as, for example, titanium, aluminum, ceramic, stainless steel or combinations thereof. The ultrasound applicator conditions may be adjusted to ensure proper adjustment of the cavitation and/or acoustic streaming characteristics such that a portion of the delivery fluid or particles within the delivery fluid preferentially enter into and penetrate to reside within a portion of a pilosebaceous unit or otherwise translate through the skin via a transfollicular pathway.

In general terms, there is provided a delivery device having a housing with an ultrasound horn and a cup assembly mated with the housing. Within the housing, a converter converts the electrical driving signal to vibrate an ultrasound horn. The horn is immersed in a fluid which is contained in the interior space of the cup assembly. In one specific embodiment, the diameter of the interior space of the cup assembly is about 2-6 mm greater than the horn diameter. In another aspect, the cup assembly interior diameter is selected to reduce/eliminate dampening of the ultrasound horn and/or to provide freedom of movement of the ultrasound horn relative to the cup assembly. The cup assembly is attached to the horn via the housing. In some aspects, the housing or the ultrasound converter have been modified in order to minimize contact between or establish a gap between the ultrasound system components and the interior walls of the housing and the cup assembly. As described further below, ports are added for example, to enable introduction and/or removal a delivery fluid or to provide access for a probe or measurement device to monitor conditions within the delivery environment of the cup assembly. In one example, a temperature monitoring probe is provided.

In one exemplary embodiment, in order to achieve a seal between the cup assembly and the target site, an annular ring is included in the distal end of the cup assembly in which negative pressure is drawn. The vacuum draws the skin into the ring and makes a seal to avoid spillage of the fluid contents, either during single-spot operation or during translation. In still further alternatives, the distal end of the cup assembly can be made of flexible material so translation across a non-planar anatomical location is possible while not compromising the seal. In an additional aspect, channels near the cup assembly interior surface are added and fluid at appropriate temperature as needed is driven through these channels to, for example, remove any heat generated by the ultrasound energy being delivered into the delivery fluid or formulation within the cup assembly interior portion. In another alternative embodiment, additional delivery fluid ports and conduits may be added to the cup assembly permitting circulation of the delivery fluid or formulation. In such a configuration, the delivery fluid or formulation may circulate from the cup assembly interior through one or more of a heat exchanger, a conditioning unit or a storage unit or other auxiliary system used with the envisioned delivery method.

In use, a seal is formed between the surface to be treated and the cup assembly lower surface. The seal - when formed - provides a closed system (reservoir) which uses a portion of the surface to be treated (i.e., the patient's skin) as the bottom of the reservoir. As such, the seal between the cup assembly and the skin aids allows for intimate contact between the skin and delivery formulation, while maintaining the closed system and appropriate fluid levels in the reservoir. Fluid level control/management in the reservoir is needed to provide a suitable environment for ultrasound operation including, for example, cavitation and/or acoustic streaming. Additionally, a suitable seal will also prevent unnecessary spills which could find their way into the patient's eyes, nose, ears, and mouth, even during translation of the cup assembly across a treatment site.

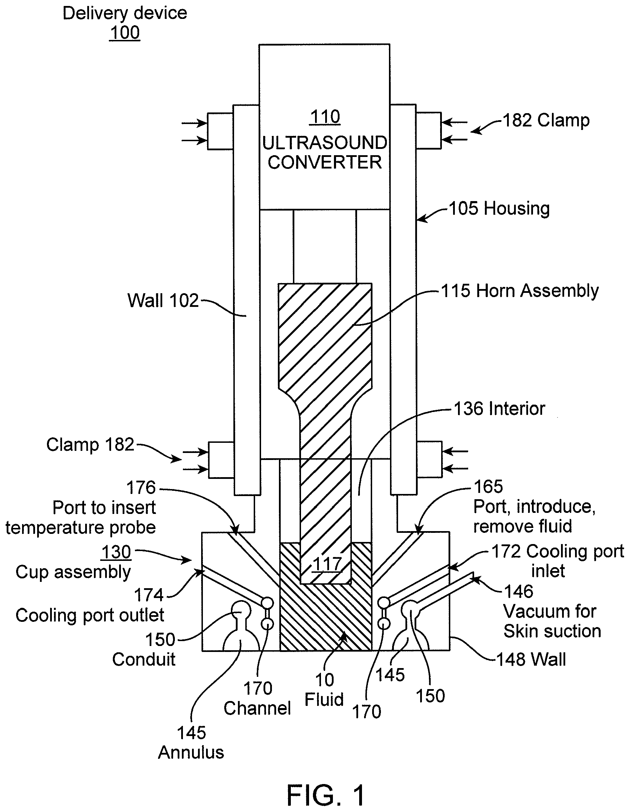

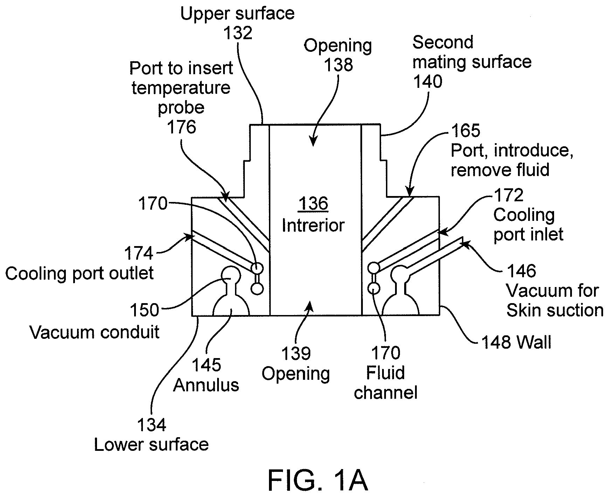

A suitable seal may be accomplished using one or more of a variety of different techniques. In one aspect, a delivery device may employ the use of vacuum to draw a portion of the target site tight with distal end of cup sufficient to prevent leakage. A suitable vacuum control system is provided for this feature such that vacuum performance is adjustable to provide patient comfort and reduction of side effects while ensuring a sufficient seal is maintained. In one aspect, as illustrated in FIGs. 1A, 6 and 7, the cup assembly includes at least one or more than one vacuum ring or annulus located immediately outside the cup assembly interior or otherwise forming a perimeter of the cup assembly lower surface. In various alterative embodiments, the width of the ring/annulus and the diameter of the cup assembly work with in conjunction with the vacuum pressure to provide the seal. In various other embodiments, described herein, changes in ring diameter, width, or amount of vacuum may also be advantageously utilized to form a seal and/or enable translation of the cup assembly while maintaining a seal.

In still other alternative aspects, the different materials may be provided in a single cup assembly to provide a seal designed for translation. For example, a cup assembly may have a rigid upper section and a flexible lower section. In another example, a cup assembly may have a lower surface having different materials more rigid to maintain the vacuum seal and other materials at the outer edges are suited to aid in translation of the cup assembly across a treatment surface. In one specific embodiment, a soft rubber skirt may be advantageous to creating and maintaining a seal. The skirt is pliable such that an effective seal is accomplished even in the presence of different skin thicknesses, geometry, and angles, e.g. (cheek vs. temple vs. forehead or in transition zones between). Considerations for a suitable flexible skirt or bottom surface flange include, for example: the length of skirt (selected to seal without deformation), the structure of skirt (selected to seal without deformation or collapse as the skirt, if used, would also assist in regulating the skin to horn distance); durometer (hardness) of skirt material; the type of skirt material - for example, a material selected for an adequate seal but with sufficient lubricity to move over skin under mild pressure; coefficient of friction of the skirt material or a portion thereof such that material can be moved across skin easily while maintaining the seal without leakage. In still another aspect, a seal material is selected which is impregnated with a lubricant which leaches the substance reducing friction.

As mentioned above, one advantage of the alterative embodiments described herein is added functionality of translation or translatability of the cup assembly over a target area. The purpose for translation is to cover a treatment in the shortest amount of time while maintaining the fluid seal. In this regard, one considers a variety of factors such as the different types of skin, thickness of skin, variations in substructure (i.e., forehead vs. cheek including proximity of underlying bone and subcutaneous fat thickness), geometry, contour angles, and transition zones between different areas or treatment. Many of the additional details above for an exemplary skirt may also be considered here as relating to the moving seal challenge posed during translation. Such factors as the size, length, structure, hardness, and coefficient of friction of the materials and cup assembly will be considered. In addition, the size of the cup assembly may also play a role not only in ability to reach smaller structures such as lips and nose, but also in effecting a seal. The smaller area the easier it would be to create and maintain a seal on a curved surface. Testing was performed using a 13mm diameter ultrasound horn (and associated cup assembly) however smaller or larger diameter horns could be used as described herein.

In still further additional or alternative aspects, there is provided reservoir Temperature Monitoring and Control. It is to be appreciated that the term reservoir is used herein to refer to the reservoir found by coupling the cup assembly to a delivery site. The terms reservoir in the context of a cup assembly not contacting a surface refers to the cup assembly interior. There are several reasons for temperature control. First is safety, but there also may be some advantage in delivery at or within a particular temperature range, e.g., warmed tissue may be more receptive to delivery fluid or a controlled temperature fluid penetration.

In one embodiment, the temperature in the reservoir is monitored via a thermocouple contained in a stainless steel sleeve which is in direct contact with the reservoir fluid. In one aspect, a temperature controlled fluid may be circulated through a loop in the cup assembly. The controlled temperature fluid may be circulated in the loop to adjust the temperature of the cup assembly and the fluid contained therein. In one embodiment, fluid cooling may be accomplished via an active cooling system with a temperature exchange radiator machined into the cup assembly housing.

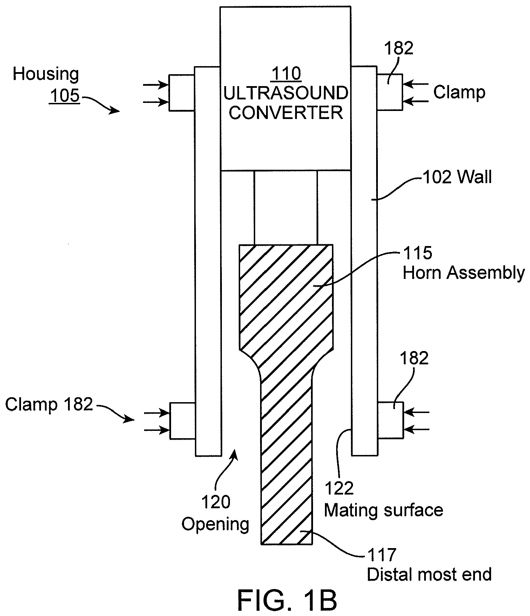

FIG. 1, FIG. 1A, and FIG. 1B considered collectively illustrate a delivery device 100 having a housing 105 and a cup assembly 130. The housing 105 includes an ultrasound converter 110 within and supported by the housing 105. There is also an ultrasound horn 115 coupled to the ultrasound converter 110 and extending through an opening 120 in the housing 105. There is a first mating surface 122 in the housing adjacent to the opening 120. The cup assembly 130 has an upper surface 132 and a lower surface 134. The cup assembly has a generally cylindrical interior portion 136 extending between an opening 138 in the upper surface 132 and an opening 139 in the lower surface 134. A second mating surface 140 is positioned adjacent to cup assembly upper surface where the first mating surface 122 is coupled to the second mating surface 140, a distal most portion 117 of the ultrasound horn 115 is disposed within the generally cylindrical anterior portion 136 without contacting the cup assembly 130.

The delivery device 100 illustrated in FIG. 1B and FIG. 1 illustrate an ultrasound horn 115 coupled to the ultrasound converter 110 that extends through without contacting an interior portion 136 with the cup assembly 130. Additional other details of the cup assembly may be appreciated by referenced FIGs. 1 and 1A. There is provided an inlet for vacuum suction 146 that connects a conduit 150 to an annulus 145.

Additional details of the components of the delivery device 100 may be provided with reference to FIGs. 1, 1A, and 1B. The delivery device 100 has the diameter of the opening 138 in the upper surface and the diameter of the opening 139 in the lower surface and the diameter of the generally cylindrical interior portion 136 are about the same, in some configurations. In another aspect, the first mating surface 122 is coupled to the second mating surface 140 where the distal most portion 117 of the ultrasound horn 115 is proximal to the opening 139 in the lower surface 134. In another aspect, when the first mating surface 122 is coupled to the second mating surface 140, a distal most portion 117 of the ultrasound horn 115 is proximal to and spaced about 13 mm from the opening 139 in the lower surface 134. In still another alternative, when the first mating surface 122 is coupled to the second mating surface 140, a distal most portion 117 of the ultrasound horn 115 is proximal to and spaced from about 8 mm to about 13 mm from the opening 139 in the lower surface 134. Put another way, when the first mating surface 122 is coupled to the second mating surface 140, and a surface of the cup assembly 130 (e.g., distal surface 134) forms a seal on a surface of skin to be treated, the distal most portion 117 of the ultrasound horn 115 is proximal to and spaced apart from the skin such that sufficient fluid may be introduced between the skin and the distal most portion 117 of the ultrasound horn 115 to facilitate an emergent ultrasound operation within the interior of the cup assembly 130. In addition or alternatively, the coupling of the first and second mating surfaces may also result in spacing of the ultrasound horn from the skin to facilitate a cavitation based ultrasound operation with the interior of the cup assembly 130. Additionally or alternatively, the spacing is adjustable to enhance ultrasound performance. Additionally or alternatively, the spacing may be adjusted to compensate for predicted or actual skin deflection in a treatment site as a result of application of sealing forces either from pressure of the cup assembly to the treatment site, use of vacuum assisted seals or both. In one aspect, the amount of compensation or adjustment of horn-surface spacing is adjusted by type of treatment site anatomy, such as, face, cheeks, forehead, chin, neck, back, chest, shoulders, abdomen or other portion of the skin or treatment site.

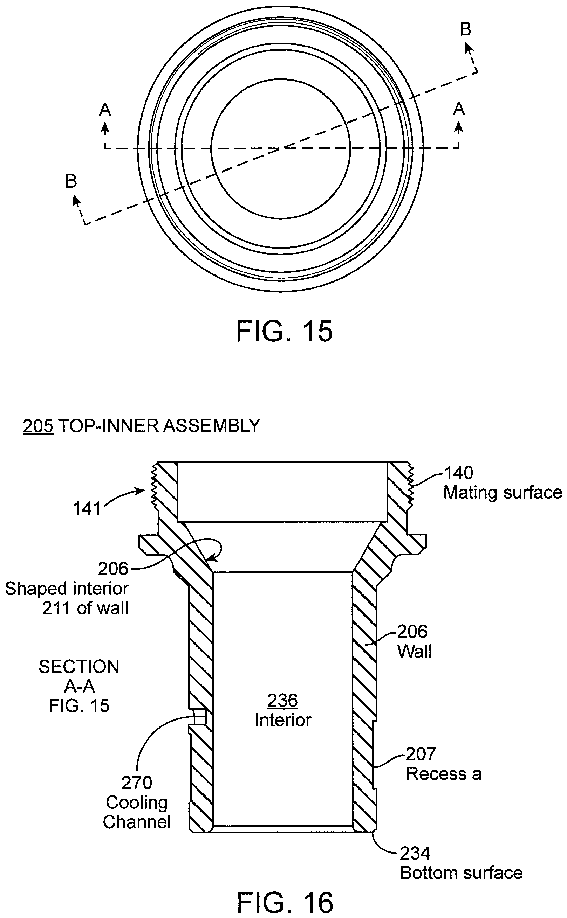

The delivery device 100 is comprised of two releasably coupled components, a housing 105 and a cup assembly 130. In one aspect, the first mating surface 122 is formed on an interior surface of the housing 105. The first mating surface 122 may be a detent and the second mating surface 140 may be a complementary shape to receive the detent when the cup assembly 130 is joined to the housing 105. In still another aspect, illustrated in various embodiments below, the first mating surface 122 is a threaded portion 123 and the second mating surface 140 has a complimentary shape 141 to mate with the threaded portion 123 (see for example FIGs. 16-22 and 38 below).

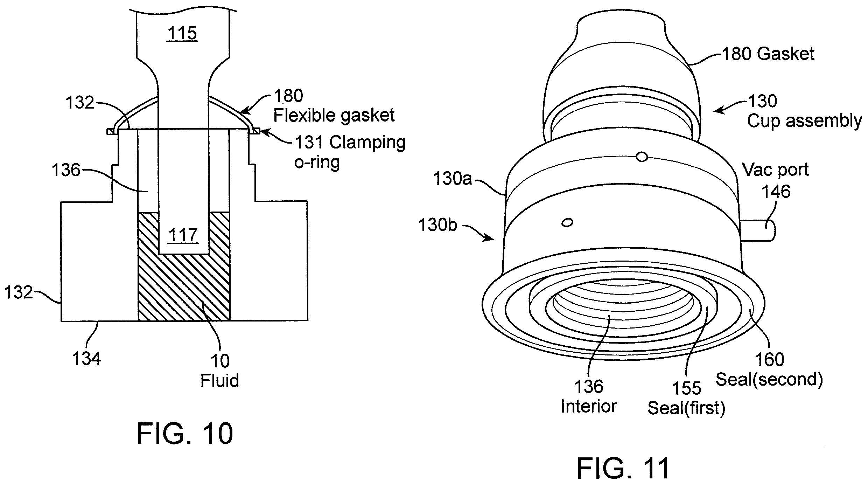

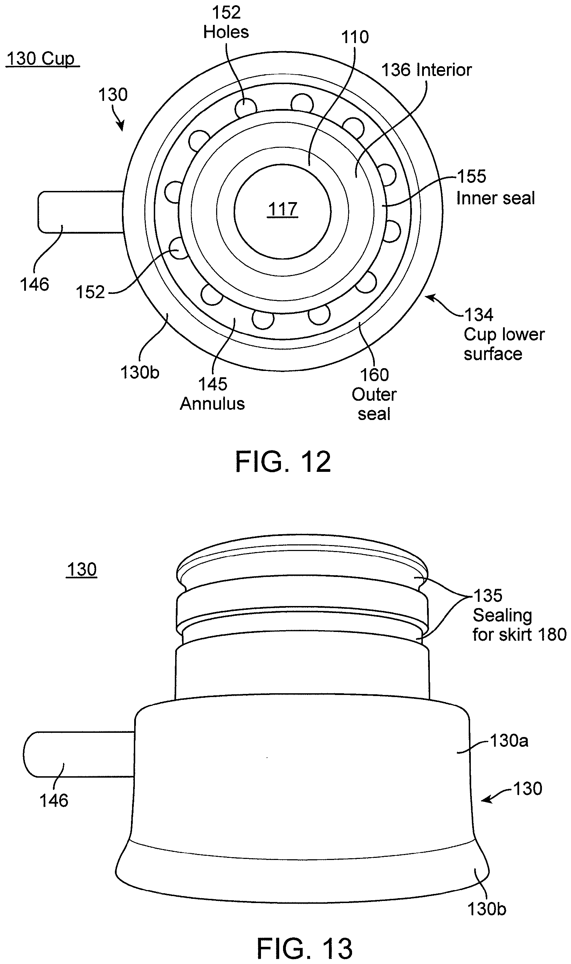

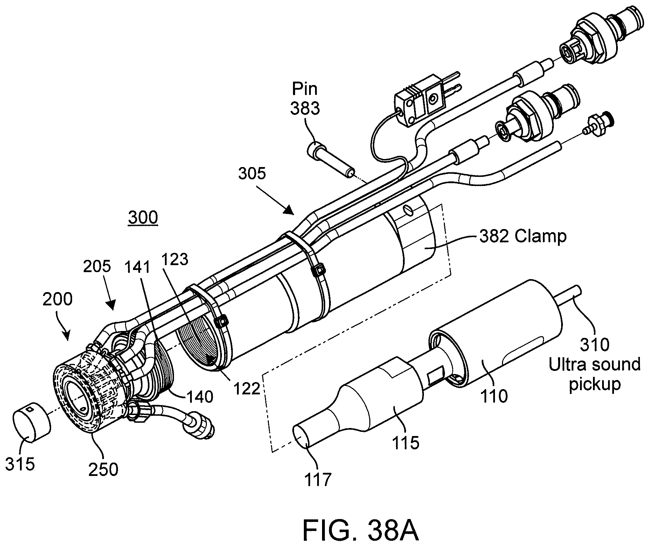

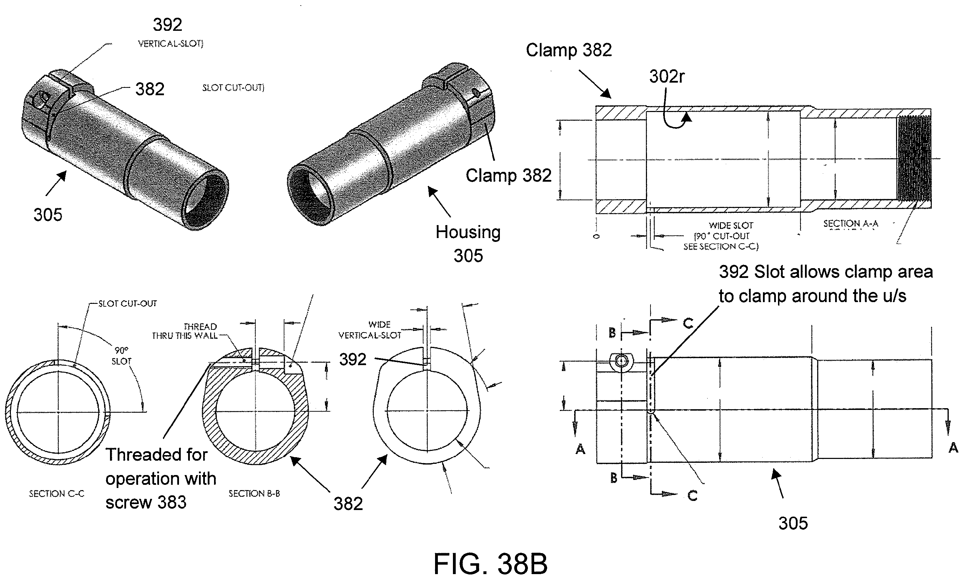

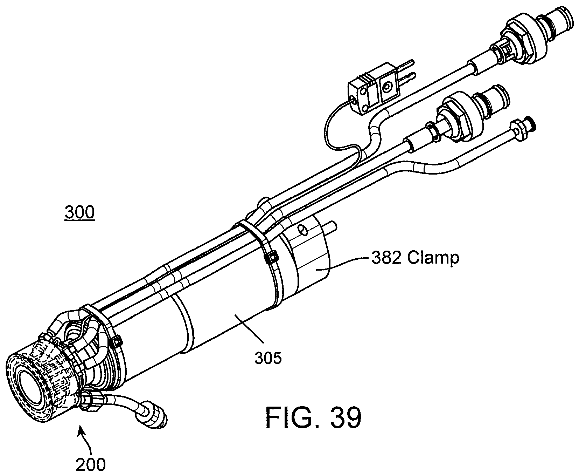

The housing and cup assembly connection may be provided using any number of coupling arrangements. The housing and cup assembly may be provided with compressing fitting components, or quick release clamps (see e.g., modification to claim in FIGs. 38, 39) or snap on fittings (see e.g., connection of gasket 180 in FIG. 11 to grooves in FIG. 13, or other suitable coupling devices such as an interlocking lug system. In still another alternative, the cup assembly 130 and the housing 105 may be joined by the use of the first mating surface 122 is sized for a friction fit with the second mating surface 140. Alternatively, the first mating surface 122 is attached to an anterior surface of the housing 105. In still another aspect, the first mating surface 122 and the second mating surface 140 form a pin and groove pair. In still another aspect, the cup assembly 130 and the housing 105 may be joined whereby less than a full rotation between the housing 105 and the cup assembly 130 joins the first mating surface 122 to the second mating surface 140.

In addition to the above described features, the cup assembly 130 also includes an annulus 145 formed in the cup assembly lower surface 134. There is also an opening 146 in a wall 148 of the cup assembly 130 providing access to a suitable vacuum system. In one exemplary implementation of the delivery device, the vacuum system in conjunction with the cup assembly is used to apply a vacuum seal (e.g., skin to cup assembly) ranging from about -0.8 atm to about -0.1 atm; from about -0.5 atm to about - 0.1 atm; or from about -0.33 atm to about -0.1 atm.

The vacuum functionality of the cup assembly also includes a conduit 150 in communication with the opening 146 and a portion of the cup assembly 130 as best seen in FIG. 1A. In one aspect, the portion of the cup assembly is an annulus 145. In one embodiment, the annulus 145 has a depth of about 1 mm to about 4 mm. In another aspect, the annulus 145 has a radius of curvature as illustrated in FIG. 1 and FIG. 1A. In still another aspect, the annulus 145 has a generally semicircular cross section shape. In still another aspect and as further described in the alternative embodiments that follow, the annulus also includes a plurality of holes 152 formed in the annulus 145 and in communication with the conduit 150 (see, e.g., FIG. 12). In another aspect, there is a portion of the cup assembly directly adjacent to a portion of the annulus and in communication with the opening 146. In still another alternative, the annulus 145 has a generally rectangular cross section shape as shown in some of the alternative embodiments that follow. In one aspect, the opening 146 is connected to an appropriate source of vacuum using connections that are further described in the alternatives that follow.

Also illustrated in FIGs. 1 and 1A is a cup assembly 130 of the delivery device 100 that includes an inlet 165 in the cup assembly 130 in communication with the cup assembly anterior portion 136. There may also be a container in communication with the inlet 165. The inlet 165 is used for injecting delivery fluid or a formulation to be applied to the skin into the interior portion of the cup assembly 130. The container may hold a formulation of the delivery fluid for introduction into the cup assembly interior portion. In still another aspect of the delivery device 100, when the first mating surface 122 is joined to the second mating surface 140, the distal most portion of the ultrasound horn is positioned below the inlet and the cup assembly interior portion 136.

Still another functional aspect of the cup assembly 130 illustrated in FIGs. 1 and 1A is the inclusion of a sealed fluid circulation loop within the cup assembly. As shown in FIGs. 1 and 1A there is a fluid channel 170 formed within a wall of the cup assembly 130 and positioned adjacent to the opening 139 in the lower surface 134. The channel 170 is connected to an inlet port 172 and an outlet port 174 on the exterior of the cup assembly 130.

Still another functional capability of the cup assembly 130 illustrated in FIGs. 1 and 1A is an opening 176 extending through a wall of the cup assembly 130 to the anterior portion 136 of the cup assembly. A probe 178 may be disposed within the opening and in communication with the interior portion 136 or, optionally, a fluid 10 or delivery formulation contained within the cup assembly 130. While the opening may accommodate a wide variety of probe types, in one aspect the probe 178 is a temperature sensor. In still another aspect, the probe 178 is selected to monitor a parameter of a fluid such as a delivery fluid 10 or a formulation, in the cup assembly (e.g., temperature, conductivity, pH and the like) or a characteristic of the environment within the cup assembly 130.

FIGs. 1, 1A, and 1B also illustrate various aspects of the housing 105. In the illustrated embodiment a pair of clamps 182 are used on the proximal and distal ends of the housing assembly 105 to clamp to a portion of the ultrasound converter while remaining clear of the horn assembly. The distal most clamp 182 is used to ensure that the first and second mating surfaces are joined in order to couple the cup assembly 130 to the housing 105. As best seen in FIG. 1B, there is a spacing in the opening 120 of the housing 105 where the horn assembly 115 passes through and extends beyond the opening 120. While a pair of clamps 182 are illustrated in this embodiment of FIGs. 1 and 1B, only a single clamp may be used as illustrated and described below. Loosening of the clamps permits adjustable movement of the ultrasound horn relative to the housing and cup assembly to provide the desired skin-horn spacing in use.

In addition, some alternative embodiments provide for a coupling between the ultrasound components and the wall housing where a gap is maintained. A gap may be provided where needed between the housing interior and the ultrasound components to ensure ultrasound operation is unimpaired by the housing. The use of a gap aids in preventing dampening of the piezoelectric elements located in the ultrasound system interior to the housing. Other methods of minimizing or eliminating dampening interference by the housing may be employed. One example is the use a reinforcing element on the horn converter.

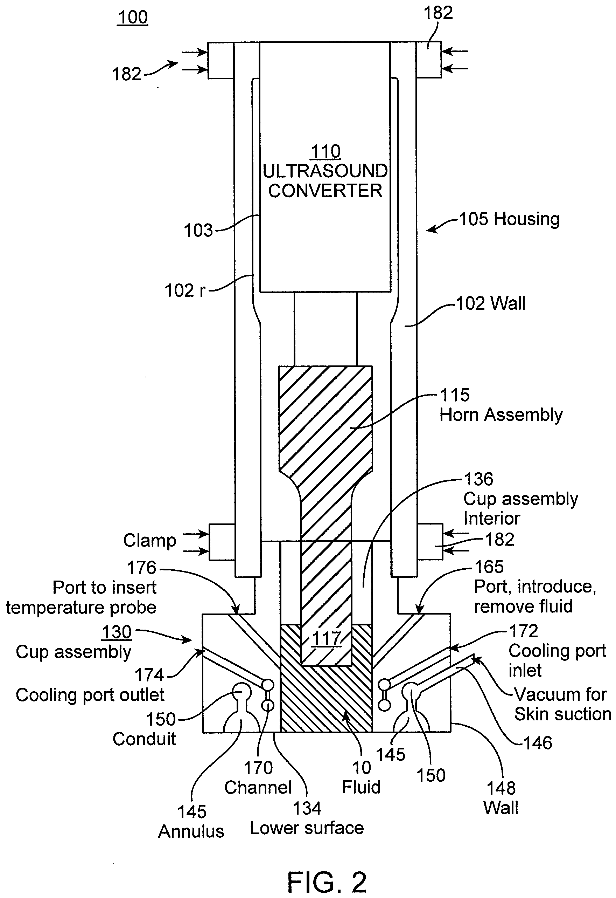

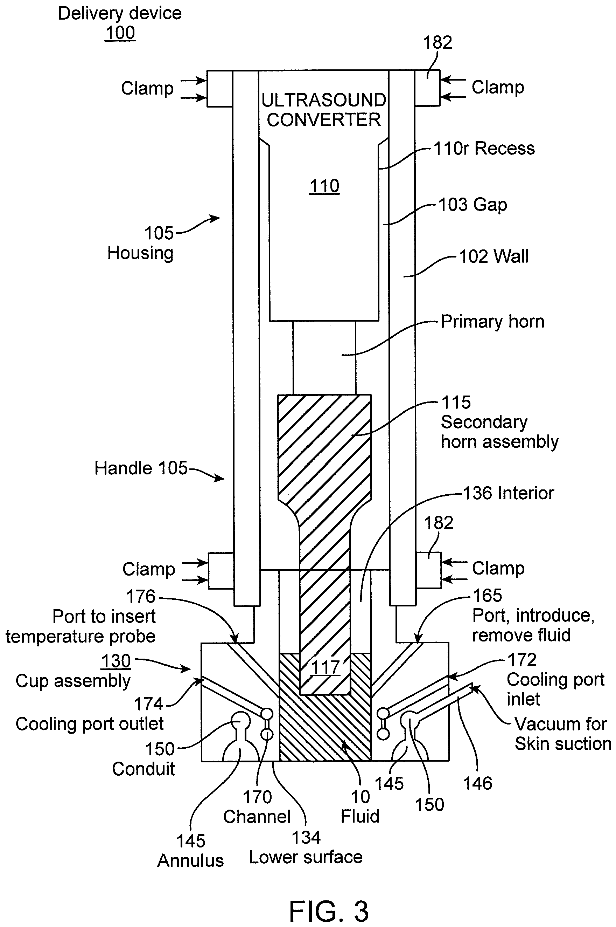

Two delivery system variations illustrating such a gap are provided in FIGs. 2 and 3. FIG. 2 illustrates a section view of the delivery device of FIG. 1 where a portion of the housing wall 102 has been recessed in a region 102r to provide a gap 103 between the housing and the ultrasound components. FIG. 3 provides an alternative configuration where instead of the wall being recessed, a portion of the ultrasound components are recessed. FIG. 3 illustrates a recessed portion 110r within the ultrasound converter 110. As a result of the recessed portion 110r or the recessed portion 102r, a gap 103 is maintained between the interior portion of the wall 102 and the ultrasound components used in the delivery device 100.

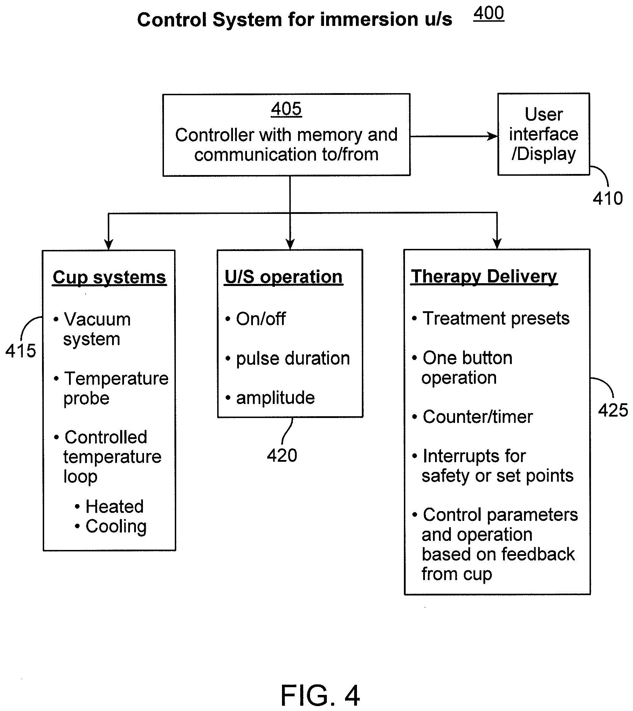

FIG. 4 is a schematic view of a representative control system 400 for use with a delivery system. The control system includes a controller 405 containing memory and other electronic components and peripherals for it to interface with and control the various other subsystems such as cup assembly systems 415, ultrasound operations 420 and therapy delivery 425. In addition, the control system 400 includes a user interface and/or display to permit user interaction with the components of the control system 400 via the controller 405 or other suitable electronic means.

Cup systems 415 includes the equipment and controls for, as appropriate to a specific delivery system, vacuum system, temperature probe or other probes (if included) as well as controlled temperature systems. Each of these systems may include pumps, circuitry and safety features appropriate to the specific system.

Ultrasound operation 420 includes all aspects of generating and delivering the desired ultrasound signal into the delivery device. This subsystem is responsible for turning the ultrasound generator on and off, controlling ultrasound parameters like pulse width, amplitude, frequency, duty cycle and any other parameter in order to provide the desired ultrasound output into the cup assembly/skin/delivery fluid environment.

Therapy delivery 425 includes the features and combination of controls for a wide variety of parameters to provide advanced features or functionality for the delivery system 100. The therapy delivery 425 would include such operation parameters or synchronized component operation to permit, for example, one button operation of the delivery system, user determined treatment presents by patient, treatment location and/or treatment regimens (i.e., first, follow up, of number in a series). Still further, the therapy delivery portion of the control system may also be used to modify or stop system operation with pre-set conditions are reached such as safety presets for temperature or vacuum. In still another aspect, the therapy delivery 425 may also provide, set or maintain one or more delivery device parameters based upon input from one or more feedback signals indicating an input from a parameter related to the operation of the delivery device or the operating environment within the cup assembly.

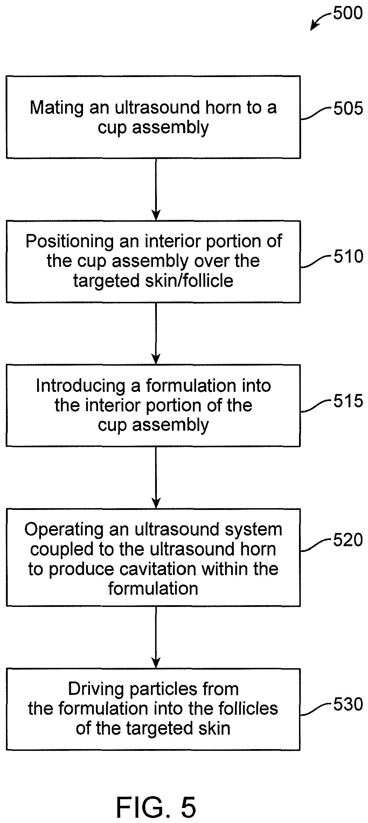

FIG. 5 is a flow chart of exemplary steps for a method for enhancing penetration of particles in a formulation into a follicle. This exemplary method may be performed using one or more of the devices or techniques described herein. First, there is a step of mating a cup assembly to an ultrasound converter housing (505). Next, there is a step of positioning an interior portion of the cup assembly over the follicle or area selected to receive the particles in the formulation (510). Next, there is a step of introducing the formulation (such as a delivery fluid 10) into the interior portion of the cup assembly (515). Thereafter, there is a step of operating an ultrasound system coupled to the ultrasound horn to produce cavitation within the formulation or delivery fluid 10 (520). Next, there is the step of driving a plurality of particles in the formulation into the follicle during the operating step (530).

The method steps outlined in method 500 are subject to modification to include operation of one or more of the auxiliary systems described herein or provided by operation of the control system 400 or instructions in the memory of controller 405. In one exemplary additional operation, there may also be added the step of applying vacuum with a portion of the cup assembly to skin surrounding adjacent the cup assembly. In addition, the applying vacuum step is performed before the introducing step. Still further, the step of applying vacuum to a portion of the skin surrounding the follicle is conducted during the operating and the driving steps. In still a further aspect, to aid cup translation, relieve vacuum in part or in whole during movement of the cup assembly from one spot to another.

In still further alternative methods of operating a delivery system described herein, there is also a step moving or translating the cup assembly across the skin during the operating and the driving steps. There may be a variety of delivery modes, spot stamping mode (multiple spots with unit off while moving from one to another, and in continuous delivery mode under continuous translation. The methods of delivery described herein may be performed in a variety of different modes alone or in any combination. Examples including spot mode, stamping mode to treat multiple spots with u/s unit off and moving from spot to spot; continuous u/s mode with or without translation and pulsed, sequential or modulated u/s mode with or without translation across a treatment area. In yet another alternative, there is also a step of circulating a temperature controlled fluid within a portion of the cup assembly. The step of circulating may be performed under guidance of instructions in the control system. In one aspect, the circulation of fluid and temperature controls are performed during at least one the introducing, the operating or the driving steps. In still another modification to the methods of operating the delivery system, there is a step of performing the circulating step based on an input from a temperature sensor providing an indication of the temperature of the skin or the formulation or fluid 10.

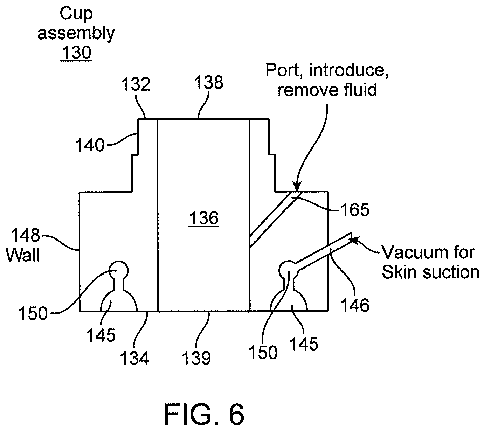

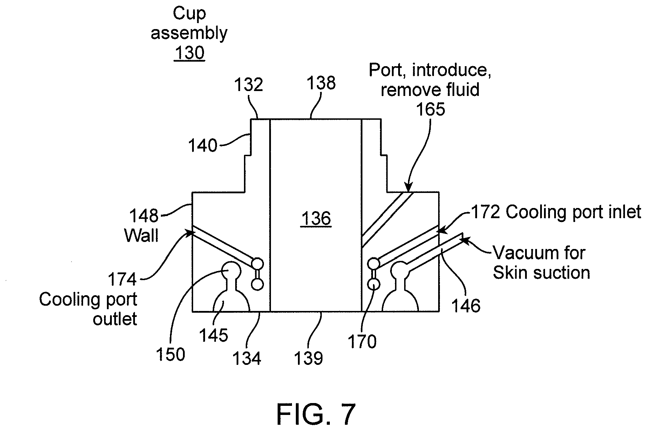

The cup assembly 130 embodiment illustrated in FIGs. 1, 1A, 2 and 3 includes a variety of functions such as fluid loops, delivery fluid injection, vacuum and monitoring. Other cup assembly embodiments may include more or fewer functions. Other functions such as, for example, additional vacuum ports and an additional annulus, additional inlets or access ports for additional instrumentation to facilitate monitoring and/or control of the environment within the cup assembly, to name a few. Alternatively, a cup assembly may also be simplified to have less functionality. FIGs. 6 and 7 illustrate two such exemplary cup assemblies.

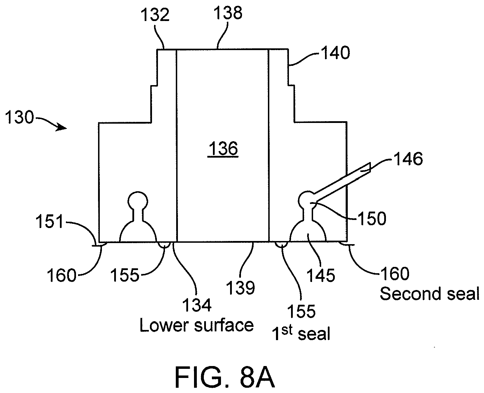

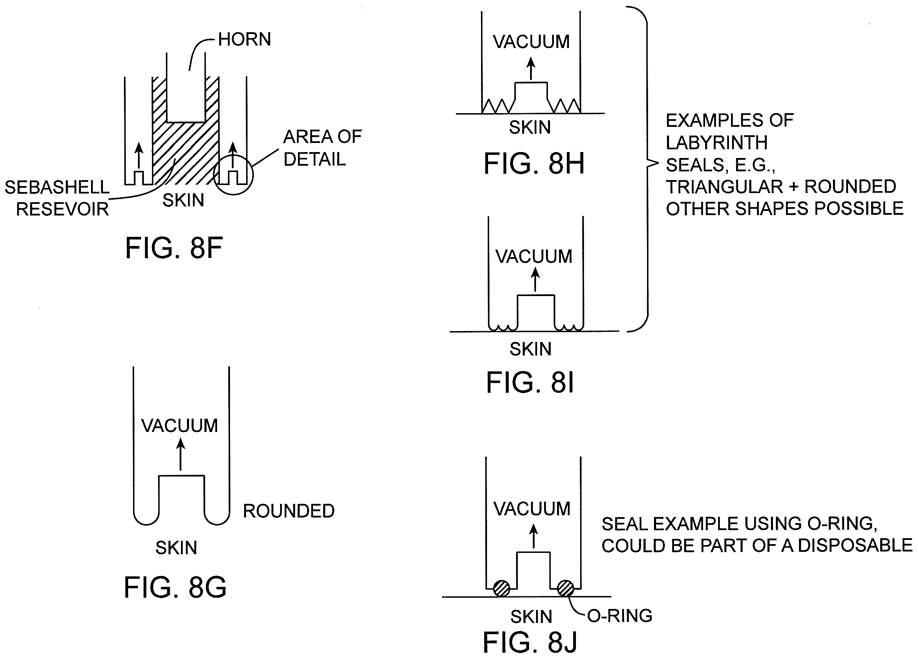

FIG. 6 is a section view of a cup assembly adapted to deliver fluid into the cup interior and provide for a vacuum seal between the cup assembly and a surface to be treated. The cup assembly 130 includes a fluid inlet 165 in communication with the cup interior 136. The cup assembly 130 also includes a port 146 for connection to a vacuum source. The port 146 is in communication with a conduit 150 and the annulus 145. The annulus 145 is formed in the lower surface 134. The cup assembly 130 illustrated in FIG. 6 may be modified to include other functionality such as one or more seals (see, e.g., FIGs. 8A-8E, 11 or 12) or formed from different materials (see FIGs. 11-14).

FIG. 7 is a section view of a cup assembly adapted as in FIG. 6 also including a cooling conduit and loop. FIG. 7 is a section view of a cup assembly adapted to deliver fluid into the cup interior and provide for a vacuum seal between the cup assembly and a surface to be treated as described in FIG. 6. As such, the cup assembly 130 of FIG. 7 includes a fluid inlet 165 in communication with the cup interior 136. The cup assembly 130 also includes a port 146 for connection to a vacuum source. The port 146 is in communication with a conduit 150 and the annulus 145. The annulus 145 is formed in the lower surface 134. Additionally, the cup assembly of FIG. 7 includes an exemplary fluid circulating system for temperature control of cup interior 136. In this exemplary embodiment, a fluid loop or channel or series of channels 170 is provided within the cup assembly. A fluid is introduced into the loop 170 via the inlet port 172 and removed via the outlet port 174. The cup assembly 130 illustrated in FIG. 7 may be modified to include other functionality such as one or more seals (see, e.g., FIGs. 8A-8E, 11 or 12) or formed from different materials (see FIGs. 11-14).

FIG. 8A is a section view of a cup assembly 130 having one or more seals on the lower surface 134. The illustrated cup assembly may have any of the above described functionalities but those additional features have been omitted for clarity in this description. A cup assembly may include a seal 155 along the cup assembly lower surface 134. In one aspect the seal circumscribes the interior portion 136 of the cup assembly (see, e.g., FIGs. 11, 12). In an alternative embodiment, the seal 155 has optionally, a plurality of elements each one having a cross section shape that is one of: u-shaped, t-shaped, v-shaped and j-shaped or the seal itself may have one of these cross section shapes. In the illustrative embodiment of FIG. 8A, there seal 155 has a u-shaped cross section and the seal 160 has a t-shaped cross section.

Still further and other sealing configurations on the cup assembly are possible. In one embodiment, there is a seal on the cup assembly lower surface 134 between an outer edge of the annulus 145 and a perimeter of the lower surface 134. In still another aspect, there is a seal on the cup assembly lower surface 134 between an inner edge of the annulus 145 and a perimeter of the interior portion 136 of the cup assembly. The seal configuration of FIG. 8A also illustrates a delivery device with a cup assembly having a first seal 155 and a second seal 160 attached to the cup assembly lower surface 134. Additionally, this illustrative embodiment shows how the first seal 155 is closer to the interior portion 136 of the cup assembly. In a similar way this embodiment also shows a configuration where the first seal 155 and the second seal 160 both circumscribe the interior portion 136 of the cup assembly.

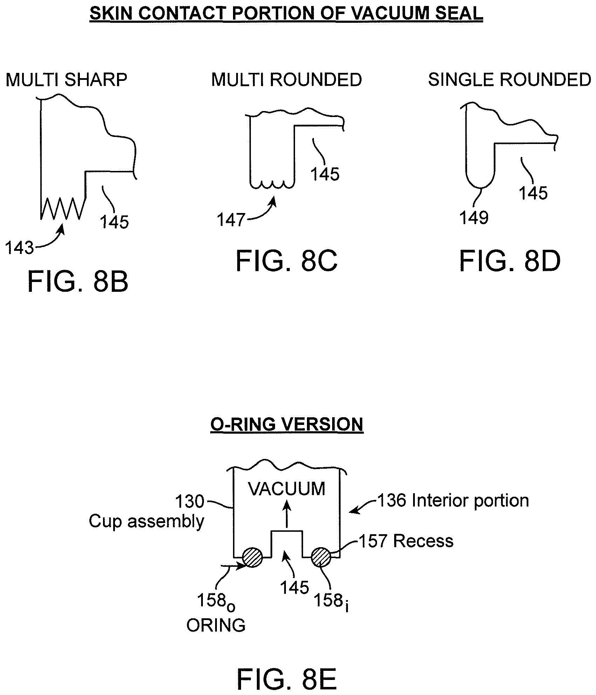

In still another alternative embodiment, the cup assembly of FIG. 8A may be further modified (see FIG. 8E) to include a pair of recesses 157 in the lower surface. One recess of the pair of recess sized to receive a portion of the first seal 155 and the other of the recesses in the pair of recesses sized to receive the second seal 160. In one specific exemplary aspect, as illustrated below in FIG. 8E, the first and second seals may be o-rings. Alternatively or additionally, the first seal 155 or the second seal 160 has a cross section shape that is one of: u-shaped, t-shaped, v-shaped and j-shaped.

Turning now to FIGs. 8B - 8J which each show a variety of different exemplary seal types adjacent the annulus 145, although other locations are possible on cup assembly 130. The delivery device 100 or portion of a cup assembly 130 may also include a first seal 155 or a second seal 160 in which one or both optionally also have a plurality of sealing elements each one of the elements having a cross section shape or a shape adapted and configured as providing a labyrinth type seal when the cup assembly contacts to a treatment site. The seal may have a single shape (149 in FIG. 8D) or multiple shapes (see FIGs. 8B and 8C). Other shapes beyond the non-limiting examples provided are possible and variances in number, size, length, thickness are envisioned to enhance effect of sealing properties and other functional properties such as translation. The sealing element cross section shape may be one or more of: multiple u-shaped 147 as shown in FIG 8C or 8I, or multiple v-shaped 143 as shown in FIG 8B or 8H.

Alternatively, the cross section shape may be t-shaped 151 as shown in FIG. 8A or reversed into a j-shape (determined by whether a portion of the seal curves away from (t-shaped) or towards (j-shaped) the cup assembly interior 136. In still further aspects, the delivery device 100 may have a first seal 150 or a second seal 160 having a beveled edge. In one embodiment, a distal most edge portion of the beveled edge is directed towards the perimeter of the lower surface 134. In another alternative, the distal most edge portion of the beveled edge is directed towards the interior portion 136 of the cup assembly 130.

FIG. 8E illustrates an embodiment of a cup assembly 130 where the lower surface 134 includes a recess 157 configured to receive a portion of a seal. In this illustrative embodiment, the recess 157 receives an o-ring 158. The O-rings 158 provide sealing positions on either side of the annulus 145. O-ring 158ᵢ has a smaller overall diameter than O-ring 158₀.

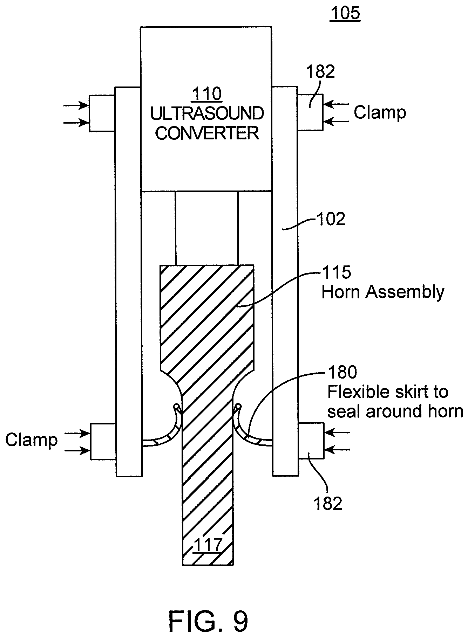

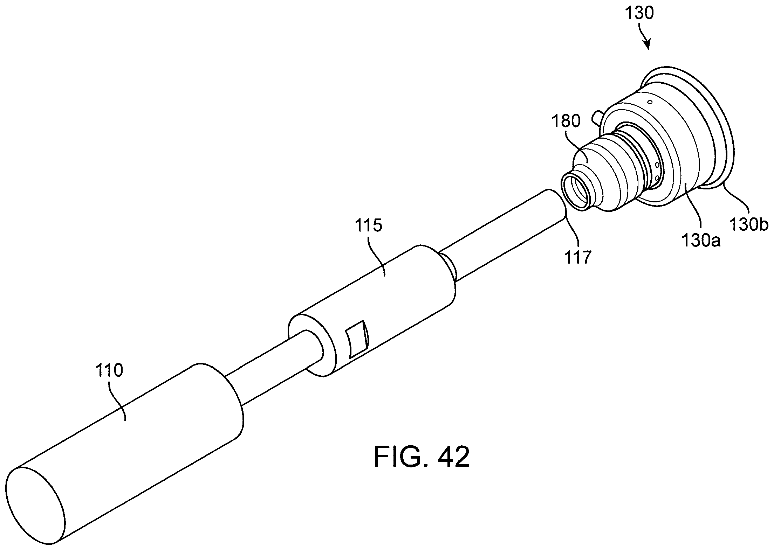

In an additional embodiment, there may also be a gasket provided around the ultrasound horn to prevent delivery fluid or formulation from leaking/splashing out in use. In one aspect there is a gasket 180 having a first portion sized to seal against an exterior portion of the ultrasound horn and a second portion sized to seal against the cup assembly interior portion. In alternative embodiments, the gasket 180 has a conical shape, a trapezoidal shape or truncated conical shape. FIG. 9 illustrates a section view of the housing 105 having a gasket 180 attached to an interior wall of the housing and extending to form a seal about a portion of the ultrasound horn 115. FIG. 10 illustrates a section view of a cup assembly 130 having a gasket 180 over the upper surface 132 affixed by a clamp ring 131 or other suitable means. The upper portion a housing gasket 180 form a sealing interface with a portion of the ultrasound horn 115. As illustrated, fluid 10 within the interior portion 136 will not pass the seal formed by the gasket 180. In another aspect, an embodiment of a gasket 180 is also illustrated in the cup assembly 130 illustrated in FIGs. 11 and 42.

FIGs. 11 through 14 illustrate various views of an alternative hybrid cup assembly 130. A cup assembly 130 may be made of one or multiple materials. Here, cup assembly 130 has an upper portion 130a that is rigid and a lower portion 130b that is flexible. FIG. 11 illustrates an upward isometric view of an embodiment of a dual material cup assembly 130. In this illustrated embodiment, the upper portion 130a has a metal body. It may be an aluminum body. The lower portion 130b may be molded to include the seals 155 and 160 be made of urethane. The two part assembly is also shown in the view of FIG. 13. FIG. 13 also shows the upper portion of the cup assembly with the gasket 180 (shown in FIG. 11) removed. In this view, grooves 135 for coupling to gasket 180 are visible. In the bottom-up view of FIG. 12, a number of different features are revealed. In this particular configuration, there are shown a plurality of vacuum ports 152 that are formed in the urethane seal of lower portion 130b. The vacuum ports 152 are arrayed in an even circular pattern within the annulus 145. The annulus 145 is bounded by the inner seal 155 and the outer seal 160. Disposed within the interior portion of the cup assembly is the distal face of the ultrasound horn 115 (bottom most surface of distal portion 117). In the illustrated embodiment, the diameter of the cup assembly interior is 16 mm while the diameter of the ultrasound horn 117 is 13 mm.

In these illustrated embodiments, the material used for the lower portion 130b is urethane, but different materials or variations in material properties could be provided to improve sealing and translation characteristics as described elsewhere. For example, a softer material would be easier to make a seal, while a harder material is easier to translate and/or maintain a vacuum seal because of structural strength provided to the annulus 145. Other considerations for selecting the material properties of the sealing surface include, for example, lubricity, coefficient of friction, and other material properties that will aid in maintaining translation and sealing characteristics. Other potential materials for use in the sealing surface, seals, or art 130b of the cup assembly include, by way of example and not limitation, silicone, PeBAX, polyurethane and polyethylene. In still further aspects, the lower portion 130b of the cup assembly 130 could be designed for single use, multiple use or limited use depending upon material selection. As described elsewhere herein, the cup embodiments illustrated in FIGs. 11 through 14 may be modified for use with O rings (see FIG. 8E) or other seals (See FIGs. 8B, 8C, and 8D). In one specific embodiment of the configuration of FIG. 12, the annulus 145 has a width of about 4 mm and is about 4 mm deep. The holes 152 are about 2.5 mm in diameter.

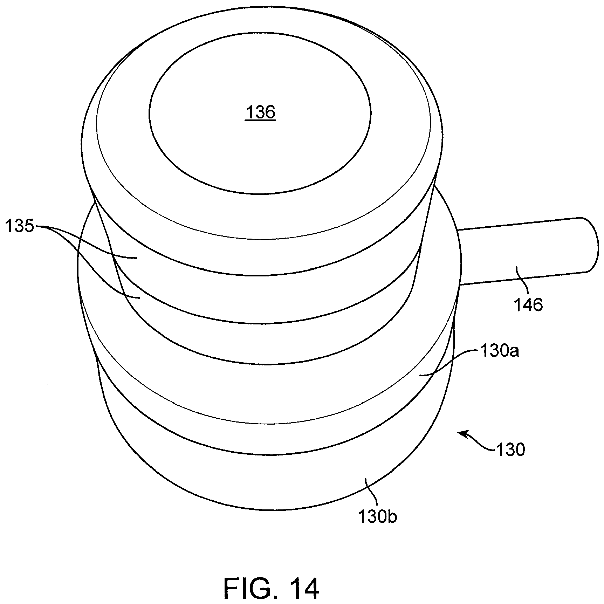

FIGs. 13 and 14 also illustrate one possible position of the vacuum port 146 which is connected to the annulus 145 and ports 152 shown in FIG. 12. The view of FIG. 14 illustrates a top down isometric view of the cup assembly 130. Visible in this view are the interior portion 136, the vacuum port 146, as well as the two parts 130a, 130b of housing 130.

The cup assembly may be formed from a single piece having all or some of the various functionalities and capabilities described herein. Alternatively, as illustrated by the hybrid material cup embodiment in FIGs. 11-14 different materials may be selected based on the purpose or function attributed to a particular component. Additionally or alternatively, the functionality of the cup assembly may be divided between two or more components in order to provide the desired functionality, simplify manufacture, and enhance reliability or for any of a number of other reasons. In one aspect, the cup assembly may be divided such that one or more functions of the cup assembly described herein are not achieved until the two or more pieces of the cup assembly are combined.

In one embodiment, a two piece cup assembly is provided whereby at least one functional attribute of the cup assembly is inoperative until the cup assembly parts are combined. In other words, the functionality of the cup assembly relies on the synergetic combination of the cup assembly components. One example of such a synergy is illustrated in an embodiment of a two part cup assembly where a fluid loop is cut or formed into a wall of one piece and then closed off or completed (i.e., forming a fluid tight conduit) when the other piece is joined. Similar combinations of component assembly and functionality are possible based on this example.

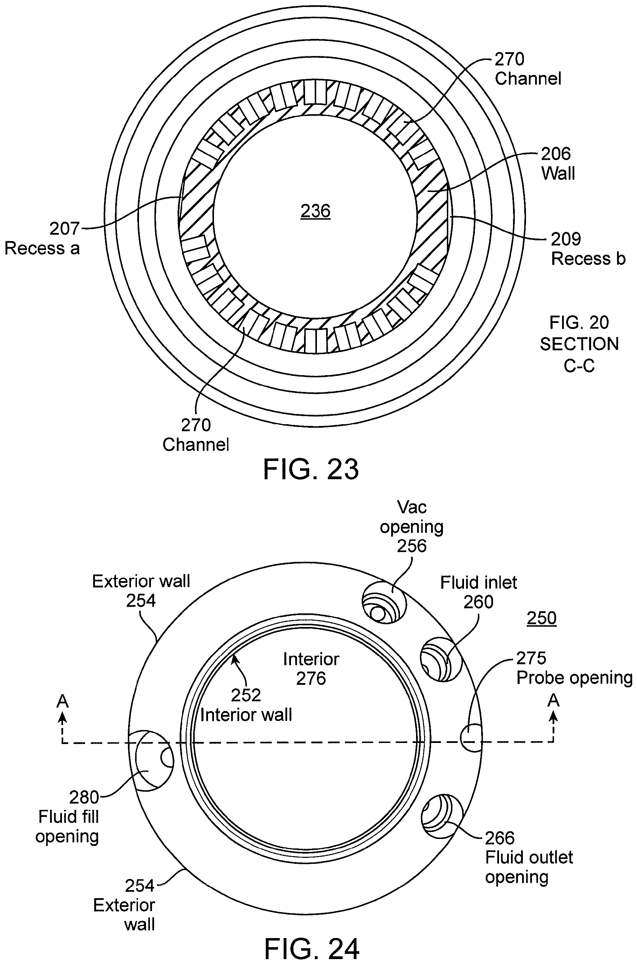

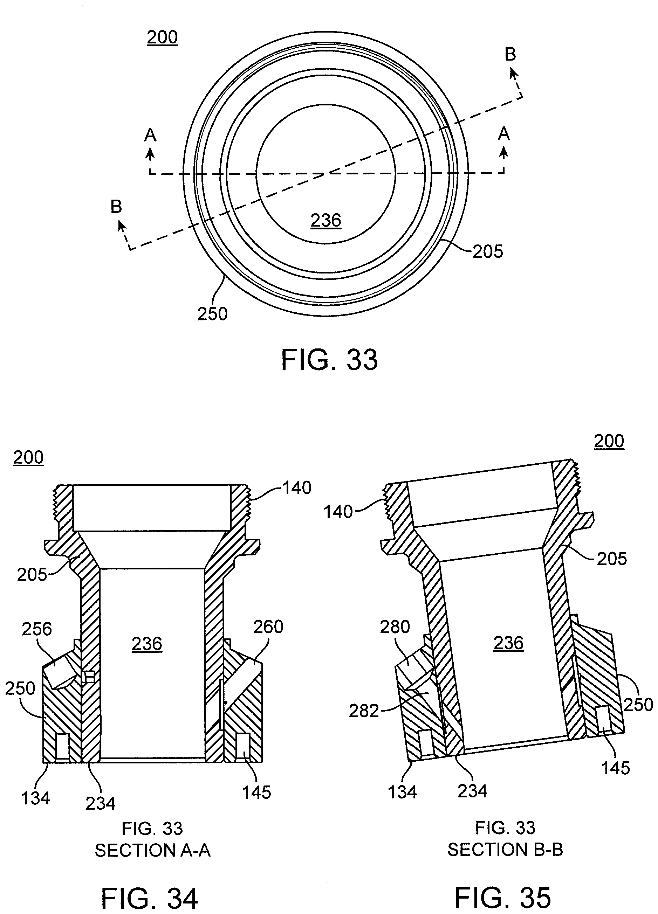

FIGs. 15 through 37 illustrate an exemplary embodiment of a two part cup assembly 200 used as part of a prototype delivery device 300. The delivery device 300 is best seen in the various views of FIGs. 38-41. The two part cup assembly 200 is made from a top inner cup 205 and a bottom outer cup 250. The top inner cup is described first with regard to the various views of FIGs. 15-23. The bottom outer cup 250 is next described with regard to the various views of FIGs. 24-32. The assembled two part cup assembly 200 is been seen in the various views of FIG. 33-37.

FIGs. 15-37 illustrate a variety of views of the two part cup assembly 200 having a top inner cup portion 205 and a bottom outer cup portion 250. As will be appreciated in the various views, the top inner cup portion includes a mating surface 140 and a bottom surface 234 and an interior portion 236. This interior portion is similar functionally to the interior portion 136 described previously. There is also a shaped interior portion 211 in the wall 206. The portion 211 is provided to accommodate the ultrasound horn 115. The bottom outer portion 250 includes a bottom surface 134, an exterior wall 254, and an interior wall 252 defining an interior space 276. The bottom outer cup 250 provides for a variety of connection ports, conduits and openings used to provide communication from outside of the cup to interior portions of either or both of the portions 205, 250.

In the various views there is provided various appropriately placed and sized openings and conduits to permit operation of the various functionality of the cup assembly. The cup assembly 200 includes a vacuum opening 256 and a vacuum conduit 258 for connection to a suitable vacuum system. A temperature controlled fluid system may be connected to the cup assembly 200 via fluid inlet 260 and outlet 266. Suitably sized and placed conduits 262, 268 and openings 264, 270 are provided to permit control fluid supply and return in the cup assembly. Related to the temperature controlled fluid circulation system are the channel pattern 270 including cross over segment 273 and end segment 272. Temperature controlled fluid provided via inlet 260 travels along channel to 270 and is removed via outlet 266. A probe inlet 275, conduit 276 and opening 277 are provided to give access to the interior portion to a variety of probes for monitoring conditions in the cup assembly interior. The delivery fluid or formulation is delivered and or removed using delivery fluid port 280, conduit 282 and opening 284. Additional other ports, conduits and openings may be provided depending upon the functional capabilities provided by a particular cup assembly.

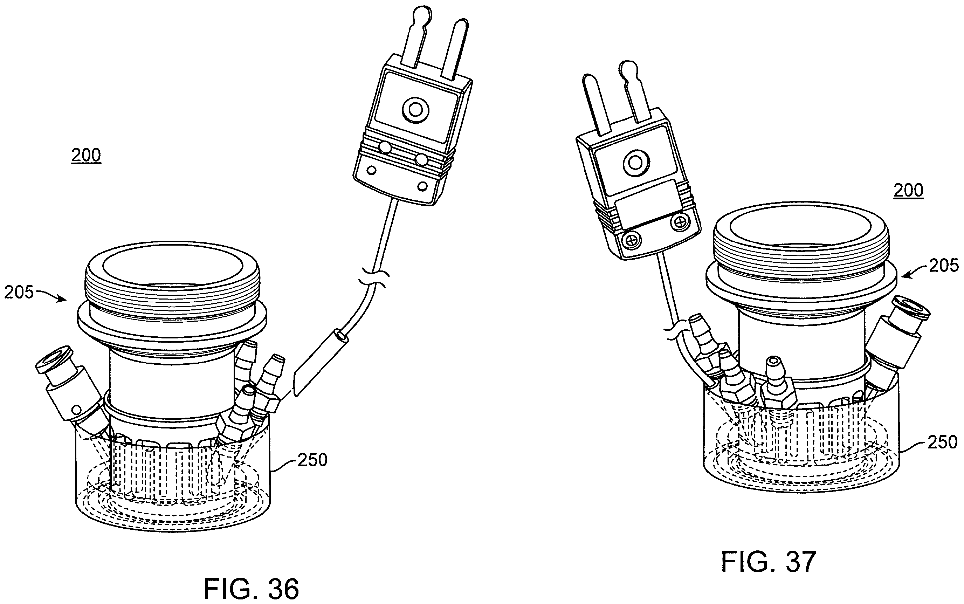

FIGs. 15 through 23 illustrate various views of one portion of the two part cup assembly. As will be apparent from the description that follows, the top inner component illustrated in FIGs. 15 through 23 is adapted and configured to operate with the bottom outer component part shown in the various views of FIGs. 24 to 32. As a result, the two parts 205, 250 of the cup assembly when assembled, form the cup assembly 200 shown in FIGs. 33 and 37. The full cup assembly 200 (i.e., FIGs. 36, 37) is shown in the assembly views of a delivery system 300 illustrated in the various views of FIGs. 38, 39, 40, and 41.

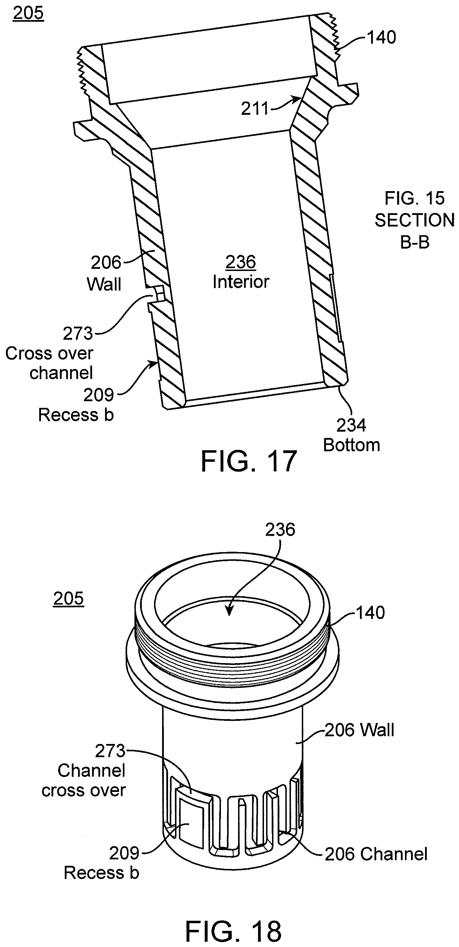

Turning now to FIG. 15, which is a top down view of the first part of the two part assembly. The top down view of FIG. 15 provides section views A-A and B-B. FIG. 16 is a section view along section A-A of FIG. 15. FIG. 17 is a section view along section B-B of FIG. 15.

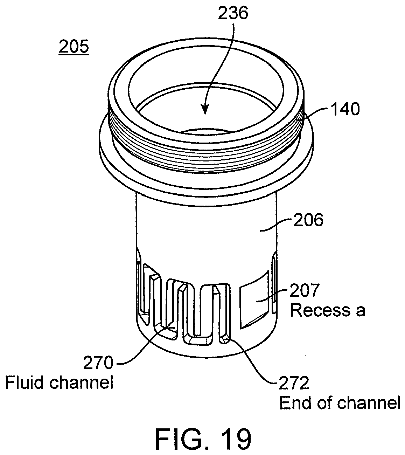

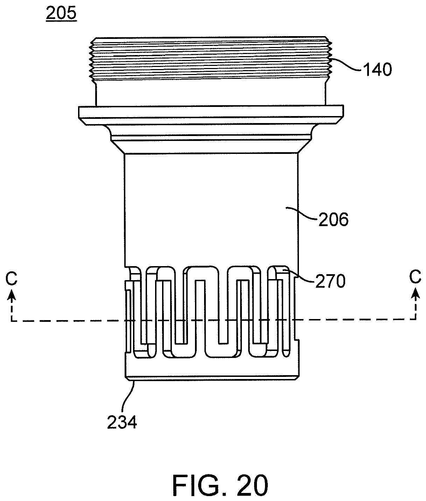

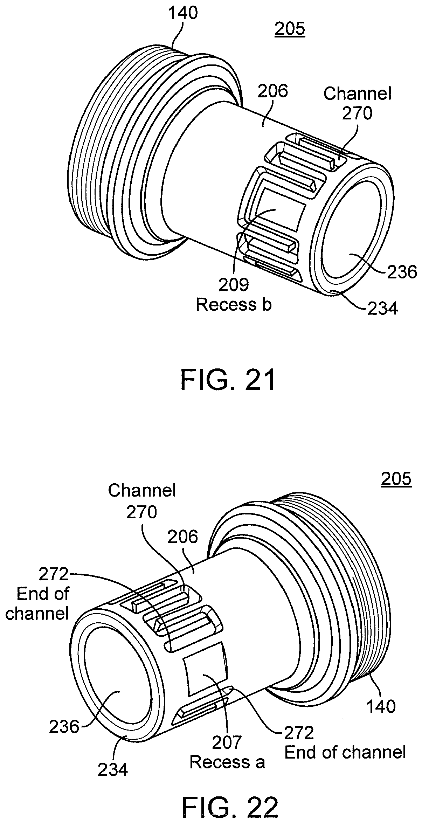

FIG. 18 is a first isometric representation of the part of the cup assembly. In the view of FIG. 18, the fluid channel 270 is visible along with the portion of section B-B passing through the side. FIG. 19 is an alternative isometric view showing the other end of the cooling channel, a right side view of the first part of the two part cup assembly. In this view, the end of the cooling channel 270 is seen. Returning to FIG. 18, also shown is the continuation of the fluid channel 270 as it extends around to complete the circumference of the component. FIG. 20 is another side view of the first part of the two part cup assembly providing section view C-C. Additional isometric views of the component are shown in FIGs. 21 and 22, again highlighting the details of the fluid channel 270 formed in the wall of the component. FIG. 23 is a bottom up view of the component along section C-C of FIG. 20. In the view of FIG. 23, the various channel diameters are shown in the outer wall of the component.

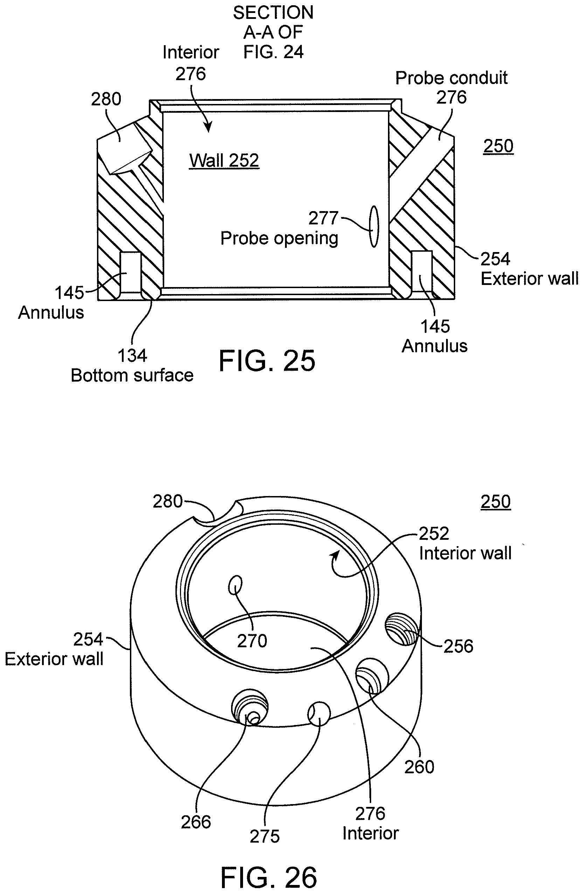

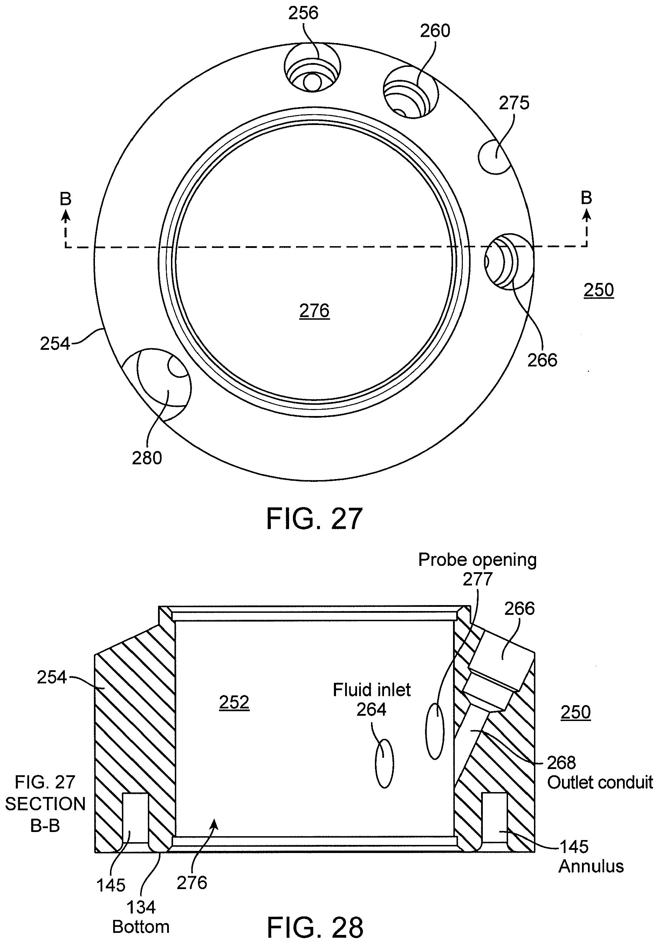

FIGs. 24 through 35 are various views of the second part of the two part housing. The parts illustrated in FIGs. 24 to 35 fit over the part illustrated in FIGs. 15 through 23. FIG. 26 is an isometric view of the second or outer component of the two part cup assembly. FIG. 24 is a top down view of the outer part of the cup assembly showing section view A-A. Also shown in FIG. 24 and FIG. 26 are the delivery fluid fill ports, the vacuum connection port, the cooling inlet, the thermal couple hole, and the cooling outlet. FIG. 25 is a section view of the second part of the cup assembly along section A-A of FIG. 24. In this view, the fluid delivery injection port, the thermocouple hole, and the annulus are shown. Also shown is an opening in the anterior wall for a temperature probe. FIG. 27 is an alternative top down view of the upper surface of the second part of the two part component showing section view B-B. FIG. 28 is a view of the interior of the outer component of the two part cup assembly along section B-B of FIG. 27. In the view of FIG. 28, the cooling inlet and thermal couplet inlet are shown on the interior wall of the housing. Also shown is the cooling inlet and the annulus shown in the side wall.

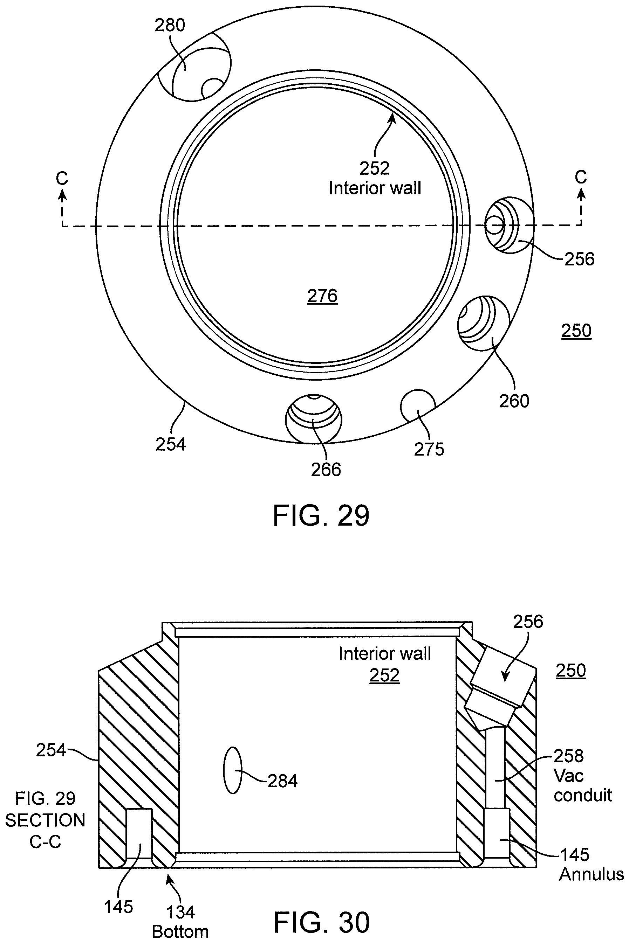

FIG. 29 is another top down view of the second part of the two part cup assembly. FIG. 29 is used to show section C-C. FIG. 30 illustrates section C-C of FIG. 29. In the view of FIG. 30, the connection port for the vacuum system is shown along with the internal conduit in communication with the annulus. Also shown is the fluid inlet used to deliver a liquid or formulation into the interior portion of the cup assembly.

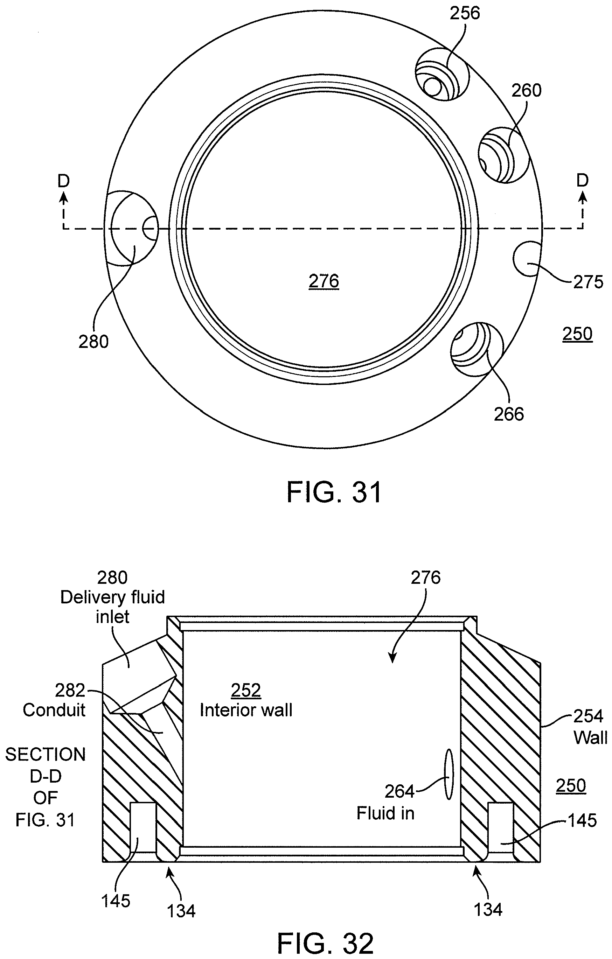

FIG. 31 is another top down view of the two part cup assembly showing the section view D-D. FIG. 32 is the section view D-D of FIG. 31. In the view of FIG. 32, the delivery fluid inlet is shown along with the annulus and the inlet port of the controlled temperature fluid system.

FIG. 33 is a bottom up view of the second part of the two part cup assembly. FIG. 33 is used to illustrate section views A-A and B-B. FIG. 34 is section A-A of FIG. 33. In this view, the vacuum port, cooling port, and annulus are shown. FIG. 35 is section B-B of FIG. 33. In the view of FIG. 35, the thermocouple port inlet, the annulus, and the delivery fluid inlet are shown. Also shown in the view of FIGs. 34 and 35 are the threaded mating surfaces used to join the two part cup assembly to the housing 105. FIG. 33 illustrates a bottom up view of the assembled two part housing to show sections A-A and B-B. FIG. 34 illustrates a section view A-A of the assembled two part cup assembly. FIG. 35 illustrates a section view B-B of the assembled two part cup assembly.

FIGs. 36 and 37 are isometric views from each side of the assembled two part cup assembly illustrated and described in the various views of FIGs. 15 to 35. In the view of FIGs. 36 and 37, the thermal couple connector is shown with the probe extending into the two part housing. In addition, the various details are shown in phantom of the interior of the two part cup assembly, for example, the cooling channels and the vacuum annulus are shown.

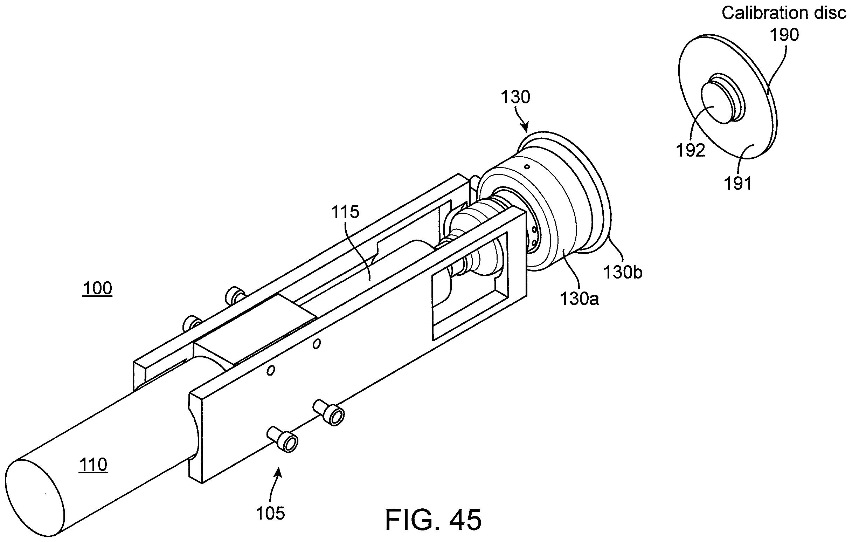

FIGs. 38A through 41 illustrate an alternative prototype delivery device 300.As best seen in the exploded view of FIG. 38A, the delivery system 300 includes the two part cup assembly 200 and a housing 305. The ultrasound components (collectively 117, 115 and 110) are inserted into the hollow interior of housing 305 as indicated by the dashed lines. When positioned within the housing, the ultrasound unit is free to move into a variety of different positions to provide an adjustable ultrasound horn-skin spacing. Housing embodiments described herein include one or more mechanisms to permit or prevent skin-horn space adjusting motion of the ultrasound units. A suitable spacer is provided (such as gage block 315 or calibration disc 190) for that purpose as illustrated and described in FIGs. 38A and 45.

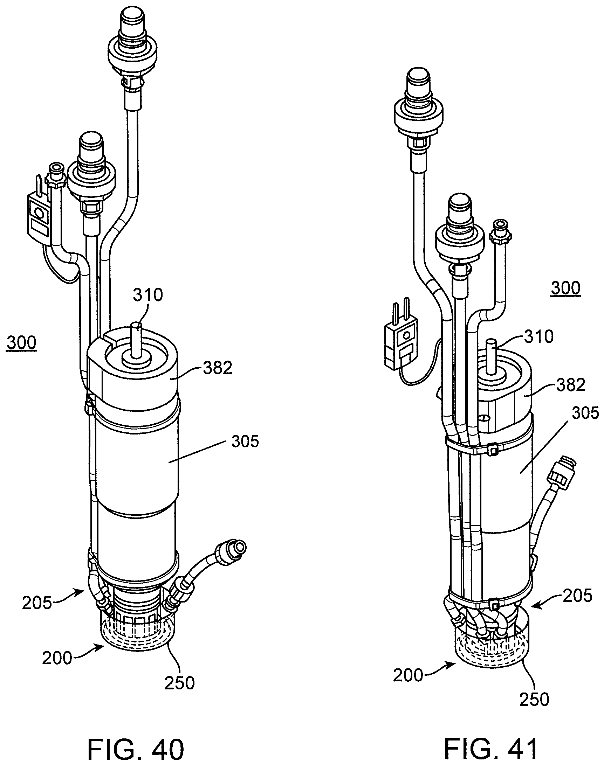

In the illustrative embodiment of FIG. 38A, once the ultrasound components are inserted into the housing 305, the clamp 382 is engaged to apply a suitable hold force to a portion of an ultrasound unit. In the illustrative embodiment, the clamp engages with an outer wall of the ultrasound converter 110. The pin or screw 383 is used with a threaded or receiving portion of the clamp to provide the desired amount of clamp force. Additional details of the housing 305 are provided in the various view of FIG. 38B. Section B-B illustrates the threaded portion to receive screw 383 along with the gap or space that permits the clamp region in the end of the housing 305 to close in on the ultrasound component. The space is visible in the view of FIG. 40 (adjacent the ultrasound connector 310). Returning to FIG. 38B, as best seen in the side, exterior view of the housing 305, the slot indicated in FIG. 40 is provided between the recessed portion and the clamp portion to permit deflection of the clamp portion. As best seen in FIG. 38B cross section view A-A, there is a recessed wall portion 302r similar to recess 102r to provide the ultrasound component gap 103 (see FIGs. 2 or the alternative configuration gap in FIG. 3).

A gage block 315 is also shown in FIG. 38A. The gage block 315 engages with the ultrasound portion 117 to provide a preset spacing for the ultrasound horn 117 to the treatment surface. The gage block may be adjustable or come in preset sizes such as 2mm, 4mm, 6mm, 8mm, 10mm, 12mm, 13mm or any other suitable spacing depending upon the delivery operation being performed with the delivery device.

In FIG. 39, the various components have been assembled by placing the ultrasound assembly within the housing and securing the cup assembly to the lower portion of the housing. In addition, the upper clamp 382 has been used to secure the ultrasound components within the housing in a manner suitable and described above to provide a gap between the ultrasound components and the interior of the housing. Also shown are the various connection lines to the coolant inlet and outlet, vacuum connection, thermal couple, and delivery fluid supply. FIGs. 40 and 41 provide alternative side isometric views of the delivery device of FIGs. 38 and 39.

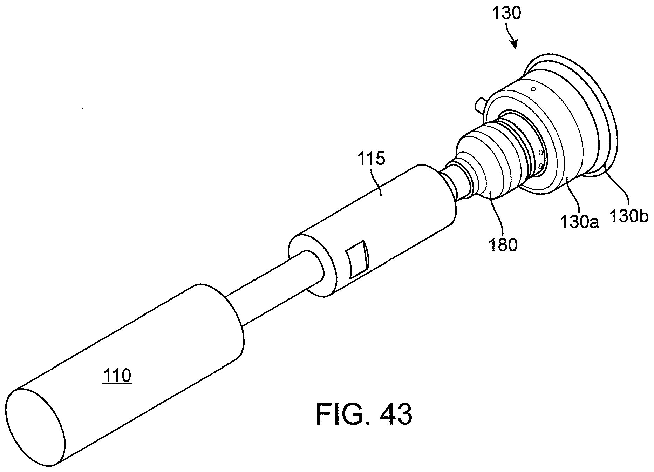

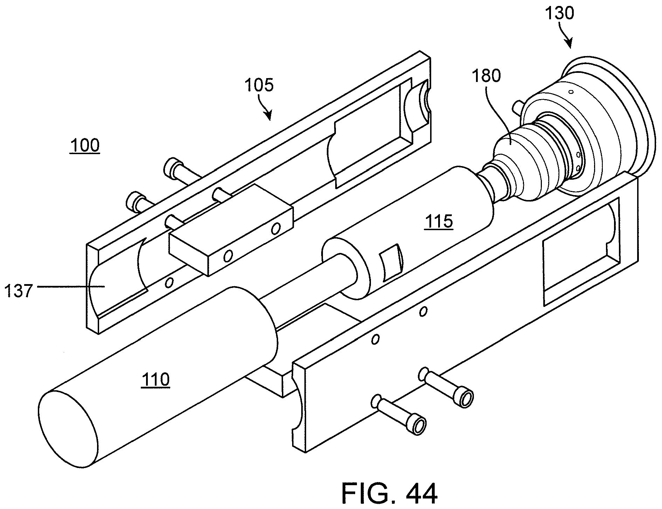

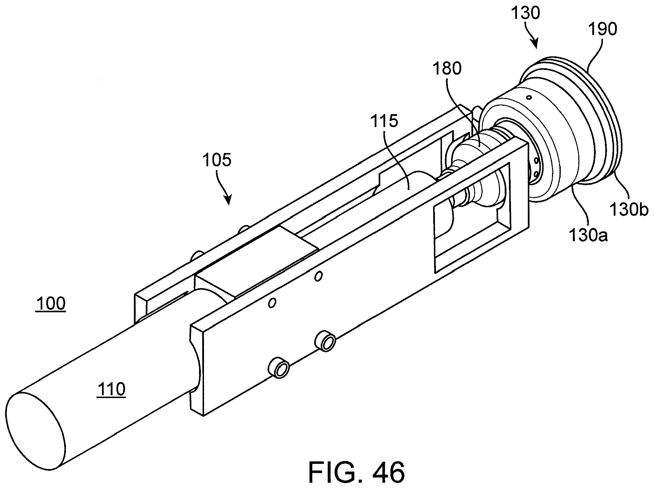

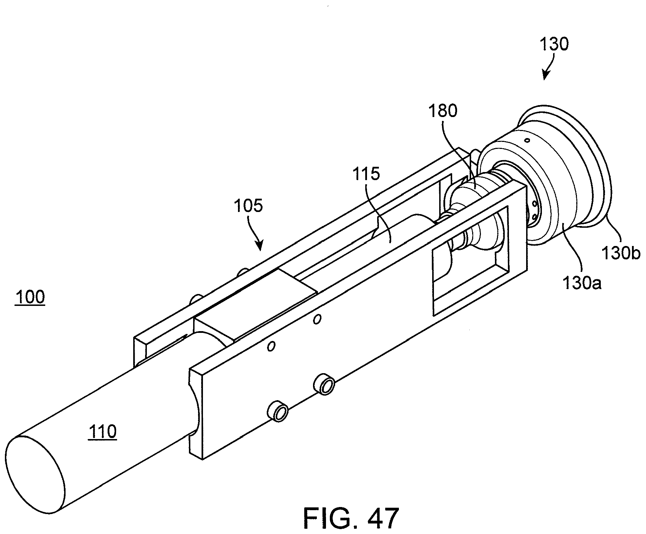

FIGs. 42 through 47 illustrate an additional alternative configuration of the delivery device. FIG. 42 is an isometric exploded view of the ultrasound components and the delivery cup 130. FIG. 43 shows the components of FIG. 42 assembled where the ultrasound horn has been extended through the cup assembly 130. FIG. 44 is an isometric view including an exploded view of the housing assembly 105 before being attached about the ultrasound components and the cup assembly. A recessed clamp region 137 is provided in the end of the housing 105 to clamp onto the ultrasound converter 110. The 4 pins and two blocks seen in this view are used to adjust the force of the clamp region 137 against the ultrasound converter 110. FIG. 45 illustrates the housing 105 assembled from the view of FIG. 44. Also shown in FIG. 45 is the calibration disc 190 which is used to set the distance from the horn distal surface to the skin. When ready to set the distance, the calibration disk is placed on a flat surface and the ultrasound horn, the pins/blocks loosened to permit movement of the ultrasound converter 100 relative to the housing. As a result, the ultrasound horn distal end 117 is lowered/raised till it touches the disk. Then, depending upon the specific configuration of the housing, all clamps or clamping mechanisms are tightened to secure the ultrasound components relative to the housing and lock down the horn spacing or distance. In the illustrated embodiment, the calibration disc 190 includes an upper surface 191 and a raised central portion 192. The size and dimensions of the raised portion 192 are selected to provide the appropriate skin to ultrasound horn gap for the desired operation of the delivery device 100 as described above with regard to gage block 315. FIG. 46 illustrates the calibration device 190 in place along the lower surface of the cup assembly 130 in order to provide proper spacing of the ultrasound components relative to the cup assembly. FIG. 47 illustrates the delivery device of FIG. 46 after calibration with the calibration disc 190 removed.

### Additional Considerations

In still further aspects, the size of the cup assembly (and associated ultrasound horn) may be adjusted smaller to smaller sizes to enhance the ability of the device to reach smaller structures such as lips and nose, but with an effective seal to aid in maintaining fluids for a suitable ultrasound environment. In some circumstances, a smaller area (i.e., smaller horn/cup assembly footprint) may be easier to create and maintain a seal on a curved surface. Still further, the size of the cup assembly (and associated ultrasound horn) may be adjusted larger to enhance the ability of the device to cover larger treatment areas more readily such as the back, chest, abdomen, legs, arms and shoulders, but with an effective seal to aid in maintaining fluids for a suitable ultrasound environment. In terms of increasing sizes, the diameter of the ultrasound horn and/or associated cup assembly may be around 13 mm or up to about 20 mm, 25 mm or even up to 30 mm. In terms of decreasing or smaller sizes, the diameter of the ultrasound horn and/or associated cup assembly may be around 13 mm, about 10mm, 8 mm, 6 mm, 4 mm or 2 mm.

While specific cup assembly and ultrasound horn dimensions are provided above, it is to be appreciated that a wide variety of different sizes may be used with the advantages of the various inventive concepts described herein by accommodating a desired ultrasound horn to target skin tissue within a range. In functional terms, this means that during operation of the ultrasound system with the cup assembly containing fluid and translating across the target surface the average skin to horn surface distance is maintained within a desired range.

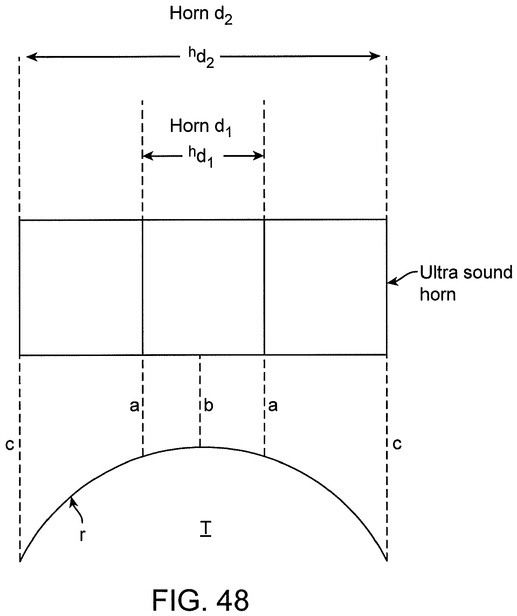

FIG. 48 illustrates a cross section area of two different sized ultrasound horns over a selected target tissue site (T) having a radius of curvature (r). In this example, there is an ultrasound horn (h) with a diameter d1 and a larger ultrasound horn (h) with a diameter d2 and d2 > d1. For both horns, the horn mid-face distance b is the same. For the horn d1, the horn edge to skin distance is a. For the horn d2 the horn edge to skin distance is c. In one embodiment, the parameters of the ultrasound horn and cup assembly are selected such that for the variation from edge-center-edge is no more than 1 mm for a target skin site. In another aspect, the horn dimensions are selected based upon estimated, planned or predicted target site parameters such that the skin-horn spacing over the radius of the target site maintains a suitable ultrasound environment including depth of material in relation to horn, horn-skin spacing and other conditions suited to maintaining cavitation, immersion and/or acoustic streaming. Still further, the horn and cup assembly characteristics may be selected that for a given target site geometry (i.e., radius of curvature, smoothness and other surface factors) the variation between horn to skin spacing from the center to edge is no more that 20% of the target skin to horn distance, e.g., for a target distance of 10mm the allowable variation across horn would be +/-2mm.

### Ultrasound facilitated Delivery

Ultrasound has been used to achieve transdermal delivery of compounds into the body. Ultrasound appears to generate shock-waves and micro-jets resulting from bubble cavitation that causes the formation of channels in the skin, which provide for the transport of molecules of interest. Previous efforts have been directed toward the delivery of the compounds through the stratum corneum. Small molecules, for example, with sizes less than 5 nm, can be delivered through the stratum corneum. The delivery rate through the stratum corneum goes down significantly as particle size increases. For example, for particles with size of 50 nm and higher, the delivery rate through the stratum corneum is very low. However, this size is still much smaller than the pore opening and the infundibulum of a follicle. For example, 150 nm size silica-core and gold shell structures are being used that are much smaller than the infundibular diameter while showing low deposition in skin through the stratum corneum.

These findings provide the basis of acne treatment in which the infundibulo-sebaceous unit is selectively targeted for first delivery of light absorbing material of appropriate size and then selective thermal damage to the unit with pulsed laser irradiation. Here, ultrasound specifically facilitates the delivery of a light absorbing material into the follicular structure. The shock waves, microjet formation, and streaming deliver the light absorbing particles into the follicular infundibulum and the associated sebaceous gland duct and the sebaceous gland.

Ultrasound is often be accompanied by heating of the target organ, skin. Some heating, for example, up to about 42.degree. C. may help in follicular delivery. However, excessive heating is undesirable, causing pain, tissue damage, and burns. In one embodiment, excessive heating can be avoided by cooling the skin, for example. In another embodiment, the topically applied formulation or a coupling gel can be pre- or parallel-cooled. A low duty cycle with repeated ultrasound pulse bursts can also be used to avoid excessive heating, where during the off-time, the body cools the skin that is being subjected to ultrasound energy.

Two methods of ultrasound delivery are disclosed. One is "contact ultrasound "and another is "immersion ultrasound".

In accordance with an embodiment of the contact ultrasound method, a formulation is topically applied to the skin by spreading into a thin layer and a horn vibrating at an ultrasound frequency is brought into close contact with the formulation-covered skin.

In accordance with an embodiment of the immersion ultrasound method, a reservoir filled with the formulation is placed on top of the skin, a horn is immersed in it without the horn touching the skin at a distance ranging from about 2 mm to about 30 mm, and the horn is then vibrated at ultrasound frequency.

Acoustic cavitation is often an effect observed with ultrasound in liquids. In acoustic cavitation, a sound wave imposes a sinusoidally varying pressure upon existing cavities in solution. During the negative pressure cycle, the liquid is pulled apart at 'weak spots'. Such weak spots can be either pre-existing bubbles or solid nucleation sites. In one embodiment, a bubble is formed which grows until it reaches a critical size known as its resonance size (Leong et al., Acoustics Australia, 2011-acoustics.asn.au, THE FUNDAMENTALS OF POWER ULTRASOUND--A REVIEW, p 54-63). According to Mitragotri (Biophys J. 2003; 85(6): 3502-3512), the spherical collapse of bubbles yields high pressure cores that emit shock waves with amplitudes exceeding 10 kbar (Pecha and Gompf, Phys. Rev. Lett. 2000; 84:1328-1330). Also, an aspherical collapse of bubbles near boundaries, such as skin yields microjets with velocities on the order of 100 m/s (Popinet and Zaleski, 2002; J. Fluid. Mech. 464:137-163). Such bubble-collapse phenomena can assist in delivery of materials into skin appendages, such as hair and sebaceous follicles. Thus, various embodiments provide for immersion ultrasound methods for optimizing bubble size before collapse to promote efficient delivery of light absorbing materials into the intended target (e.g., sebaceous glands, hair follicles).

The resonance size of the bubble depends on the frequency used to generate the bubble. A simple, approximate relation between resonance and bubble diameter is given by F (in Hz).times.D (in m)=6 mHz, where F is the frequency in Hz and D is the bubble diameter (size) in m. In practice, the diameter is usually smaller than the diameter predicted by this equation due to the nonlinear nature of the bubble pulsation.

Table A below gives the size of the resonance size of the bubble as a function of frequency, calculated from the above relationship.

**TABLE A**

| F, kHz | | | | | | | | | | | |
|---|---|---|---|---|---|---|---|---|---|---|---|
| | 10 | 20 | 30 | 40 | 50 | 100 | 200 | 300 | 400 | 500 | 1,000 |
| D_microns | 600 | 300 | 200 | 150 | 120 | 60 | 30 | 20 | 15 | 12 | 6 |

Computer simulations of bubble oscillations give more accurate estimates of the bubble size. For example, in work by Yasui (J. Acoust. Soc. Am. 2002; 112: 1405-1413), three frequencies were investigated in depth. The sizes for single bubble sonoluminescing (SBSL) stable bubbles are lower and ranges are given in the Table B below (estimated from FIGS. 1, 2, and 3 of Yasui, 2002):

**TABLE B**

| F, kHz | | | |
|---|---|---|---|
| | 20 | 140 | 1,000 |
| D_microns | 0.2-200 | 0.6-25 | 0.2-6 |

For efficient delivery into the follicles with cavitation bubbles, there is an optimal cavitation bubble size range. Strong cavitational shock waves are needed, which are generated with relatively large bubbles. However, if the bubble size is too large, it produces strong shock waves, which may compress the skin, reducing the pore size, and reducing efficient delivery to a target (e.g., sebaceous gland, follicle). For example, if the bubble size is much larger than the follicle opening, the resulting shock waves compress not only the pore opening, but also the skin surrounding the pore opening. This inhibits efficient delivery into the follicle opening. Desirably, bubble sizes should be about the same size as the target pore. Typical pore sizes of follicles on human skin are estimated to be in the range of 12-300 microns. Thus, an advantageous ultrasound frequency range is 20 kHz to 500 kHz. In other alternatives, the application of ultrasound frequency is in the range of 20 kHz to 100 kHz, or 20 kHz to 60 kHz or even 30 kHz to 50 kHz. The desired power density is estimated to be in the range of 0.5-50 W/cm.sup.2. This is sufficient to generate cavitation bubbles in the desired size range.

"Immersion cavitation" as used herein is defined as formation and collapse of cavitation bubbles due to the ultrasound energy within the fluid formulation.

In light of the above description, there is also provided a method of facilitating delivery of light absorbing materials into a hair follicle by selecting characteristics for the acoustically created microjets to create bubbles in the formulation about the same size as the hair follicle pore. Selecting the characteristics permits the bubbles to be about the same size as a terminal follicle, a vellus follicle, or a sebaceous follicle. In another alternative implementation in light of the above description, there is also provided a method of facilitating delivery of light absorbing materials into a hair follicle by selecting characteristics for the low frequency ultrasound induced cavitation for creating bubbles in the formulation about the same size as the hair follicle. In one implementation, the hair follicle is a terminal follicle. In another implementation, the hair follicle is a vellus follicle. In still another implementation, the hair follicle is a sebaceous follicle. In still other aspects the ultrasound created microjects or low frequency ultrasound induced cavitation occurs in the formulation between about 50 microns to about 100 microns of the surface of the skin.

In another embodiment, there is also provided a method of treating or ameliorating a follicular skin disease of a subject. The method includes the step of exposing the subject's skin to a formulation comprising a sub-micron particle comprising a light absorbing material to a subject's skin. Next, there is a step of facilitating delivery of said material from the skin into a hair follicle by low frequency ultrasound induced cavitation within the formulation near the surface of the skin adjacent to the hair follicle. Thereafter, exposing said sub-micron particle to energy activation, thereby treating the follicular skin disease. In one alternative, there is also a step of exposing by placing a volume of the formulation in a container so that the formulation is in contact with the subject's skin. Still further, there is also a step of facilitating the method by placing an ultrasound applicator into the container and immersed in the formulation.

In still another embodiment, there is provided a method of facilitating delivery of a light absorbing material to a target volume within the skin of a subject. The method includes the step of topically applying a formulation comprising a light absorbing material to a subject's skin to deliver the material to a reservoir within the target volume of the skin. Next, there is a step of facilitating delivery of said material to a target volume within the skin of the subject substantially via a transfollicular pathway. Next, there is a step of exposing the light absorbing material to a series of light pulses to heat the material and thermally damage the target volume to achieve a therapeutic effect. In one alternative, the formulation has an optical density of between 5-500. In another alternative, the formulation has an optical density of about 75. In still another alternative, the formulation has an optical density of about 125. In still another alternative, the formulation has an optical density of about 250. In one aspect, the target volume is the sebaceous gland. In another aspect, the target volume is within the follicle beneath the skin.

In still another aspect, the facilitating step includes an immersion cavitation step. In another alternative, there is provided a step of facilitating delivery into a sebaceous gland using immersion ultrasound. In one alternative, the facilitating step includes forming microjets within the formulation. In one aspect, the facilitating using ultrasound produces cavitation within a formulation and about 50 to 100 microns of the surface of the skin. In any of the above described methods, there is also the step of acoustically cavitating the formulation for selectively facilitating delivery of said particles in the formulation into a sebaceous gland primarily through the corresponding hair follicle. Thereafter, there is the step of irradiating said particles with light to treat the follicular skin disease. In one embodiment, the particles are sized from about 1 micron to about 5 microns. In another aspect, the particles are sized to enter into and along a follicle pore. In still other embodiments, the particles are between about 50 nm about 250 nm in diameter. In another embodiment, the particles are nanoshells.

### Additional Methods of Immersion

### Ultrasound

As described above, delivery of particles as well as dissolved substances into follicles and follicular appendages of the skin can be achieved via 'immersion ultrasound.' In this technique, the particulate suspension or the solution is placed in an enclosure on top of the skin with gravity holding the fluid in contact with the skin. An ultrasound horn, vibrating at ultrasonic frequency is immersed in the liquid. The horn does not touch the skin; the skin-horn distance is in the range of about 1 mm to about 25 mm. The vibrating horn surface can induce pressure amplitudes in the fluid, leading to formation of cavitation bubbles. The collapsing cavitation bubbles near the skin surface cause microjets directed toward the skin surface due to the asymmetry. With such microjets impinging on skin over some time, delivery of the fluid suspension or solution can be achieved selectively into the follicles.

When ultrasound has been used in humans, two kinds of sounds have been reported. First, there is broadband acoustic sound generated due to collapse of multitude of cavitation bubbles in a given time period. It has been described as 'hissing.' For acceptable and safe treatments, the level of the hissing sound should not be uncomfortably high. Hissing can be reduced by placing ear-plugs in the ears and may also be reduced by using noise-cancellation headphones. In addition to the broadband sound, it is possible that the patients perceive a high-pitched tone, despite the ultrasound frequency being higher than the highest frequency in audible frequency range of 20 Hz-20 kHz.

Ultrasound has been used on skin of extremities (arms and legs) in humans successfully in commercial devices, one example being SonoPrep by Sontra Medical (in the range of 50 to 60 kHz). To our knowledge, perception of ultrasound by patients was not an issue with that device. However, when treating the face, ultrasound is more likely to be perceived as a high-pitch tone and it can be important that this tonal perception not be uncomfortable. It can be important to eliminate or bring this perception to an acceptable level in treatments on the face while improving the follicular delivery performance.

It was observed that the perceived high-pitched tone is reduced at higher horn displacement amplitudes. This may be due to the numerous cavitation bubbles in the fluid between the horn surface and skin. The cavitation bubbles scatter and absorb the ultrasound waves, thus reducing the pressure amplitude reaching the skin. The reduced tone is desirable; however, the higher amplitude can also reduce the density of cavitation bubbles near the skin surface and these are the bubbles that lead to skin-directed microjets, thus potentially reducing the follicular delivery performance.

Thus, there can be an optimal distance and amplitude range that leads to acceptable follicular delivery performance and minimal perception of tonal sound. Such a range has been determined for the Sonics VCX 134, 40 kHz, Ultrasound device with a 13-mm diameter horn and a gain of 3.6. The horn-skin distance can be between about 10 mm and about 15 mm. In some embodiments, the horn-skin distance is between about 11 mm and about 14 mm. The amplitude range can be between about 25% and about 43 %. In some embodiments, the amplitude range can be between about 28% and about 40%. For example, the horn-skin distance can be about 13mm; the amplitude range can be about 30%. This translates to a peak-to-peak displacement of ∼9.45 microns for the far horn surface. As noted above, there is a range around this set of parameters for which both efficacy and perceived tonal sound are in the acceptable range.

Another set of distance and amplitude can also be used to achieve the same goals. At very high horn displacement-amplitudes, the tonal perception is significantly reduced. But, as described earlier, this leads to reduced follicular delivery. However, even with high amplitude, follicular delivery can be increased by moving the horn closer to skin. Based on this, another parameter set was used: skin-horn distance of about 2-3 mm and amplitude of about 80% to about 100% for the same ultrasound device as discussed above. For about 100% amplitude, the peak-to-peak displacement is about 31.5 microns for the far horn surface. With this set, the perception is manageable while still achieving acceptable follicular delivery. Another advantage of lower skin-horn distance is that a much smaller amount of fluid can be needed, thus lowering the fluid suspension cost.

So far, continuous ultrasound exposure has been described where the exposure time is chosen to obtain good follicular penetration. Instead of continuous application of ultrasound, pulsing can be employed which has some advantages. Pulsing is defined as a temporal train of ultrasound pulses, separated by times when ultrasound is turned off. For example, one can use 20 pulses, each consisting of 0.25 s on-time and 0.25 s off-time (translating to 50% duty cycle). In this case, the total time is 10 seconds and total on-time is 5 seconds. It has been observed that with pulsing, one can get higher performance when compared to the same total-time but delivered continuously. In addition to the higher performance, another benefit can be reduction in average heat released into the fluid by ultrasound, thus reducing the heat removal requirements.

### Example 1. Demonstration of tolerable treatment and performance in delivery to glands

An ultrasound applicator was placed on pre-auricular skin with water as the medium. The following was used: 40-KHz ultrasound, Sonics VCX134, 13 mm horn, gain of 3.6. Various distances and amplitudes were chosen. The subject was asked to rate the sound tolerability. Table 1 shows the tolerability of the total sound. As shown in Table 1, a set has been identified where the perception is well tolerated.

**Table 1: Ultrasound Perception when performed on pre-auricular skin**

| | Horn Distance | | | | |
|---|---|---|---|---|---|
| Amplitude | | 8-mm | 11-mm | 12-mm | 13-mm |
| | 30% | Loud | Tolerable | Loud (limited N) | Tolerable |
| | 35% | Loud | Tolerable | Tolerable | Tolerable |
| | 40% | Loud | Tolerable | Tolerable | Tolerable |

An experiment showing performance of delivery into the sebaceous glands was performed on an ex vivo epilated pig ear model. Pig ears were frozen after sacrifice and stored frozen. They were thawed prior to experimentation. Pig ear ridge hairs were wax epilated. Ultrasound delivery was performed, followed by wiping off the particles from the skin surface, and was followed by laser irradiation. In the treated area, individual follicles were examined by making a cut perpendicular to skin through the follicle under a dissecting microscope. These experiments investigating follicular delivery were done at various amplitude and skin-horn distance pairs with FP-78 formulation. The composition of FP-78 is as follows. All percentages are w/w. Stock suspension of sebashell particles of 1,200 OD: 25%, 190 proof ethanol: 54%, diisopropyl adipate: 20%, Polysorbate 80: 1%. Ultrasound delivery was performed for 60 seconds. Laser irradiation was performed with a 9 mm x 9 mm square spot at 50 J/cm² and 30 ms pulse duration. The fraction of glands affected was calculated by carefully cutting each follicle in the experimental zone and counting total number of glands and number of affected glands and calculating the ratio of affected glands divided by the total number of glands. This data is provided in Table 2. These fractions are promising as they are much better than the fractions obtained by massage alone which are typically less than 5% in this model.

**Table 2: Penetration into Sebaceous glands in an Epilated Pig Ear Model**

| | Skin-Horn Distance | | | | | |
|---|---|---|---|---|---|---|
| Amplitude | | 8-mm | 11-mm | 12-mm | 13-mm | 14-mm |
| | 28-30% | NA | NA | 51% | 62% | 72% |
| | 35% | NA | 54% | 52% | 52% | 41% |
| | 40% | 46% | 34% | 44% | NA | NA |

Table 1 indicates that the perception is tolerable when the skin-horn distance is about 11 mm or higher. It can be discerned from Table 2 that a good fraction of sebaceous glands are affected, much higher than 'massage alone' when the amplitude ranges from about 28% to 40%. Thus, this leads to a range of about 11 mm-14 mm for the horn-skin distance and a range of about 28%-40% for the ultrasound horn amplitude. Distances higher than 14 mm will lead to even lower perception and it is possible to choose appropriate ultrasound horn amplitude to get acceptable performance. However, higher skin-horn distance can have the disadvantage of higher size of the applicator and increased amount of fluid required.

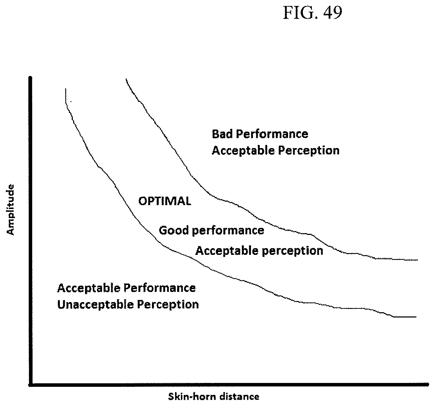

As a guiding principle, various zones can be established upon the work as in Figure 49. Figure 49 illustrates a graph showing performance and perception as a function of skin-horn distance (x-axis) and ultrasound horn amplitude (y-axis). As described above and shown in FIG. 49, there may be an optimal range or band in which good performance and acceptable perception can both be achieved.

### Example 2. Demonstration of enhanced pitting on Al-foils with use of pulsed ultrasound.

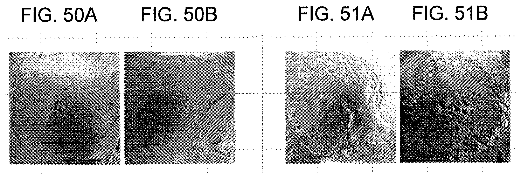

The number of jets impinging on the skin-surface during treatment can be estimated by performing an experiment on Al foil placed at the same distance as the skin surface and by observing the pitting on the foil. A Franz cell with 25 mm diameter was filled with 8 mL tap water. The ultrasound source was VCX134 with a frequency of 40 kHz. An ultrasound horn of 13-mm diameter and 3.6 gain was immersed in it at a distance of 13 mm from an Al foil. The bottom cell contained water. The photographs of Al foil from the experiment are shown in Figures 50A-51B. The Al foil in FIGs. 50A and 50B was subject to continuous ultrasound, lasting for 30 s. The Al foil in FIGs. 51A and 51B was subject to pulsed ultrasound, lasting for a total of 30 s. Each pulse consisted of 1 s on time and 1 s off time, thus a 50% duty cycle. As shown in FIGs. 50A and 50B, the Al foil subject to continuous ultrasound show a very low number of pits. The Al foil subject to pulsed ultrasound, shown in FIGs. 51A and 51B show a higher number of pits, even though the actual on-time is half. Thus, for the same total time, pulsing can lead to better microject impingement at the surface, though the net on-time is lower.

### Example 3. Demonstration of enhanced pitting on Al-foils with use of pulsed ultrasound.

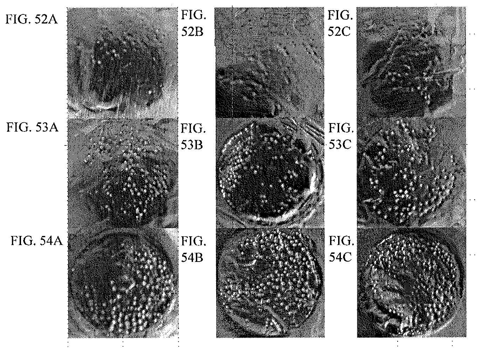

A Franz cell with 25 mm diameter was filled with 8 mL tap water. The ultrasound source was VCX134 with a frequency of 40 kHz. An ultrasound horn of 13-mm diameter and 3.6 gain was immersed at a distance of 13 mm from an Al foil. The bottom cell contained water. Three experiments were performed. The photographs are presented in Figures 52A-54C.

The Al foil in FIGs. 52A-52C were subject to a continuous 10 s of ultrasound. The Al foil in FIGs. 53A-53C were subject to pulsed ultrasound for a total of 10 s, with each pulse being 1 s with a 1 s pause between pulses. The Al foil in FIGs. 54A-54C were subject to pulsed ultrasound for a total of 10 s, with each pulse being 0.1 s, with a 0.1 s pause between pulses. As shown in FIGs. 52A-52C, the Al foil subject to continuous ultrasound has a minimal number of pits. The Al foil subject to 1s pulsed ultrasound, shown in FIGs. 53A-53C has a higher number of pits as compared to the Al foil subject to continuous ultrasound. The Al foil shown in FIGs. 54A-54C, subject to 0.1 s pulsed ultrasound, has a higher number of pits as compared to the Al foil shown in FIGs., 53A-53C, subject to 1 s pulsing.

FIGs. 52A-54C show that pulsing can lead to better microjet formations at the surface than continuous ultrasound exposure. Additionally, pulsing with shorter pulses can be better than pulsing with longer pulses for obtaining microjets in the proximity of the surface.

### Example 4. Gland delivery performance in an ex vivo pig ear model with pulsing and low skin-horn distance.

Ex vivo pig ear experiments as described in Example 1 were done but with a short skin-horn distance of 1 and 3 mm. The results are described in Table 3 below.

**Table 3: Performance of glands and deep-glands involvement with pulsing and short skin-horn distances of 1 and 3 mm**

| Skin-horn dist. | Amplitude | Total time | Pulse-on time | Pulse-off time | Duty cycle | % glands involved | % deep glands involved |
|---|---|---|---|---|---|---|---|
| 1 mm | 60% | 20 s | 1 s | 1 s | 50% | 60% | 20% |
| 1 mm | 60% | 20 s | 0.5 s | 0.5 s | 50% | 45% | 21% |
| 1 mm | 60% | 20 s | 0.25 s | 0.25 s | 50% | 52% | 24% |
| 1 mm | 100% | 20 s | 0.5 s | 0.5 s | 50% | 22% | 4% |
| 1 mm | 100% | 10 s | 0.5 s | 0.5 s | 50% | 14% | 0% |
| 1 mm | 80% | 20 s | 0.5 s | 0.5 s | 50% | 48% | 22% |
| 1 mm | 80% | 20 s | 0.25 s | 0.25 s | 50% | 67% | 20% |
| 1 mm | 80% | 10 s | 0.25 s | 0.25 s | 50% | 55% | 13% |
| 1 mm | 90% | 20 s | 0.5 s | 0.5 s | 50% | 16% | 2% |
| 1 mm | 90% | 20 s | 0.25 s | 0.25 s | 50% | 47% | 16% |
| 3 mm | 80% | 20 s | 0.25 s | 0.25 s | 50% | 63% | 26% |
| 3 mm | 80% | 10 s | 0.25 s | 0.25 s | 50% | 58% | 26% |
| 3 mm | 100% | 10 s | 0.25 s | 0.25 s | 50% | 35% | 11% |

There is some expected variation due to the variability in pig ears and inherent variability in the analysis. However, there are trends that lead to several inferences, some of which are:
- Sebaceous gland and deep sebaceous gland involvement is noted. It is believed that this can lead to a significant improvement in acne appearance when targeting acne.
- Decreasing the total time can reduce the fraction of deep sebaceous gland involvement.
- Reducing the pulsing time from 1 s to 0.5 s, and to 0.25 s can lead to improvement in fraction of deep sebaceous glands that show treatment effect.
- Lower amplitude can leads to better deep sebaceous gland involvement.

There is not very strong dependence on the skin-horn distance in the range of 1 to 3 mm. The perception with higher amplitudes, for example, 100%, is tolerable and hence, such parameters can be used in the treatments to achieve desired improvement of the follicular disease or condition.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/-10% of the stated value (or range of values), etc. Any numerical range recited herein is intended to include all sub-ranges subsumed therein.

Although various illustrative embodiments are described above, any of a number of changes may be made to various embodiments without departing from the scope of the invention as described by the claims. For example, the order in which various described method steps are performed may often be changed in alternative embodiments, and in other alternative embodiments one or more method steps may be skipped altogether. Optional features of various device and system embodiments may be included in some embodiments and not in others. Therefore, the foregoing description is provided primarily for exemplary purposes and should not be interpreted to limit the scope of the invention as it is set forth in the claims.

The examples and illustrations included herein show, by way of illustration and not of limitation, specific embodiments in which the subject matter may be practiced. In one aspect, the operation of the delivery device for the delivery of a delivery fluid is the desired therapy. In this case, the operation of the delivery device is a complete treatment operation. In another aspect, the operation of the delivery device for the delivery of a delivery fluid precedes or follows another treatment or another desired therapy. In this case, the operation and use of the delivery device is one part of a multi-part therapy. In one specific example of a multiple part therapy is the use of the delivery system to deliver a fluid, a formulation particles, shells, pharmaceuticals, liposomes, other treatment agents or pharmacologic materials onto, into or within a structure within a treatment or delivery site followed by a further treatment of the delivery or treatment site. In one specific example the further treatment is providing an activating energy to a fluid, a formulation or a pharmacologic material. Exemplary fluids, formulations and treatments are described in U.S. Patent 6,183,773; U.S. Patent 6,530,944; U.S. Published Patent Application US 2013/0315999, U.S. Published Patent Application US 2013/0323305 and U.S. Published Patent Application US 2012/005 9307.

An object of the subject matter described herein is to provide compositions, methods and systems for noninvasive and minimally-invasive treatment of skin and underlying tissues, or other accessible tissue spaces with the use of nanoparticles, including the use of nanoparticles or particles, modified particle formulations, enhanced particle formulations or formulations having additional materials selected so as to enhance one or more ultrasound transport modes for a nanoparticle mixture, mircoparticle mixture or particle mixture. The treatment includes, but is not limited to, hair removal, hair growth and regrowth, and skin rejuvenation or resurfacing, acne removal or reduction, wrinkle reduction, pore reduction, ablation of cellulite and other dermal lipid depositions, wart and fungus removal, thinning or removal of scars including hypertrophic scars and keloids, abnormal pigmentation (such as port wine stains), tattoo removal, and skin inconsistencies (e.g. in texture, color, tone, elasticity, hydration). Other therapeutic or preventative methods include but are not limited to treatment of hyperhidrosis, anhidrosis, Frey's Syndrome (gustatory sweating), Horner's Syndrome, and Ross Syndrome, actinici keratosis, keratosis follicularis, dermatitis, vitiligo, pityriasis, psoriasis, lichen planus, eczema, alopecia, psoriasis, malignant or non-malignant skin tumors,

Unless explained otherwise, all technical and scientific terms used herein have the same meaning as commonly understood to one of ordinary skill in the art to which this disclosure belongs. Although methods and materials similar or equivalent to those described herein can be used in the practice or testing of the present disclosure, suitable methods and materials are described herein. The materials, methods, and examples are illustrative only and not intended to be limiting. Other features of the disclosure are apparent from the following detailed description and the claims.

"Administer" and "administration" as used herein, include providing or causing the provision of a material to a subject, such as by a topical, subdermal, subcutaneous, intradermal, enteral, parenteral, rectal, nasal, intravenous, intramuscularly, intraperitoneal, or other route.

A "carrier suitable for administration" to a subject is any material that is physiologically compatible with a topical or route of administration to a desired vertebrate subject. Carriers can include solid-based, dry materials for formulation; or the carrier can include liquid or gel-based materials for formulations into liquid or gel forms. The specific type of carrier, as well as the final formulation depends, in part, upon the selected route(s) of administration and the type of product and, optionally, modification of or addition of one of more materials to enhance the suitability of a formulation having a carrier to one or more ultrasound transport modes.

A "comparable amount" is an amount that is measurably similar to a given reference or standard.

The "components" of a formulation include any products or compounds associated with or contained within it.

An "effective dose", "effective amount" or "therapeutic amount" is an amount sufficient to elicit the desired pharmacological, cosmetic or therapeutic effects, thus resulting in effective prevention or treatment of a disease or disorder, or providing a benefit in a vertebrate subject.

A "therapeutic effect" or "therapeutically desirable effect" refers to a change in a domain or region being treated such that it exhibits signs of being effected in the manner desired, e.g., cancer treatment causes the destruction of tumor cells or halts the growth of tumor cells, acne treatment causes a decrease in the number and/or severity of blemishes, hair removal treatment leads to evident hair loss, or wrinkle reduction treatment causes wrinkles to disappear.

An "isolated" biological component (such as a nucleic acid molecule, protein, or cell) has been substantially separated or purified away from other biological components in which the component was produced, including any other proteins, lipids, carbohydrates, and other components.

A "nanoparticle", as used herein, refers generally to a particle having at least one of its dimensions from about 0.1 nm to about 9000 nm.

A "subject" or "patient" as used herein is any vertebrate species.

As used herein, a "substantially pure" or "substantially isolated" compound is substantially free of one or more other compounds.

A "target tissue" includes a region of an organism to which a physical or chemical force or change is desired. As described herein, exemplary target tissues for acne treatment include a sebaceous gland, while exemplary target tissues for hair removal include a pilosebaceous unit, a hair infundibulum, a hair follicle, or a non-follicular epidermis. A "region" of a target tissue includes one or more components of the tissue. Exemplary target tissue regions include the stem cell niche, bulge, sebaceous gland, dermal papilla, cortex, cuticle, inner root sheath, outer root sheath, medulla, Huxley layer, Henle layer or pylori muscle. A "domain" of a target tissue region includes basement membrane, extracellular matrix, cell-surface proteins, unbound proteins/analytes, glycomatrices, glycoproteins, or lipid bilayer.

A compound that is "substantially free" of some additional contents is largely or wholly without said contents.

A "plasmonic nanoparticle" is a nanometer-sized metallic structure within which localized surface plasmons are excited by light. These surface plasmons are surface electromagnetic waves that propagate in a direction parallel to the metal/dielectric interface (e.g., metal/air or metal/water).

A "light-absorbing nanomaterial" includes a nanomaterial capable of demonstrating a quantum size effect.

As described herein, provided are compositions that contain plasmonic nanoparticles to induce selective thermomodulation in a target tissue.

### Plasmonic Nanoparticles

Such compositions contain from about 109 to about 1016 nanoparticles, such as 109, 1010, 1011, 1012, 1013, 1014, 1015, 1016 particles. Preferably, the compositions contain about 1011 to 1013 particles so that the amount of particles localized to an effective 1 ml treatment volumes is from 109 to 1011. In certain embodiments wherein increased concentration of nanoparticles to a target region is desired, compositions contain particle concentrations with optical densities (O.D.) of 10 O.D.-1000 O.D., or optical densities greater than 1,000 O.D. In some embodiments these correspond to concentrations of about 1-10% w/w or more of nanoparticles.

Nanoparticles may be homogenous or heterogeneous in size and other characteristics. The size of the nanoparticle is generally about 0.1 nm to about 5,000 nm in at least one dimension. Some variation in the size of a population of nanoparticles is to be expected. For example, the variation might be less than 0.01%, 0.1%, 0.5%, 1%, 5%, 10%, 15%, 25%, 50%, 75%, 100%, 200% or greater than 200%. In certain embodiments where optimal plasmonic resonance is desired, a particle size in the range of from about 10 nm to about 100 nm is provided. Alternatively, in embodiments where enhanced penetration of the nanoparticles into a target tissue region such as a hair follicle is desired, a particle size in the range of from about 100 nm to about 1000 nm is provided. Modulation of particle size present in the composition is also a useful means of concentrating the composition in a target domain. Further, as described herein, nanoparticles having a size range of from about 10 nm to about 100 nm can be used as component of a larger molecular structure, generally in the range of from about 100 nm to about 1000 nm. For example, the plasmonic nanoparticle can be surface coated to increase its size, embedded into an acceptable carrier, or it can be cross-linked or aggregated to other particles, or to other materials, that generate a larger particle. In certain embodiments where at least one dimension of at least one nanoparticle within a solution of plasmonic nanoparticles is below 50-100 nm, the nanoparticle surface can be coated with a matrix (e.g. silica) of 10-100 nm thickness or more in order to increase that dimension or particle to 50-100 nm or more. This increased dimension size can increase the delivery of all nanoparticles to a target region (e.g., hair follicle) and limit delivery to non-target region (e.g. dermis).

Important considerations when generating nanoparticles include: 1) the zeta potential (positive, negative, or neutral) and charge density of the particles and resulting compositions; 2) the hydrophilicity/hydrophobicity of the particles and resulting compositions; 3) the presence of an adsorption layer (e.g., a particle slippage plane); and 4) target cell adhesion properties. Nanoparticle surfaces can be functionalized with thiolated moieties having negative, positive, or neutral charges (e.g. carboxylic acid, amine, hydroxyls) at various ratios. Moreover, anion-mediated surface coating (e.g. acrylate, citrate, and others), surfactant coating (e.g., sodium dodecyl sulfate, sodium laureth 2-sulfate, ammonium lauryl sulfate, sodium octech-1/deceth-1 sulfate, lecithin and other surfactants including cetyl trimethylammonium bromide (CTAB), lipids, peptides), or protein/peptide coatings (e.g. albumin, ovalbumin, egg protein, milk protein, other food, plant, animal, bacteria, yeast, or recombinantly-derived protein) can be employed. Block-copolymers are also useful. Further, one will appreciate the utility of any other compound or material that adheres to the surface of light-absorbing particles to promote or deter specific molecular interactions and improve particle entry into pores or follicles. In some embodiments, the particle surface is unmodified. Modulation of hydrophilicity versus hydrophobicity is performed by modifying nanoparticle surfaces with chemistries known in the art, including silanes, isothiocyanates, short polymers (e.g., PEG), or functionalized hydrocarbons. Polymer chains (e.g., biopolymers such as proteins, polysaccharides, lipids, and hybrids thereof; synthetic polymers such as polyethyleneglycol, PLGA, and others; and biopolymer-synthetic hybrids) of different lengths and packing density are useful to vary the adsorption layer/slippage plane of particles.

Optical absorption. Preferred nanoparticles have optical absorption qualities of about 10 nm to about 10,000 nm, e.g., 100-500 nm. In specific embodiments, the nanoparticles have optical absorption useful to excitation by standard laser devices or other light sources. For example, nanoparticles absorb at wavelengths of about 755 nm (alexandrite lasers), in the range of about 800-810 nm (diode lasers), or about 1064 nm (Nd: YAG lasers). Similarly, the nanoparticles absorb intense pulsed light (IPL), e.g., at a range of about 500 nm to about 1200 nm.

Assembly. The nanoparticles provided herein can generally contain a collection of unassembled nanoparticles. By "unassembled" nanoparticles it is meant that nanoparticles in such a collection are not bound to each other through a physical force or chemical bond either directly (particle-particle) or indirectly through some intermediary (e.g. particle-cell-particle, particle-protein-particle, particle-analyte-particle). In other embodiments, the nanoparticle compositions are assembled into ordered arrays. In particular, such ordered arrays can include any three dimensional array. In some embodiments, only a portion of the nanoparticles are assembled, e.g., 5, 10, 15, 20, 25, 30, 35, 40, 45, 50, 55, 60, 65, 70, 75, 80, 86, 90, 95, 99% or greater than 99% of the nanoparticles are assembled in an ordered array. The nanoparticles are assembled by a van der Walls attraction, a London force, a hydrogen bond, a dipole-dipole interaction, or a covalent bond, or a combination thereof.

"Ordered array" "Ordered arrays" can take the form of a macrostructure from individual parts that may be patterned or unpatterned in the form of spheres, colloids, beads, ovals, squares, rectangles, fibers, wires, rods, shells, thin films, or planar surface. In contrast, a "disordered array" lacks substantial macrostructure.

Geometrically tuned nanostructures. The nanoparticles provided herein are formable in all shapes currently known or to be created that absorb light and generate a plasmon resonance at a peak-wavelength or composition of wavelengths from 200 nm to 10,000 nm. In non-limiting examples, the nanoparticles are shaped as spheres, ovals, cylinders, squares, rectangles, rods, stars, tubes, pyramids, stars, prisms, triangles, branches, plates or comprised of a planar surface. In non-limiting examples, the plasmonic particles comprise nanoplates, solid nanoshells, hollow nanoshells nanorods, nanorice, nanospheres, nanofibers, nanowires, nanopyramids, nanoprisms, nanoplates or a combination thereof. Plasmonic particles present in the composition comprise a substantial amount of geometrically-tuned nanostructures defined as 5, 10, 15, 25, 50, 75, 80, 85, 90, 95, 98, 99, 99.9 or greater than 99.9% of particles.

Composition. The nanoparticle is a metal (e.g., gold, silver), metallic composite (e.g., silver and silica, gold and silica), metal oxide (e.g. iron oxide, titanium oxide), metallic salt (e.g., potassium oxalate, strontium chloride), intermetallic (e.g., titanium aluminide, alnico), electric conductor (e.g., copper, aluminum), electric superconductor (e.g., yttrium barium copper oxide, bismuth strontium calcium copper oxide), electric semiconductor (e.g., silicon, germanium), dielectric (e.g., silica, plastic), or quantum dot (e.g., zinc sulfide, cadmium selenium). In non-limiting examples, the materials are gold, silver, nickel, platinum, titanium, palladium, silicon, galadium. Alternatively, the nanoparticle contains a composite including a metal and a dielectric, a metal and a semiconductor, or a metal, semiconductor and dielectric.

Coating. Preferentially, the composition contains coated nanoparticles.

| **Type of Material** | **Properties** | **Exemplary Materials** |
|---|---|---|
| biorecognitive material | Moiety with affinity or avidity for a substrate or analyte | Antibody, peptide, phage, DNA, RNA |
| bioactive material | Moiety (e.g., protein, analyte) that interrogates or modulates the activity of biologic entity or cell | Growth factor (e.g. VEGF), cytokine, cell surface receptors, receptor ligands, G-protein, kinase/ phosphatase |
| biological material | Material that is sourced from living matter | albumin, ovalbumin, egg protein, milk protein, other food, plant, animal, bacteria, yeast, or recombinantly-derived protein; peptides; enzymes, lipids, fatty acids, sugars |
| biocide material | Material that is active in killing, destroying, or disturbing biological matter | Synthetic or natural pesticides, synthetic or natural antimicrobials |
| dielectric materials | An insulator that may be polarized by an electric field | Silicon, doped semiconductors |
| chemorecognitive material | Material that is able to interact with a moiety for binding, biological or chemical reactions | Receptor, receptor ligand, chemical molecule |
| chemical active material | Material that causes the transformation of a substance | Aldehyde, halogens, metals |
| Polymer/ dendrimer | Long chain molecule (linear or branched, block or co-block) | PLGA, PEG, PEO, polystyrene, carboxylate styrene, rubbers, nylons, silicones, polysaccharides |
| environmentally sensitive polymer | Surface molecule that changes by its environment (e.g. acid) | Ph sensitive bond, light sensitive bond, heat sensitive bond, enzyme sensitive bond, hydrolytic bond |
| Hydrogel | Polymer with high hydrophilicity and water "ordering" capacity | Synthetic 2-hydroxyethyl metacrylate (HEMA)- based, polyethylene glycol (PEG)-based, PLGA, PEG- diacrylate; Natural ionic gels, alginate, gelatin, hyaluronic acids, fibrin |
| Metal | Thin metal coating to achieve improved resonance and/or functionalization capacity | Gold, silver, nickel, platinum, titanium, and palladium. |
| Semiconductors | Semiconductor layer or core that enhance Plasmon resonance | Silicon and galadium. |
| polymer containing a fluorescent marker | Fluorophore cross linked to a polymer coat or directly to the surface of the particle | Fluorescein, rhodamine, Cy5, Cy5.5, Cy7, Alexa dyes, Bodipy dyes |
| Matrix | Matrix coating that increases solubility of nanoparticles and/or reduces "stickiness" to biological structures | Silica, polyvinyl pyrrolidone, polysulfone, polyacrylamide, polyethylene glycol, polystyrene cellulose, carbopol. |

Biological molecules. The composition may contain a peptide, a nucleic acid, a protein, or an antibody. For example a protein, antibody, peptide, or nucleic acid that binds a protein of a follicular stem cell (e.g., keratin 15), a protein, glycomatrix, or lipid on the surface of a cell or stem cell, a protein, peptide, glycomatrix of the extracellular matrix or basement membrane.

Charged moieties. The coated nanoparticles may contain charged moieties whereby those charges mediate enhanced or diminished binding to components within or outside the hair follicle via electrostatic or chemical interactions.

| **Class of Moiety** | **Properties** | **Exemplary Moieties** |
|---|---|---|
| Polar moieties | Neutral charge but increases hydrophilicity in water | Hydroxyl groups, isothiocyanates |
| Non-polar moieties | Increases hydrophobicity and or improves solubility | Hydrocarbons, myristoylated compounds, silanes, isothiocyanates |
| Charged moieties | Functional surface modifications that change the zeta potential, isoelectric point, or pKa, and impact adsorption/binding to complementary charge compounds | Amines, carboxylic acids, hydroxyls |
| Ionic moieties | Surface groups that have a single ion | Ammonium salts, chloride salts |
| Basic moieties | Groups that donate a hydrogen ions | Amides, hydroxides, metal oxides, fluoride |
| Acidic moieties | Moieties that accept hydrogen ions | Carboxylic acids, sulfonic acids, mineral acids |
| Oxidative moieties | Moieties that oxidize | Manganese ions, reactive oxygen species |
| Hydrophobic moieties | Moieties that improve solubility in non-aqueous solution and/or improve adsorption on the skin within a hair follicle | Hydrocarbons, myristoylated compounds, silanes |
| Hydrophilic moieties | Moieties that are water-loving and prevent adsorption | PEG, PEO, PLGA |
| Agnostic moieties | Moieties that bind a target cell, structure, or protein of interest | Antibodies, peptides, proteins |
| Antagonistic moieties | Moieties that block the binding to a target of interest | Antibodies, peptides, proteins |
| Reactive moieties | Moieties that react with biological or non-biological components with a resulting change in structure on the target or | Aldehydes |

### Description of Target Tissues.

Topical and Dermatological Applications. Target tissues for topical and dermatological applications include the surface of the skin, the epidermis and the dermis. Diseases or conditions suitable for treatment with topical and dermatological applications include acne, warts, fungal infections, psoriasis, scar removal, hair removal, hair growth, reduction of hypertrophic scars or keloids, skin inconsistencies (e.g. texture, color, tone, elasticity, hydration), and malignant or non-malignant skin tumors

As used herein, the term "acne" includes acne vulgaris as well as other forms of acne and related cutaneous conditions, including acne aestivalis, acne conglobata, acne cosmetic, acne fulminans, acne keloidalisnuchae, acne mechanica, acne miliarisnecrotica, acne necrotica, chloracne, drug-induced acne, excoriated acne, halogen acne, lupus miliaris disseminates faciei, pomade acne, tar acne, and tropical acne.

Subdermal Applications. Target tissues for subdermal applications include the adipose tissue and connective tissue below the integumentary system. Diseases or conditions suitable for treatment with subdermatological applications include wrinkles and tattoos. Other applications include skin rejuvenation and/or resurfacing, the removal or reduction of stretch marks and fat ablation.

Often, a specific region of the target tissue is a hair follicle, a sebaceous gland, a merocrine sweat gland, an apocrine sweat gland, or an arrector pili muscle, within which a specific domain is targeted. For example, the bulge region of the hair follicle is targeted. Because in one embodiment the nanoparticles are useful to thermally ablate hair follicle stem cells for hair removal, regions containing hair follicle stem cells are of particular interest for targeting. Thus, the target tissue region may include a stem cell niche, bulge, sebaceous gland, dermal papilla, cortex, cuticle, inner root sheath, outer root sheath, medulla, Huxley layer, Henle layer or pylori muscle. Each of these regions may contain cells, stem cells, basement membrane, extracellular matrix, growth factors, analytes, or other biologic components that mediate hair follicle rejuvenation. Disruption or destruction of these components would have a therapeutic effect, e.g. slow or stop the processes that mediate hair regrowth, prevent the secretion of sebum from the sebaceous gland, damage or deter tumor cells, reduce the appearance of wrinkles. Structures can also be targeted that are in close proximity to a desired target for ablation, especially when capable of conducting heat effectively.

Localization Domains. Provided are compositions containing nanoparticles that preferentially localize to a domain of a target tissue region of a mammalian subject to whom the composition is administered.

Targeting moieties. The nanoparticles can be engineered to selectively bind to a domain of the target tissue. For example, the nanoparticles are operably linked to the domain via a biologic moiety, in order to effectively target the nanoparticles to the target tissue domain. Preferably, the moiety contains a component of a stem cell, a progenitor cell, an extracellular matrix component, a basement membrane component, a hair shaft component, a follicular epithelial component, or a non-follicular epidermal component. Biological moieties include proteins such as cell surface receptors, glycoproteins or extracellular matrix proteins, as well as carbohydrates, analytes, or nucleic acids (DNA, RNA) as well as membrane components (lipid bilayer components, microsomes).

Delocalization Domains. Nanoparticles present in the composition preferentially delocalize away from a domain of a target tissue region. Delocalization domains include specific regions of a tissue into which nanoparticles do not substantially aggregate, or alternatively, are removed from the domain more effectively. In preferred embodiments, the delocalization domain is a non-follicular epidermis, dermis, a component of a hair follicle (e.g., a hair stem cell, a stem cell niche, a bulge, a sebaceous gland, a dermal papilla, a cortex, a cuticle, an inner root sheath, an outer root sheath, a medulla, a Huxley layer, a Henle layer, a pylori muscle), a hair follicle infundibulum, a sebaceous gland, a component of a sebaceous gland, a sebocyte, a component of a sebocyte, or sebum

Energy sources. Provided herein are nonlinear excitation surface plasmon resonance sources, which include various light sources or optical sources. Exemplary light sources include a laser (ion laser, semiconductor laser, Q-switched laser, free-running laser, or fiber laser), light emitting diode, lamp, the sun, a fluorescent light source or an electroluminescent light source. Typically, the energy source is capable of emitting radiation at a wavelength from about 100, 200, 300, 400, 500, 1000, 2000, 5000 nm to about 10,000 nm or more. The nonlinear excitation surface plasmon resonance source is capable of emitting electromagnetic radiation, ultrasound, thermal energy, electrical energy, magnetic energy, or electrostatic energy. For example, the energy is radiation at an intensity from about 0.00005 mW/cm2 to about 1000 TW/cm2. The optimum intensity is chosen to induce high thermal gradients from plasmonic nanoparticles in regions from about 10 microns to hundreds of microns in the surrounding tissue, but has minimal residual effect on heating tissue in which particles do not reside within a radius of about 100 microns or more from the nanoparticle. In certain embodiments, a differential heat gradient between the target tissue region and other tissue regions (e.g., the skin) is greater than 2-fold, 3-fold, 5-fold, 10-fold, 15-fold, 20-fold, 50-fold, 100-fold, or greater than 100 fold.

The energy can be tuned by monitoring thermal heat gradients on the surface of the skin with a thermal/infrared camera. As demonstrated herein, the methods and systems of the present disclosure provide superior efficacy when a surface plasmon is generated on the nanoparticles by the action of the radiation. Typically, the plasmon is generated in a one-photon mode or, alternatively, a two-photon mode, a multi-photon mode, a step-wise mode, or an up-conversion mode.

Delivery of radiation. Physical means of delivery of the energy from the nonlinear excitation surface plasmon resonance source to the target tissue region include a fiber, waveguide, a contact tip or a combination thereof.

Optical sources include a CW optical source or a pulsed optical source, which may be a single wavelength polarized (or, alternatively, unpolarized) optical source capable of emitting radiation at a frequency from about 200 nm to about 10,000 nm. Alternatively, the optical source is a multiple wavelength polarized (or, alternatively, unpolarized) optical source capable of emitting radiation at a wavelength from about 200 nm to about 10,000 nm. The pulsed optical source is generally capable of emitting pulsed radiation at a frequency from about 1 Hz to about 1 THz. The pulsed optical source is capable of a pulse less than a millisecond, microsecond, nanosecond, picoseconds, or femtosecond in duration. The optical source may be coupled to a skin surface cooling device to reduce heating of particles or structures on the skin surface and focus heating to components within follicles or tissue structures at deeper layers.

Nanoparticle-containing compositions. In order to provide optimal dermal penetration into the target tissue, the plasmonic nanoparticles in certain embodiments are formulated in various compositions. Preferentially, the nanoparticles are formulated in compositions containing 1-10% v/v surfactants (e.g. sodium dodecyl sulfate, sodium laureth 2-sulfate, ammonium lauryl sulfate, sodium octech-1/deceth-1 sulfate). Surfactants disrupt and emulsify sebum or other hydrophobic fluids to enable improved targeting of hydrophilic nanoparticles to the hair follicle, infundibulum, sebaceous gland, or other regions of the skin. Surfactants also lower the free energy necessary to deliver hydrophilic nanoparticles into small hydrophobic crevices such as the space between the hair shaft and follicle or into the sebaceous gland. Nanoparticle-containing compositions may also include emulsions at various concentrations (1-20% w/v) in aqueous solutions, silicone/oil solvents, propylene glycol or creams (e.g. comprising alcohols, oils, paraffins, colloidal silicas). In other embodiments, the formulation contains a degradable or non-degradable polymer, e.g., synthetic polylactide/co-glycolide co-polymer, porous lauryllactame/caprolactame nylon co-polymer, hydroxyethylcellulose, polyelectrolyte monolayers, or alternatively, in natural hydrogels such as hyaluronic acid, gelatin and others. In further embodiments, a hydrogel PLGA, PEG-acrylate is included in the formulation. Alternatively, a matrix component such as silica, polystyrene or polyethylene glycol is provided in the formulation. Other formulations include components of surfactants, a lipid bilayer, a liposome, or a microsome. A nanoparticle may comprise a larger micron-sized particle.

Effective doses. As described herein, an effective dose of the nanoparticle-containing compositions includes an amount of particles required, in some aspects, to generate an effective heat gradient in a target tissue region, such that a portion of the target tissue region is acted upon by thermal energy from excited nanoparticles. A "minimal effective dose" is the smallest number or lowest concentration of nanoparticles in a composition that are effective to achieve the desired biological, physical and/or therapeutic effect(s). Preferentially, the plasmonic nanoparticles have an optical density of 10 O.D.-1 ,000 O.D. at one or a plurality of peak resonance wavelengths.

Cosmetically acceptable carriers. Provided are cosmetic or pharmaceutical compositions with a plurality of plasmonic nanoparticles and a cosmetically or pharmaceutically acceptable carrier. Generally, the carrier and composition must be suitable for topical administration to the skin of a mammalian subject, such that the plasmonic nanoparticles are present in an effective amount for selective thermomodulation of a component of the skin. Preferentially, the nanoparticles are formulated with a carrier containing 1-10% v/v surfactants (e.g. sodium dodecyl sulfate, sodium laureth 2-sulfate, ammonium lauryl sulfate, sodium octech-1/deceth-1 sulfate) to enable disruption of the epidermal skin barrier, emulsify sebum, improve mixing of hydrophilic nanoparticles with hydrophobic solutions, and reduce entropic barriers to delivering hydrophilic particles to hydrophobic regions of the skin (e.g. between the hair shaft and surrounding sheath or follicle). In some embodiments, the carrier contains a polar or non-polar solvent. For example, suitable solvents include alcohols (e.g., n-Butanol, isopropanol, n-Propanol, Ethanol, Methanol), hydrocarbons (e.g., pentane, cyclopentane, hexane, cyclohexane, benzene, toluene, 1,4-Dioxane), chloroform, Diethyl-ether, water, water with propylene glycol, acids (e.g., acetic acid, formic acid), bases, acetone, isooctanes, dimethyl sulfoxide, dimethylformamide, acetonitrile, tetrahydrofuran, dichloromethane, ethylacetate, tetramethylammonium hydroxide, isopropanol, and others. In other embodiments, a stabilizing agent such as antioxidants, preventing unwanted oxidation of materials, sequestrants, forming chelate complexes and inactivating traces of metal ions that would otherwise act as catalysts, emulsifiers, ionic or non-ionic surfactants, cholesterol or phospholipids, for stabilization of emulsions (e.g. egg yolk lecithin, Sodium stearoyllactylate, sodium bis(2-ethylhexyl-sulfosuccinate (AOT)), ultraviolet stabilizers, protecting materials, especially plastics, from harmful effects of ultraviolet radiation is provided. In further embodiments, a composition with a cosmetically acceptable carrier is generated such that the nanoparticles are substantially in a suspension.

Other components are also optionally included, including an emulsion, polymer, hydrogel, matrix, lipid bilayer, liposome, or microsome. Additionally, inclusion of a detectable colorant (e.g., a pigment), a fragrance, a moisturizer, and/or a skin protectant is optional. In some examples, the formulation has a viscosity of above, below or within 0.1-1000 as measured in millipascal-seconds (mPa•s).

Nanoparticle quantities per milliliter in a composition are subject to modification for specific binding and can range from 109 to1018 particles but generally about 1011 to 1013 nanoparticles per milliliter. In certain embodiments wherein increased concentration of nanoparticles to a target region is desired, compositions contain particle concentrations with optical densities of 10 O.D.-1000 O.D., or optical densities greater than 1,000 O.D. In some embodiments these correspond to concentrations of about 0.1-10% w/w or more of nanoparticles.

Prior to application of nanoparticle formulations, skin and hair follicles can be pre-treated to increase the delivery of nanoparticles to a target region. In some embodiments, hair shafts are cut or removed via shaving, waxing, cyanoacrylate surface peels, calcium thioglycolate treatment, or other techniques to remove the hair shaft and/or hair follicle plugs and create a void wherein nanoparticles can accumulate. Orifices of active or inactive follicles can be blocked by plugs formed of corneocytes and/or other material (e.g. cell debris, soot, hydrocarbons, cosmetics). In some embodiments pre-treatment with surface exfoliation including mechanical exfoliation (e.g., salt glow or microdermabrasion) and chemical exfoliation (e.g., enzymes, alphahydroxy acids, or betahydroxy acids) removes plugs from the orifice of follicles to increase the targeting of nanoparticle formulations to target regions within the hair follicle.

In some embodiments, the nanoparticle formulations are formulated for application by a sponge applicator, cloth applicator, direct contact via a hand or gloved hand, spray, aerosol, vacuum suction, high pressure air flow, or high pressure liquid flow, roller, brush, planar surface, semi-planar surface, wax, ultrasound and other sonic forces, mechanical vibrations, hair shaft manipulation (including pulling, massaging), physical force, thermal manipulation, and other treatments. In some embodiments, nanoparticle formulation treatments are performed alone, in combination, sequentially or repeated 1-24 times. In other embodiments, the plasmonic nanoparticles are capable of selectively localizing to a first component of the skin, where physical massage or pressure, ultrasound, or heat increase the selective localization of the nanoparticles to this first component. Additionally, the nanoparticles are selectively removable from components of the skin other than the first component, such removal accomplished with acetone, alcohol, water, air, peeling of the skin, chemical peeling, waxing, or reduction of the plasmonic compound. Further, in some embodiments the nanoparticles have a coat layer to increase solubility of the nanoparticles in the carrier and/or reduce "stickiness" and accumulation in non-target areas. The subject matter described herein also provides embodiments in which at least a portion of an exterior surface of the nanoparticle is modified, such as to include a layer of a polymer, polar monomer, non-polar monomer, biologic compound, a metal (e.g., metallic thin film, metallic composite, metal oxide, or metallic salt), a dielectric, or a semiconductor. Alternatively, the exterior surface modification is polar, non-polar, charged, ionic, basic, acidic, reactive, hydrophobic, hydrophilic, agonistic, or antagonistic. In certain embodiments where at least one dimension of at least one nanoparticle within a solution of plasmonic nanoparticles is below 50-100 nm, the nanoparticle surface can be coated with a matrix (e.g. silica) of 10-100 nm thickness or more in order to increase that dimension or particle to 50-100 nm or more. This increased dimension size can increase the delivery of all nanoparticles to a target region (e.g., hair follicle) and limit delivery to non-target region (e.g. dermis).

### Penetration Means.

Preferably, the compositions of the instant disclosure are topically administered. Provided herein area means to redistribute plasmonic particles from the skin surface to a component of dermal tissue including a hair follicle, a component of a hair follicle, a follicle infundibulum, a sebaceous gland, or a component of a sebaceous gland using high frequency ultrasound, low frequency ultrasound, massage, iontophoresis, high pressure air flow, high pressure liquid flow, vacuum, pre-treatment with Fractionated Photothermolysis laser or derm-abrasion, or a combination thereof. For example, the compositions can be administered by use of a sponge applicator, cloth applicator, spray, aerosol, vacuum suction, high pressure air flow, high pressure liquid flow direct contact by hand ultrasound and other sonic forces, mechanical vibrations, hair shaft manipulation (including pulling, massaging), physical force, thermal manipulation, or other treatments. Nanoparticle formulation treatments are performed alone, in combination, sequentially or repeated 1-24 times.

### Cosmetic and Therapeutic Uses of Plasmonic Nanoparticles.

In general terms, Applicant(s) have created systems and methods for the cosmetic and therapeutic treatment of dermatological conditions, diseases and disorders using nanoparticle-based treatments methods.

### Acne Treatment.

Acne is caused by a combination of diet, hormonal imbalance, bacterial infection (Propionibacterium acnes), genetic predisposition, and other factors. The nanoparticle-based methods and systems described herein for acne treatment are able to focally target causative regions of the dermis, the sebaceous gland and the hair follicle, and thus have advantages compared to the existing techniques known in the art, including chemical treatment (peroxides, hormones, antibiotics, retinoids, and antiinflammatory compounds), dermabrasion, phototherapy (lasers, blue and red light treatment, or photodynamic treatment), or surgical procedures.

In particular, laser-based techniques are becoming an increasingly popular acne treatment, but a substantial limitation is the lack of selective absorptive properties among natural pigments (e.g. fat, sebum) for specific wavelengths of light such that selective heating of one cell, structure, or component of tissue, particularly in the sebaceous glands, infundibulum, and regions of the hair follicle, is not achieved without heating of adjacent off-target tissue. The nanoparticles described herein provide significantly higher photothermal conversion than natural pigments enabling laser energy to be focused to specific cells, structures, or components of tissue within the sebaceous gland, infundibulum, or regions of the hair follicle for selective photothermal damage.

Using the materials and techniques described herein may provide acne treatments of greater duration than existing methodologies. In certain embodiments, tuned selective ablation of the sebaceous gland or infundibulum is achieved as described herein. In particular, plasmonic nanoparticles are specifically localized to regions of hair follicles in or proximate to the sebaceous gland or infundibulum.

Plasmonic nanoparticles exhibit strong absorption at wavelengths emitted by standard laser hair removal devices (e.g., 755 nm, 810 nm, 1064 nm) relative to surrounding epidermal tissue. Thus, irradiation of targeted plasmonic nanoparticles with laser light induces heat radiation from the particles to the adjacent sebum, sebaceous gland, infundibulum, and other acne causing agents.

### Hair Removal.

The nanoparticle-based methods and systems described herein for skin treatment have advantages compared to the existing techniques known in the art, including laser-based techniques, chemical techniques, electrolysis, electromagnetic wave techniques, and mechanical techniques (e.g., waxing, tweezers). Such techniques fail to adequately provide permanent hair removal across a breadth of subjects. In particular, subjects having light to medium-pigmented hair are not adequately served by these techniques, which suffer from side-effects including pain and the lack of beneficial cosmetic affects including hair removal. Laser-based techniques are popular in a variety of applications, but a substantial limitation is the lack of selective absorptive properties among natural pigments (e.g. melanin) for specific wavelengths of light such that selective heating of one cell, structure, or component of tissue is achieved without heating of adjacent off-target tissues. The nanoparticles described herein provide significantly higher photothermal conversion than natural pigments enabling laser energy to be focused to specific cells, structures, or components of tissue for selective photothermal damage.

More permanent reduction or removal of all hair types is provided herein, relative to hair removal treatments known in the art. In certain embodiments, tuned selective ablation of the hair shaft and destruction of stem cells in the bulge region is provided, as described herein. In particular, plasmonic nanoparticles are specifically localized to regions of hair follicles in or proximate to the bulge region, a stem cell-rich domain of the hair follicle. Moreover, the plasmonic nanoparticles are localized in close approximation of "50-75% of the hair shaft structure.

Plasmonic nanoparticles exhibit strong absorption at wavelengths emitted by standard laser hair removal devices (e.g., 755 nm, 810 nm, 1064 nm) relative to surrounding epidermal tissue. Thus, irradiation of targeted plasmonic nanoparticles with laser light induces heat radiation from the particles to the adjacent stem cells (or in some cases, the architecture of the hair shaft itself), resulting in cell death and a disruption of the normal regenerative pathway.

### Non-Malignant and Malignant Skin Tumors.

Laser therapies for the prevention and treatment of non-malignant, malignant, melanoma and non-melanoma skin cancers have been focused largely on photodynamic therapy approaches, whereby photosensitive porphyrins are applied to skin and used to localize laser light, produce reactive oxygen species and destroy cancer cells via toxic radicals. For example, 5-ALA combined with laser treatment has been FDA-approved for the treatment of non-melanoma skin cancer actinic keratoses, and it is used off-label for the treatment of widely disseminated, surgically untreatable, or recurrent basal cell carcinomas (BCC). However, this procedure causes patients to experiences photosensitivity, burning, peeling, scarring, hypo- and hyper-pigmentation and other side effects due to non-specific transdermal uptake of porphyrin molecules. The nanoparticles described herein provide significantly higher photothermal conversion than natural pigments and dyes, enabling laser energy to be focused to specific cells, structures, or components of tissue for selective thermomodulation

Using the materials and techniques described herein may provide cancer treatments of greater degree and duration than existing methodologies. In certain embodiments, tuned selective ablation of specific target cells as described herein. In particular, plasmonic nanoparticles are specifically localized to regions of hair follicles where follicular bulge stem cells arise to form nodular basal cell carcinomas and other carcinomas. Plasmonic nanoparticles may also be delivered to other target cells that cause tumors, for example, the interfollicular epithelium, which include the cell of origin for superficial basal cell carcinomas.

Plasmonic nanoparticles exhibit strong absorption at wavelengths emitted by standard laser hair removal devices (e.g., 755 nm, 810 nm, 1064 nm) relative to surrounding epidermal tissue. Thus, irradiation of targeted plasmonic nanoparticles with laser light induces heat radiation from the particles to the adjacent keratinocyte, melanocyte, follicular bulge stem cell, cancer cell, or cancer cell precursor, resulting in cell death or inhibited cell growth for cancer prevention and treatment.

Subdermal Applications. Target tissues for subdermal applications include the adipose tissue and connective tissue below the integumentary system. Diseases or conditions suitable for treatment with subdermatological applications include wrinkles and tattoos. Other applications include skin rejuvenation and/or resurfacing, the removal or reduction of stretch marks and fat ablation.

Vascular Applications. Target tissues for vascular applications include arteries, arterioles, capillaries, veins, and venules. Diseases or conditions suitable for treatment with vascular applications include spider veins, leaky valves, and vascular stenosis. In particular, vein abnormalities account for a substantial proportion of cosmetic diseases or conditions affecting the vasculature. Individuals with vein abnormalities such as spider veins or faulty venous valves suffer from pain, itchiness, or undesirable aesthetics.

Additionally, there are several indication for which ablation of other vessels including arteries, arterioles, or capillaries could provide therapeutic or cosmetic benefit including: 1) ablation of vasculature supplying fat pads and/or fat cells, 2) ablation of vasculature supporting tumors/cancer cells, 3) ablation of vascular birth marks (port-wine stains, hemangiomas, macular stains), and 4) any other indication whereby ablation of vessels mediates the destruction of tissue and apoptosis or necrosis of cells supported by those vessels with therapeutic or cosmetic benefit. Provided herein are methods for using the compositions described herein for the selective destruction of component(s) of veins from plasmonic nanoparticles focally or diffusely distributed in the blood. Plasmonic nanoparticles are combined with a pharmaceutically acceptable carrier as described above and are introduced into the body via intravenous injection. Nanoparticles diffuse into the blood and, in some embodiments, localize to specific vascular tissues. Subsequently, the nanoparticles are activated with laser or light-based systems as known in the art for treating skin conditions such as hair removal or spider vein ablation. Alternatively, image or non-image guided fiber optic waveguide-based laser or light systems may be used to ablate vessel or blood components in larger veins. In one embodiment, a device with dual functions for both injecting nanoparticles and administering light through on optical waveguide may be used. Activated nanoparticles heat blood and adjacent tissue (vessels, vessel walls, endothelial cells, components on or in endothelial cells, components comprising endothelial basement membrane, supporting mesenchymal tissues, cells, or cell components around the vessel, blood cells, blood cell components, other blood components) to ablative temperatures (38-50 degrees C. or higher).

Provided herein is a composition comprising a pharmaceutically acceptable carrier and a plurality of plasmonic nanoparticles in an amount effective to induce thermomodulation of a vascular or intravascular target tissue region with which the composition is intravenously contacted. Furthermore, the composition of plasmonic nanoparticle may comprise a microvascular targeting means selected from the group consisting of anti-microvascular endothelial cell antibodies and ligands for microvascular endothelial cell surface receptors. Also provided is a method for performing thermoablation of a target vascular tissue in a mammalian subject, comprising the steps of contacting a region of the target vascular tissue with a composition comprising a plurality of plasmonic nanoparticles and a pharmaceutically acceptable carrier under conditions such that an effective amount of the plasmonic nanoparticles localize to a domain of the target vascular region; and exposing the target tissue region to energy delivered from a nonlinear excitation surface plasmon resonance source in an amount effective to induce thermoablation of the domain of the target vascular region.

Oral and nasal Applications. Target tissues for oral applications include the mouth, nose, pharynx, larynx, and trachea. Diseases or conditions suitable for treatment with vascular applications include oral cancer, polyps, throat cancer, nasal cancer, and Mounier-Kuhn syndrome.

Endoscopic Applications. Target tissues for endoscopic applications include the stomach, small intestine, large intestine, rectum and anus. Diseases or conditions suitable for treatment with vascular applications include gastrointestinal cancer, ulcerative colitis, Crohn's disease, Irritable Bowel Syndrome, Celiac Disease, Short Bowel Sydrome, or an infectious disease such as giardiasis, tropical sprue, tapeworm infection, ascariasis, enteritis, ulcers, Whipple's disease, and megacolon.

Methods of thermomodulation. Provided are methods for performing thermomodulation of a target tissue region. A nanoparticle composition comprising a plurality of plasmonic nanoparticles under conditions such that an effective amount of the plasmonic nanoparticles localize to a domain of the target tissue region; and exposing the target tissue region to energy delivered from a nonlinear excitation surface plasmon resonance source in an amount effective to induce thermomodulation of the domain of the target tissue region.

### Removal of non-specifically bound nanoparticles.

Removing nanoparticles localized on the surface of the skin may be performed by contacting the skin with acetone, alcohol, water, air, a debriding agent, or wax. Alternatively, physical debridement may be performed. Alternatively, one can perform a reduction of the plasmonic compound.

Amount of energy provided. Skin is irradiated at a fluence of 1-60 Joules per cm2 with laser wavelengths of about, e.g., 750 nm, 810 nm, 1064 nm, or other wavelengths, particularly in the range of infrared light. Various repetition rates are used from continuous to pulsed, e.g., at 1-10 Hz, 10-100 Hz, 100-1000 Hz. While some energy is reflected, it is an advantage of the subject matter described herein is that a substantial amount of energy is absorbed by particles, with a lesser amount absorbed by skin. Nanoparticles are delivered to the hair follicle, infundibulum, or sebaceous gland at concentration sufficient to absorb, e.g., 1.1-100× more energy than other components of the skin of similar volume. This is achieved in some embodiments by having a concentration of particles in the hair follicle with absorbance at the laser peak of 1.1-100× relative to other skin components of similar volume.

To enable tunable destruction of target skin structures (e.g., sebaceous glands, infundibulum, hair follicles), light-absorbing nanoparticles are utilized in conjunction with a laser or other excitation source of the appropriate wavelength. The laser light may be applied continuously or in pulses with a single or multiple pulses of light. The intensity of heating and distance over which photothermal damage will occur are controlled by the intensity and duration of light exposure. In some embodiments, pulsed lasers are utilized in order to provide localized thermal destruction. In some such embodiments, pulses of varying durations are provided to localize thermal damage regions to within 0.05, 0.1, 0.5, 1, 2, 5, 10, 20, 30, 50, 75, 100, 200, 300, 500, 1000 microns of the particles. Pulses are at least femtoseconds, picoseconds, microseconds, or milliseconds in duration. In some embodiments, the peak temperature realized in tissue from nanoparticle heating is at least 5, 10, 15, 20, 25, 30, 40, 50, 60, 70, 80, 90, 100, 200, 300, or 500 degrees Celsius. In some embodiments that utilize pulsed heating, high peak temperatures are realized locally within the hair shaft without raising the macroscopic tissue temperature more than 0.1, 0.5, 1, 2, 3, 4, 5, 7, 9, 12, 15, or 20 degrees Celsius. In some embodiments short pulses (100 nanoseconds-1000 microseconds) are used to drive very high transient heat gradients in and around the target skin structure (e.g., sebaceous gland and/or hair follicle) from embedded particles to localize damage in close proximity to particle location. In other embodiments, longer pulse lengths (1-500 ms) are used to drive heat gradients further from the target structure to localize thermal energy to stem cells in the bulge region or other components greater than 100 µm away from the localized particles. Fluences of 1-30 Joules per cm2 are generally sufficient to thermally ablate follicles that have high particle concentrations and thus higher absorbance than skin (e.g., 1.1-100 times per volume absorbance of skin). These fluences are often lower than what is currently employed (e.g., Diode: 25-40 J/cm2, Alexandrite: 20 J/cm2, Nd:YAG: 30-60 J/cm2) and lead to less damage to non-follicular regions, and potentially less pain.

Plasmon Resonance Systems. Provided are plasmon resonance systems containing a surface that includes a plurality of plasmonic nanoparticles, and a nonlinear excitation source. Preferably, the surface is a component of skin that is targeted for cosmetic or therapeutic treatment (e.g., bulge region for hair removal, infundibulum or sebaceous gland for acne prevention). Also provided as a component of the system is a means for delivering plasmonic nanoparticles to the skin surface, such as an applicator, a spray, an aerosol, vacuum suction, high pressure air flow, or high pressure liquid flow. Further provided are means of localizing plasmonic nanoparticles to a component of the skin (e.g., hair follicle, bulge region, sebaceous gland, infundibulum). Useful surface delivery means include a device that generates high frequency ultrasound, low frequency ultrasound, heat, massage, contact pressure, or a combination thereof.
Further provided are systems that contain a removal means for removing nanoparticles on a non-follicular portion of the skin. The removal means includes at least one of acetone, alcohol, water, air, chemical peeling, wax, or a compound that reduces the plasmonic compound.

In addition, the systems of the present disclosure provide nonlinear excitation source that generates a continuous wave optical source or a pulsed optical source. Alternatively, the nonlinear excitation source is capable of generating electromagnetic radiation, ultrasound, thermal energy, electrical energy, magnetic energy, or electrostatic energy. Provided are systems wherein the nonlinear excitation source is capable of irradiating the nanoparticles with an intensity from about 0.00005 mW/cm2 to about 1000 TW/cm2. Further, the nonlinear excitation source is capable of functioning in a one-photon mode, two-photon mode, multi-photon mode, step-wise mode, or up-conversion mode. A fiber, a waveguide, a contact tip, or a combination thereof may be used in the instant systems.

In some embodiments, the system contains a monitoring device such as a temperature sensor or a thermal energy detector. In other embodiments, the systems also contain a controller means for modulating the nonlinear excitation source (e.g., a "feedback loop controller"). In a related embodiment, the system contains a means for detecting a temperature of the surface or a target tissue adjacent to the surface, wherein the controller means modulates the intensity of the nonlinear excitation source and/or the duration of the excitation. In such embodiments, the controller means preferably modulates the intensity of the nonlinear excitation source such that a first component of the hair follicle is selectively thermoablated relative to a second component of the hair follicle. In further embodiments, a cooling device is directly contacted with the skin during irradiation to minimize the heating of nanoparticles or skin at the surface, while nanoparticles that have penetrate more deeply into the follicle, skin, or sebaceous gland heat to temperatures that selectively ablate the adjacent tissues.

Skin is an exemplary target tissue. The skin preferably contains a hair follicle and/or a sebaceous gland, where the nonlinear excitation source generates energy that results in heating the skin in an amount effective to induce thermomodulation of a hair follicle, a infundibulum, a sebaceous gland, or a component thereof, such as by heating sufficient to cause the temperature of the skin to exceed 37° C., such as 38° C., 39° C., 40° C., 41° C., 42° C., 43° C., 44° C., 45° C., 46° C., 47° C., 48° C., 49° C., to about 50° C. or greater.

Methods of Formulation. Also provided are methods for formulating the nanoparticles of the present disclosure into a form suitable for use as described herein. In particular, the nanoparticle compositions are generated by:
a) forming a first mixture containing a plurality of nanoparticles and a first solvent;
b) exchanging the first solvent for a second solvent to form a second mixture; and
c) combining the second mixture and a cosmetically or pharmaceutically acceptable carrier;
thereby forming a nanoparticle composition.

The exchanging step is optionally performed using liquid chromatography, a solvent exchange system, a centrifuge, precipitation, or dialysis. Preferably, the nanoparticles are surface modified through a controlled reduction step or an oxidation step. Such surface modification may involve a coating step, such as the adsorbance of a monomer, polymer, or biological entity to a surface of the nanoparticle. Typically, the coating step involves contacting the nanoparticles with an oxidative environment. Further, the coating step may include monomer polymerization to create polymer coat.

The methods described herein may also include the steps of dissolving the nanoparticles in a non-polar solvent and subsequently mixing the dissolved nanoparticles with a polar solvent so as to encapsulate the nanoparticles in an emulsion. Further, the addition of surfactants (e.g. sodium dodecyl sulfate, sodium laureth 2-sulfate, ammonium lauryl sulfate, sodium octech-1/deceth-1 sulfate) at concentrations of 0.1-10% may be used to disrupt the epidermal skin barrier, emulsify the sebum and enable improved mixing of hydrophilic nanoparticles in aqueous solutions. Further, a concentration of the nanoparticles such as centrifugation or lyophilization may be employed. Further, the nanoparticles may be pretreated with heat or radiation. Also provided is the optional step of conjugating a biological entity or plurality of biological entities to the nanoparticles. Such a conjugating step may involve a thiol, amine, or carboxyl linkage of the biological entities to the nanoparticles.

Diseases and disorders. The present disclosure can be used on human (or other animal) skin for the treatment of wrinkles and other changes related to photo-aging or chronologic aging (generally termed skin rejuvenation), for the treatment of diseases including skin diseases, for the reduction of acne and related disorders such as rosacea, folliculitis, pseudofolliculitis barbae or proliferative or papulosquamous disorders such as psoriasis, for the stimulation or reduction of hair growth, and for reduction of cellulite, warts, hypopigmentation such as port-wine stain (PWS; nevus flammeus), birthmarks, hyperhidrosis, varicose veins, pigment problems, tattoos, vitiligo, melasma, scars, stretch marks, fungal infections, bacterial infections, dermatological inflammatory disorders, musculoskeletal problems (for example, tendonitis or arthritis), to improve healing of surgical wounds, burn therapy to improve healing and/or reduce and minimize scarring, improving circulation within the skin, and the like.

The present disclosure can also be useful in improving wound healing, including but not limited to chronic skin ulcers, diabetic ulcers, thermal burn injuries, viral ulcers or disorders, periodontal disease and other dental disease. The present disclosure, in certain embodiments, is also useful in enhancing the effects of devices that create an injury or wound in the process of performing cosmetic surgery including non-ablative thermal wounding techniques for treating skin wrinkles, scars, stretch marks and other skin disorders. Under such circumstances, it may be preferable to use conventional non-ablative thermal treatments in combination with the methods of the present disclosure. The instant application, in certain embodiments, are used in conjunction with micro- or surface abrasion, dermabrasion, or enzymatic or chemical peeling of the skin or topical cosmeceutical applications, with or without nanoparticle application to enhance treatment, as the removal of the stratum corneum (and possibly additional epithelial layers) can prove beneficial for some treatment regimen. The methods of the present disclosure are particularly applicable to, but are not limited to, acne treatment, hair removal, hair growth/hair follicle stimulation, reduction/prevention of malignant and non-malignant skin tumors, and skin rejuvenation, as described herein.

The dermatologically therapeutic methods described herein may be formed using nanoparticle irradiation alone, nanoparticle irradiation in combination with nano- or microparticles, or nanoparticle irradiation with a composition comprising nano- or microparticles and one or more therapeutic agents. Such nanoparticle irradiation may be produced by any known nanoparticle generator, and is preferably a focused nanoparticle generator capable of generating and irradiating focused nanoparticle waves.

In still further aspects, any or all or a portion of a component in a formulation described herein may be modified to include one or more characteristics primarily governed by or characteristics selected to foster modification of the nucleation density of a formulation or the ultrasound response of a formulation as to bubble nucleation, ultrasound cavitation or other sonification factors related to enhancing one or a combination of ultrasound assisted particle transport modes. It is to be appreciated that ultrasound response includes the characteristics or factors used for any ultrasound transport mode including by way of example a surface ultrasound delivery mode, an immersion ultrasound delivery mode, a cavitation ultrasound delivery mode as well as any jet or microjet or acoustically created microjet provided by an ultrasound delivery mode. Modifications may include formulations with particles of various different characteristics such as coated and uncoated particles, particles of the same and different shapes and same or different sizes, depending upon the desired ultrasound transport mode or delivery characteristics such as depth of penetration, speed of penetration, and other factors.

In one aspect, therapeutic particle formulations may be provided as described above and used with one or more ultrasound transport modes tailored to the particle formulation. One alternative particle formulation having improved ultrasound transport capabilities is a modified particle formulation for ultrasound transport. In this exemplary class of particles formulations, the components of the therapeutic particle formulation have been adapted to enhance one or more ultrasound transport characteristic. By way of example, one modified particle formulation may have less water by percentage that a therapeutic particle formulation. In still another example, one or more other components in a formulation may be modified (either increased, decreased or removed) so as to increase the cavitation collapse forces within the remaining formulation components. In one specific example, the surfactant concentration in a modified particle formulation is changed to increase cavitation collapse forces. In one aspect, a particle formulation has a lowered ratio or portion of ethanol to enhance the ultrasound transport properties of a formulation. In still another aspect, there is a formulation having less DIA while also maintaining the desired lipophilicity and lipid solubility of a ultrasound modified particle formulation. In one specific aspect, there is provided a particle formulation having high light absorbing particles. In one example the high light absorbing particles are nanoplates or nanorods sized below the pore and infundilular diameter. In one aspect, the major dimension of a nanoplate or nanorod is on the order of 200 nm or less.

In still another aspect, a modified particle formulation includes a plurality bubbles or a plurality of microbubbles. In some exemplary embodiments, a modified particle formulation includes one or more of seeds useful in forming nucleation sites to modify the nucleation density of a particle formulation. In one embodiment, a plurality of nucleation seed material is added wherein that material is selected that is not light absorbing or minimally light absorbing. In one specific embodiment, silica particles are added to a particle formulation as nucleation seeds. In another one seed example, galactose is ground into tiny crystals having irregular surfaces that act as nidation sites on which air pockets form. In one aspect, a surfactant is added into a formulation so as to adapt the stability of microbubbles formed in a modified particle formulation. One exemplary surfactant is palmitic acid. In another aspect, there is provided an amount of microbubbles having gas filled shells, including gases of high molecular weight, such as perflurocarbon. One specific example is perfluoropropane gas. In another aspect, the microbubbles provided herein may have a shell or a membrane selected to remain intact during all or a portion of the ultrasound transport phase of a particle based therapy describe herein. In still another aspect, there are provided microbubbles from one or more of any of the commercially available echogenic microbubbles approved for ultrasound imaging wherein the microbubbles are formulated to have the desired ultrasound response characteristics for the ultrasound properties in the selected ultrasound transport mode. Exemplary microbubble formulations that may be modified as needed from other ultrasound applications for use in ultrasound transport modes herein include, for example, those microbubble compositions provided under the commercial names of Levovist, Optison and Sonovue.

In another aspect, there is provided a combination mode or a dual mode particle formulation having a first or therapeutic particle or particles and also one or a plurality of particles or a percentage or concentration of particles or materials within a selected therapeutic particle formulation wherein the particles or materials are added to assist in one or more ultrasound transport modes of the therapeutic nanoparticle formulation. In one aspect, the particles combined into a therapeutic particle formulation are referred to as ultrasound transport mode particles. The characteristics of, amount of and other attributes of ultrasound transport mode particles are selected based on the ultrasound mode used and its specific properties and the desired transport effect.

In some embodiments, there is described a variety of compositions one or more particles, nanoparticles, or microparticles, or particle formulations, modified particle formulations, enhanced particle formulations, first therapeutic portions or transport delivery portions encompassing the use of delivery of light absorbing particles and molecules followed by irradiation with an appropriate source, such as near-IR wavelengths. It is believed that the typical pulse durations (i.e., in a range of 1 to 1,000 ms.) are effective in hair removal. However, in some treatment or therapy situations, additional selectivity to one target volume over another target volume may be beneficial in some patient populations. In one embodiment, there is provided one or more particles, nanoparticles, or microparticles, or particle formulations, modified particle formulations, enhanced particle formulations, first therapeutic portions or transport delivery portions selected so as to enable the selectivity of one tissue structure over an adjacent tissue structure. The selectivity could be accomplished using a variety techniques. One may select different light source, different materials or a modification to a level of transport effectiveness. In one aspect, an enhanced transport mode level may result in deeper penetration of activatable materials or, put another way, a more complete transport mode or a series of transport modes, or additional transport steps in the absence of additional activatable materials may reduce materials within more shallow regions of a target tissue volume. Conversely, reducing the transport mode or adjusting the transport mode for shallower penetration into a targeted tissue volume may be used to be more selective to treating shallow structure over deeper tissue volumes.

An additional variation as to being selective between adjacent tissue volumes includes adjustments to one or more steps of a method or adjustment of characteristics to a particle or a particle formulation so as to provide a desired or primary therapeutic effect while minimizing or reducing an undesired effect or an undesired side effect. In one aspect, there is provided a method for treating acne in a portion in a bearded area of a male wherein the side effect to be avoided is the removal of the beard hair. In one aspect, a bearded region is treated with a Q-switched laser at the same wavelengths for activation of the activatable material selected. In one aspect, the Q-switched laser is operated to provide a selective therapy using a pulse duration from 0.1 to 100 ns. It is believed that light activation energy will still be absorbed by the deposited energy activatable materials or light absorbers and lead to damage to the sebaceous units. Advantageously, such pulse durations do not lead to long term hair removal thereby avoiding the undesired side effect of loss of beard hair while treating acne in a bearded portion of a tissue volume.

In some embodiments of combinational or dual mode particles or particle formulations, there are particles included for ultrasound transport enhancement having therapeutic response characteristics different from those of the therapeutic particles in the formulation or assisted in transporting. In one embodiment, a calculated therapeutic effect or therapeutic response of the particles in a dual mode particle formulation is substantially only provided by the therapeutic particles in the formulation. In another aspect, a portion of the ultrasound transport particles are also active or responsive to the therapeutic method or therapy conducted or intended to be performed using the therapeutic particles in the formulation. In still another embodiment, a calculated therapeutic effect or therapeutic response of the particles in a dual mode particle formulation is provided by or determined based on contributions to the therapy or method provided by both the therapeutic particles and the transport particles in the formulation. In other alternative aspects, the specific particle formulation (i.e., particle formulation, modified particle formulation or enhanced particle formulation) as well as intended therapeutic effect for such formulation includes any formulation combination to achieve a therapeutic effect described herein. In one specific aspect, the therapeutic effect includes absorption of light by one or more or a plurality of particles responsive to the light and the resulting selective heating of nearby tissue, structures, or fluids.

In still another aspect, there is provided a combination mode or a dual mode particle formulation having a first portion of the particles included for a therapy and a second portion of particles included in order to aid in one or more ultrasound transport modes for the first portion or a substantial portion of the first portion of particles. In an additional aspect, there is a first or selective phototherapy particle or particles and also one or a plurality of particles or a percentage or concentration of particles or materials within a selected selective phototherapy particle formulation wherein the particles or materials are added to assist in one or more ultrasound transport modes of the selective phototherapy nanoparticle formulation. In one aspect, the particles or materials to assist in one or more ultrasound transport modes particles included within a selective phototherapy particle formulation are referred to as ultrasound transport mode particles. The characteristics of, amount of and other attributes of ultrasound transport mode particles are selected based on the ultrasound mode used and its specific properties and the desired transport effect.

In some embodiments of combinational or dual mode particles or particle formulations, there are particles included for ultrasound transport enhancement having selective phototherapy response characteristics different from those of the selective phototherapy particles in the formulation or assisted in transporting. In one embodiment, a calculated therapeutic effect or therapeutic response of the particles in a dual mode particle formulation is substantially only provided by the selective phototherapy particles in the formulation. In another aspect, a portion of the ultrasound transport particles are also active or responsive to the selective phototherapy method or therapy conducted or intended to be performed using the selective phototherapy particles in the formulation. In still another embodiment, a calculated therapeutic effect or therapeutic response of the particles in a dual mode particle formulation is provided by or determined based on contributions to the therapy or method provided by both the selective phototherapy particles and the transport particles in a given selective phototherapy formulation.

In still other embodiments of combinational or dual mode nanoparticles, there are particles included for ultrasound transport enhancement having optical characteristics different from those of the therapeutic nanoparticles. In another aspect, a portion of the ultrasound transport particles are also optically active in the same range as the therapeutic nanoparticles.

In still another aspect, one or more particles or a percentage of particles in a formulation are selected to provide one or more or a range of sonochemical actions or reactions within the operating range of the ultrasound mode. In one aspect, one or a plurality or a percentage of particles in the formulation is selected to be responsive to the ultrasound mode used for particle transport. Responsive to the ultrasound mode includes any of a variety of immediate, delayed or amplitude based responses to ultrasound energy or duration of exposure to ultrasound energy including, for example bursting all or a portion of a particle, undergoing a chemical reaction and/or undergoing a physical modification and/or undergoing a modification so as to release another compound or particle.

It is believed that ultrasound assisted particle delivery as described herein has the ability to drive particles into the skin appendages to a much higher extent and in much higher fractions of appendages than mechanical massage. As a result, there are new therapeutic methods having the possibility of achieving highly efficient selective photothermolysis of these appendages by loading of energy absorbable materials such as light absorbing chromophores as described herein into the appendages, removing any excess material from the skin and then treating with pulsed laser or light irradiation. Examples of appendages and corresponding clinical applications are:
1. Sebaceous follicles including infundibulum, sebaceous duct, and sebaceous gland including treatments for acne
2. Eccrine and apocrine sweat glands: hyperhidrosis
3. Hair follicles: light colored hair, all fine (dark and light) hair.
4. Lesional epidermis and demis in lesions.

As a result of the embodiments of the device, methods or particle formulations alone or in combination there is provided improved treatments that may now begin by utilizing an ultrasound delivery mode for driving the particles within an appendage to achieve higher particle density and targeting a larger fraction of appendages allows: more complete selective photothermolysis, requirement of lower light energy density - less pain and safer, higher and more durable efficacy.

In one aspect, an embodiment of ultrasound assisted particulate delivery is mediated via collapsing cavitation bubbles. The collapse near the skin surface generates high speed jets directed towards the surface. Also, shock waves propel the particles. These can be successful in driving spherical particles in formulations, modified formulations or enhanced formulations. In some embodiments, however, a particle formulation includes a portion of the particles having non-spherical shapes such as shapes having at least one edge and one corner. It is believed that there may be advantages to having particles with edges and corners. In one embodiment, there is a particle formulation having at least a portion of the particles having shape with edges and corners. In one aspect, one or more of an edge or a corner or plural edges or plural corners act as nucleation seeds and make formation of cavitation bubbles more likely in such a particle formulation. In one aspect, the particles with edges and corners are also therapeutically responsive to the later performed therapy. In one alternative embodiment, the particles with edges and corners are unresponsive or responsive below a therapeutic threshold in a later performed therapy step. In one aspect related to ultrasound transport, particles with edges and corners present in a fluid, lower ultrasound energy can generate desired density of cavitation bubbles. Alternatively, for the same ultrasound energy density, higher cavitation events per volume per time ensue, increasing the performance. For example, nanoplates such as silver nanoplates can be designed to absorb in the near IR region of the electromagnetic spectrum. In one aspect, such particles may have a typical dimension of 100 nm side length and 5-10 nm width. With the 9 edges and 6 corners, enhanced density and frequency of cavitation events is expected. Other shapes such as nanorods, nanorice, and the like have a similar effect on the nucleation and cavitation phenomena.

### Examples:

1. Hyperhidrosis: Tortuous path of the sweat carrying tube has to be traversed to reach deeply located sweat glands. Hence, longer times are needed to drive the particles deep. This time can be shortened by increasing the cavitation bubble formation and collapse frequency as suggested above.
2. Light and Fine Hair Removal: Light and fine hair don't have enough melanin to get efficacy with traditional light based hair removal techniques that target melanin pigment as the light absorber. In addition to lower melanin, for fine hair, the channel is narrower. Higher cavitation collapse density and frequency will aid in driving more particles in a shorter time.

Additionally or optionally, one or more of the delivery device operating parameters, and/or methods of use of the delivery system described herein may be modified based upon one or more characteristics of the delivery fluid, a component of the delivery fluid or a particle within the delivery fluid being used to enhance one or both of a therapeutic performance of a particle, particles or a formulation containing particles or to modify a particle delivery transport modality for a particle formulation. By way of example, one or more parameters or user settings may be adjusted to provide specific ultrasound delivery conditions design to match a desired particle transport mode for a formulation.

As mentioned, other embodiments may be utilized and derived there from, such that structural and logical substitutions and changes may be made without departing from the scope of this disclosure. Such embodiments of the inventive subject matter may be referred to herein individually or collectively by the term "invention" merely for convenience and without intending to voluntarily limit the scope of this application to any single invention or inventive concept, if more than one is, in fact, disclosed. Thus, although specific embodiments have been illustrated and described herein, any arrangement calculated to achieve the same purpose may be substituted for the specific embodiments shown. This disclosure is intended to cover any and all adaptations or variations of various embodiments. Combinations of the above embodiments, and other embodiments not specifically described herein, will be apparent to those of skill in the art upon reviewing the above description.

## Claims

1. A device (100) for delivery of a fluid substance into skin comprising:
a distal end surface (134) for contacting a skin surface;
an ultrasound horn (115), said ultrasound horn (115) mated to an assembly (130), said assembly (130) adapted to hold a fluid (10) surrounding said ultrasound horn (115), said device positions said ultrasound horn (115) about 0 to 30 mm from the skin surface, and said fluid (10) comprises a plurality of nanoparticles having a size in the range of 100 to 1000 nm,
said ultrasound horn (115) having an adjustable frequency of about 20 kHz to about 200 kHz with an amplitude of between about 5 and 35 microns, and
said device (100) further comprising an annulus (145) in said end surface (134) for contacting a skin surface in communication with a reduced pressure source, said annulus (145) comprising less than half of the surface for contacting a skin surface.

2. The device for delivery of a substance into skin of Claim 1 wherein said device (100) further comprises:
means (122, 140) for positioning said ultrasound horn (115) about 11 to 14 mm from the skin.

3. The device for delivery of a substance into skin of Claim 1 or Claim 2 wherein said device (100) further comprises a means (146) for adjusting the reduced pressure applied to said skin surface through said aperture (145) in said device's surface for contacting a skin surface.

4. The device for delivery of a substance into skin of Claim 3 wherein said means for adjusting the reduced pressure (146) is effective to apply a reduced pressure of between about -0.8 atm to about -0.1 atm.

5. The device for delivery of a substance into skin of Claim 3 wherein said means for adjusting the reduced pressure (146) is effective to apply a reduced pressure of between about -0.5 atm to about -0.1 atm.

6. The device for delivery of a substance into skin of Claim 3 wherein said means for adjusting the reduced pressure (146) is effective to apply a reduced pressure of between about -0.33 atm to about -0.1 atm.

7. The device for delivery of a substance into skin of any one of the preceding Claims wherein said device (100) further comprises a heat exchanger (172, 174) in said cup assembly (130), said heat exchanger (172, 174) in fluid communication with a temperature control fluid reservoir external to said cup assembly.

8. The device for delivery of a substance into skin of any one of the preceding Claims wherein said ultrasound horn (115) further comprises the ability to deliver controlled ultrasound with an amplitude peak-to-peak displacement of about 8.8-12.0 microns.

9. The device for delivery of a substance into skin of any one of the preceding Claims wherein said ultrasound horn (115) further comprises the ability to deliver controlled ultrasound with a frequency in the range of 20 kHz to 100 kHz.

10. The device for delivery of a substance into skin of Claim 9 wherein said ultrasound horn (115) further comprises the ability to deliver controlled ultrasound with a frequency in the range of 20 kHz to 60 kHz.

11. The device for delivery of a substance into skin of Claim 10 wherein said ultrasound horn (115) further comprises the ability to deliver controlled ultrasound with a frequency in the range of 30 kHz to 50 kHz.

12. The device for delivery of a substance into skin of any one of the preceding Claims wherein said ultrasound horn (115) further comprises the ability to deliver controlled ultrasound with a power density during the operating step is from about 0.5-50 W/cm².

13. The device for delivery of a substance into skin of any one of the preceding Claims wherein said ultrasound horn (115) further comprises the ability to deliver controlled ultrasound with a face peak-to-peak amplitude displacement in the range of 0.5 to 30 microns.

## Patentansprüche

1. Vorrichtung (100) zur Abgabe einer fließfähigen Substanz in die Haut, umfassend:
eine distale Endfläche (134) zum Inkontaktbringen einer Hautoberfläche;
ein Ultraschallhorn (115), wobei das Ultraschallhorn (115) mit einer Baugruppe (130) verbunden ist, wobei die Baugruppe (130) geeignet ist, ein Fluid (10) aufzunehmen, das das Ultraschallhorn (115) umgibt, wobei die Vorrichtung das Ultraschallhorn (115) etwa 0 bis 30 mm von der Hautoberfläche entfernt positioniert, und wobei das Fluid (10) mehrere Nanopartikel mit einer Größe im Bereich von 100 bis 1000 nm umfasst, das Ultraschallhorn (115) eine einstellbare Frequenz von etwa 20 kHz bis etwa 200 kHz mit einer Amplitude zwischen etwa 5 und 35 Mikron aufweist, und die Vorrichtung (100) ferner einen Ring (145) in der Endfläche (134) zum Inkontaktbringen einer Hautoberfläche in Verbindung mit einer Unterdruckquelle aufweist, wobei der Ring (145) weniger als die Hälfte der Oberfläche zum Inkontaktbringen einer Hautoberfläche umfasst.

2. Vorrichtung zur Abgabe einer Substanz in die Haut nach Anspruch 1, wobei die Vorrichtung (100) ferner Folgendes umfasst:
Einrichtung (122, 140) zum Positionieren des Ultraschallhorns (115), in einem Abstand zur Haut von etwa 11 bis 14 mm.

3. Vorrichtung zur Abgabe einer Substanz in die Haut nach Anspruch 1 oder Anspruch 2, wobei die Vorrichtung (100) ferner eine Einrichtung (146) zum Einstellen des auf die Hautoberfläche ausgeübten reduzierten Drucks durch die Öffnung (145) in der Oberfläche der Vorrichtung zum Inkontaktbringen einer Hautoberfläche umfasst.

4. Vorrichtung zur Abgabe einer Substanz in die Haut nach Anspruch 3, wobei die Einrichtung zum Einstellen des Unterdrucks (146) geeignet ist, um einen reduzierten Druck von etwa -0,8 bis etwa -0,1 atm anzuwenden.

5. Vorrichtung zur Abgabe einer Substanz in die Haut nach Anspruch 3, wobei die Einrichtung zum Einstellen des Unterdrucks (146) geeignet ist, um einen reduzierten Druck von etwa -0,5 bis etwa -0,1 atm anzuwenden.

6. Vorrichtung zur Abgabe einer Substanz in die Haut nach Anspruch 3, wobei die Einrichtung zum Einstellen des Unterdrucks (146) geeignet ist, um einen reduzierten Druck von etwa -0,33 atm bis etwa -0,1 atm anzuwenden.

7. Vorrichtung zur Abgabe einer Substanz in die Haut nach einem der vorhergehenden Ansprüche, wobei die Vorrichtung (100) ferner einen Wärmetauscher (172, 174) in der Becherbaugruppe (130) umfasst, wobei der Wärmetauscher (172, 174) in Fluidverbindung mit einem Temperierfluidreservoir außerhalb der Becherbaugruppe steht.

8. Vorrichtung zur Abgabe einer Substanz in die Haut nach einem der vorhergehenden Ansprüche, wobei das Ultraschallhorn (115) ferner die Fähigkeit besitzt, einen gesteuerten Ultraschall mit einer Spitze-zu-Spitze-Amplitudenverschiebung von etwa 8,8-12,0 Mikron zu erzeugen.

9. Vorrichtung zur Abgabe einer Substanz in die Haut nach einem der vorhergehenden Ansprüche, wobei das Ultraschallhorn (115) ferner die Fähigkeit besitzt, einen gesteuerten Ultraschall im Frequenzbereich von 20 kHz bis 100 kHz zu erzeugen.

10. Vorrichtung zur Abgabe einer Substanz in die Haut nach Anspruch 9, wobei das Ultraschallhorn (115) ferner die Fähigkeit besitzt, einen gesteuerten Ultraschall im Frequenzbereich von 20 kHz bis 60 kHz zu erzeugen.

11. Vorrichtung zur Abgabe einer Substanz in die Haut nach Anspruch 10, wobei das Ultraschallhorn (115) ferner die Fähigkeit umfasst, einen gesteuerten Ultraschall im Frequenzbereich von 30 kHz bis 50 kHz zu erzeugen.

12. Vorrichtung zur Abgabe einer Substanz in die Haut nach einem der vorhergehenden Ansprüche, wobei das Ultraschallhorn (115) ferner die Fähigkeit besitzt, während des Betriebs einen gesteuerten Ultraschall mit einer Leistungsdichte von etwa 0,5-50 W/cm² zu liefern.

13. Vorrichtung zur Abgabe einer Substanz in die Haut nach einem der vorhergehenden Ansprüche, wobei das Ultraschallhorn (115) ferner die Fähigkeit besitzt, einen gesteuerten Ultraschall mit einer Spitze-zu-Spitze-Amplitudenverschiebung im Bereich von 0,5 bis 30 Mikron zu erzeugen.

## Revendications

1. Dispositif (100) d'administration d'une substance fluide dans la peau comprenant :
une surface d'extrémité distale (134) pour l'entrée en contact avec une surface cutanée ;
une trompe à ultrasons (115), ladite trompe à ultrasons (115) étant couplée à un ensemble (130), ledit ensemble (130) étant adapté pour contenir un fluide (10) entourant ladite trompe à ultrasons (115), ledit dispositif place ladite trompe à ultrasons (115) à environ 0 à 30 mm de la surface cutanée, et ledit fluide (10) comprend une pluralité de nanoparticules ayant une taille dans la plage de 100 à 1000 nm,
ladite trompe à ultrasons (115) ayant une fréquence réglable d'environ 20 kHz à environ 200 kHz avec une amplitude comprise entre environ 5 et 35 microns, et
ledit dispositif (100) comprenant en outre un anneau (145) dans ladite surface d'extrémité (134) pour l'entrée en contact avec une surface cutanée en communication avec une source de pression réduite, ledit anneau (145) comprenant moins de la moitié de la surface pour l'entrée en contact avec une surface cutanée.

2. Dispositif d'administration d'une substance dans la peau selon la revendication 1, ledit dispositif (100) comprenant en outre :
un moyen (122, 140) de positionnement de ladite trompe à ultrasons (115) à environ 11 à 14 mm de la peau.

3. Dispositif d'administration d'une substance dans la peau selon la revendication 1 ou la revendication 2, ledit dispositif (100) comprenant en outre un moyen (146) de réglage de la pression réduite appliquée à ladite surface cutanée à travers ladite ouverture (145) dans la surface dudit dispositif pour l'entrée en contact avec une surface cutanée.

4. Dispositif d'administration d'une substance dans la peau selon la revendication 3, dans lequel ledit moyen de réglage de la pression réduite (146) est efficace pour appliquer une pression réduite comprise entre environ -0,8 atm et environ -0,1 atm.

5. Dispositif d'administration d'une substance dans la peau selon la revendication 3, dans lequel ledit moyen de réglage de la pression réduite (146) est efficace pour appliquer une pression réduite comprise entre environ -0,5 atm et environ -0,1 atm.

6. Dispositif d'administration d'une substance dans la peau selon la revendication 3, dans lequel ledit moyen de réglage de la pression réduite (146) est efficace pour appliquer une pression réduite comprise entre environ -0,33 atm et environ -0,1 atm.

7. Dispositif d'administration d'une substance dans la peau selon l'une quelconque des revendications précédentes, ledit dispositif (100) comprenant en outre un échangeur de chaleur (172, 174) dans ledit ensemble de coupelle (130), ledit échangeur de chaleur (172, 174) étant en communication fluide avec un réservoir de fluide de contrôle de température externe audit ensemble de coupelle.

8. Dispositif d'administration d'une substance dans la peau selon l'une quelconque des revendications précédentes, dans lequel ladite trompe à ultrasons (115) comprend en outre la capacité d'administrer des ultrasons contrôlés avec un déplacement d'amplitude crête à crête d'environ 8,8-12,0 microns.

9. Dispositif d'administration d'une substance dans la peau selon l'une quelconque des revendications précédentes, dans lequel ladite trompe à ultrasons (115) comprend en outre la capacité d'administrer des ultrasons contrôlés à une fréquence dans la plage de 20 kHz à 100 kHz.

10. Dispositif d'administration d'une substance dans la peau selon la revendication 9, dans lequel ladite trompe à ultrasons (115) comprend en outre la capacité d'administrer des ultrasons contrôlés à une fréquence dans la plage de 20 kHz à 60 kHz.

11. Dispositif d'administration d'une substance dans la peau selon la revendication 10, dans lequel ladite trompe à ultrasons (115) comprend en outre la capacité d'administrer des ultrasons contrôlés à une fréquence dans la plage de 30 kHz à 50 kHz.

12. Dispositif d'administration d'une substance dans la peau selon l'une quelconque des revendications précédentes, dans lequel ladite trompe à ultrasons (115) comprend en outre la capacité d'administrer des ultrasons contrôlés avec une densité de puissance durant l'étape de fonctionnement qui est d'environ 0,5 à 50 W/cm2.

13. Dispositif d'administration d'une substance dans la peau selon l'une quelconque des revendications précédentes, dans lequel ladite trompe à ultrasons (115) comprend en outre la capacité d'administrer des ultrasons contrôlés avec un déplacement d'amplitude crête à crête de face dans la plage de 0,5 à 30 microns.
